(19) Europäisches Patentamt
European Patent Office
Office européen des brevets

(11) Numéro de publication : 0 636 614 A1

(12) # DEMANDE DE BREVET EUROPEEN

(21) Numéro de dépôt : 94401736.7

(22) Date de dépôt : 28.07.94

(51) Int. Cl.[6] : C07D 235/26, C07D 401/04, C07D 403/12, A61K 31/415, C07D 417/12, C07D 405/04

(30) Priorité : 30.07.93 FR 9309403

(43) Date de publication de la demande : 01.02.95 Bulletin 95/05

(84) Etats contractants désignés : AT BE CH DE DK ES FR GB GR IE IT LI LU MC NL PT SE

(71) Demandeur : SANOFI
32-34, rue Marbeuf
F-75008 Paris (FR)

(72) Inventeur : Di Malta, Alain
170, rue Vignebelle
34980 Saint Clement de Riviere (FR)
Inventeur : Mettefeu, Daniel
13 Rue du Bosquet
F-34790 Grabels (FR)
Inventeur : Roux, Richard
420 Chemin des Rossignols
F-34570 Vailhauques (FR)
Inventeur : Garcia, Georges
27 Rue des Aires
F-34980 Saint Gely du Fesc (FR)
Inventeur : Nisato, Dino
2, Rue de Terre Rouge
F-34680 Saint Georges d'Orques (FR)
Inventeur : Serradeil-Legal, Claudine
45 Avenue des Troubadours
F-31750 Escalquens (FR)

(74) Mandataire : Gillard, Marie-Louise et al
Cabinet Beau de Loménie
158, rue de l'Université
F-75340 Paris Cédex 07 (FR)

(54) **Dérivés de 1-benzènesulfonyl-1,3-dihydro-2H-benzimidazol-2-one, leur préparation, les compositions pharmaceutiques en contenant.**

(57) La présente invention concerne des dérivés de 1-benzènesulfonyl-1,3-dihydro-2H-benzimidazol-2-one de formule :

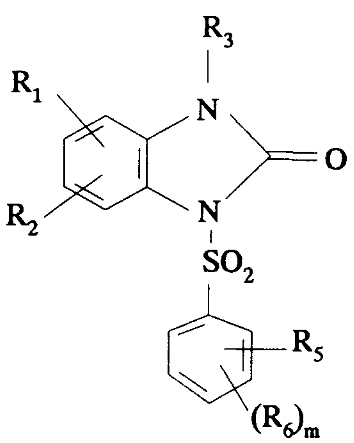

(I)

leur préparation et les compositions pharmaceutiques en contenant.

Ces composés ont une affinité pour les récepteurs de la vasopressine et de l'ocytocine.

EP 0 636 614 A1

Jouve, 18, rue Saint-Denis, 75001 PARIS

La présente invention a pour objet des dérivés de 1-benzènesulfonyl-1,3-dihydro-2*H*-benzimidazol-2-one, leur préparation et les compositions pharmaceutiques en contenant.

Récemment, plusieurs demandes de brevet ont décrit des familles de composés à structure non-peptidique ayant une activité sur les récepteurs de la vasopressine et/ou de l'ocytocine. On peut citer les demandes de brevets européens EP 382 185, EP 444 945, EP 514 667, EP 469 984 et EP 526 348, les demandes WO 91/05 549 et WO 93/15 051, et les demandes de brevet japonais JP-04/321 669 et 03/127732.

Les dérivés de 1-benzènesulfonyl-1,3-dihydro-2*H*-benzimidazol-2-one selon la présente invention ont une affinité pour les récepteurs de la vasopressine et de l'ocytocine.

La vasopressine est une hormone connue pour son effet antidiurétique et son effet dans la régulation de la pression artérielle. Elle stimule plusieurs types de récepteurs : $V_1$ ($V_{1a}$, $V_{1b}$), $V_2$. Ces récepteurs sont localisés dans le foie, les vaisseaux (coronaires, rénaux, cérébraux), les plaquettes, le rein, l'utérus, les glandes surrénales, le système nerveux central, l'hypophyse. L'ocytocine a une structure peptidique proche de celle de la vasopressine. Les récepteurs de l'ocytocine se trouvent aussi sur le muscle lisse de l'utérus, ils se trouvent également sur des cellules myoépithéliales de la glande mammaire, dans le système nerveux central et dans le rein. La localisation des différents récepteurs est décrite dans : S. JARS et al., Vasopressin and oxytocin receptors : an overview, *in* Progress in Endocrinology. H. IMURA and K. SHIZURNE ed., Experta Medica, Amsterdam, 1988, 1183-1188, ainsi que dans les articles suivants : Presse Médicale, 1987, 16 (10), 481-485, J. Lab. Clin. Med., 1989, 114 (6), 617-632 et Pharmacol. Rev., 1991, 43 (1), 73-108. La vasopressine exerce ainsi des effets cardiovasculaires, hépatiques, antidiurétiques, agrégants et des effets sur le système nerveux central et périphérique, et sur la sphère utérine. L'ocytocine intervient dans la parturition, la lactation et le comportement sexuel.

Les composés selon la présente invention permettent soit de mimer les effets de l'hormone (pour les agonistes), soit de les inhiber (pour les antagonistes), de façon sélective. Les antagonistes des récepteurs de la vasopressine peuvent intervenir sur la régulation de la circulation centrale et périphérique, notamment les circulations coronaire, rénale et gastrique, ainsi que sur la régulation hydrique et la libération de l'hormone adrénocorticotrophique (ACTH). Les agonistes de la vasopressine peuvent remplacer avantageusement la vasopressine ou ses analogues dans le traitement du diabète insipide ; ils peuvent également être utilisés dans le traitement de l'énurésie, et dans la régulation de l'hémostase : traitement de l'hémophilie, du syndrome de Von Willebrand, antidote des agrégants plaquettaires, F.A. LASZLO,, Pharmacol. Rev., 1991, 43, 73-108. Drug Investigation, 1990, 2 (Suppl. 5), 1-47. Les hormones elles-mêmes : la vasopressine et l'ocytocine ainsi que certains de leurs analogues peptidiques ou non peptidiques sont utilisés en thérapeutique et ont montré leur efficacité. On peut citer plusieurs revues et de nombreux articles de la littérature:Vasopressin, P. GROSS et al. ed. John Libbey Eurotext, 1993, en particulier 243-257 et 549-562. F.A. LASZLO and F.A. LASZLO Jr., Clinical perspectives for vasopressin antagonists, Drug News Perspect., 1993, 6 (8) ; W.G. NORTH, J. Clin. Endocrinol., 1991, 73, 1316-1320. J.J. LEGROS et al., Prog. Neuro-Pharmacol. Biol. Psychiat., 1988, 12, 571-586 ; K.E. ANDERSSON et al., Drugs Today, 1988, 24 (7) 509-528 ; D.L. STUMP et al., Drugs, 1990, 39, 38-53 ; S. CALTABIANO et al., Drugs Future, 1988, 13, 25-30 ; Y. MURA et al., Clin. Nephrol. 1993, 40, 60-61 ; Faseb J., 1994, 8 (5), A 587 : 3398.

Ainsi les composés selon l'invention sont utiles notamment dans le traitement des affections du système nerveux central et périphérique, du système cardiovasculaire, de la sphère rénale, de la sphère gastrique et dans les troubles du comportement sexuel, chez l'homme et chez l'animal.

Selon l'un de ses aspects, la présente invention a pour objet des composés de formule :

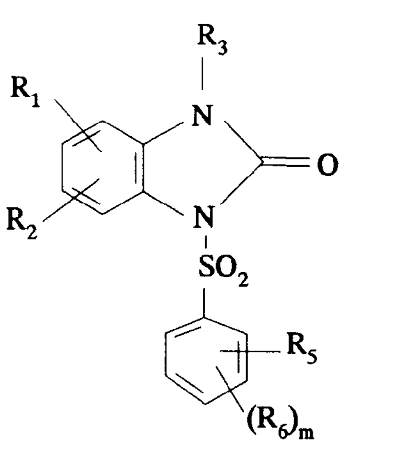

(I)

dans laquelle :

- $R_1$ et $R_2$ représentent chacun indépendamment un hydrogène ; un halogène ; un hydroxy ; un ω-halogéno($C_1$-$C_7$)alcoxy ; un ($C_1$-$C_7$)alkyle ; un trifluorométhyle ; un ($C_1$-$C_7$)alcoxy ; un polyhalogéno($C_1$-$C_7$)alcoxy ; un ω-hydroxy($C_2$-$C_7$)alcoxy ; un ω-méthoxy($C_2$-$C_7$)alcoxy ; un ω-amino($C_2$-$C_7$)alcoxy dans lequel l'amino est libre ou substitué par un ou deux ($C_1$-$C_7$)alkyles ; un ($C_3$-$C_7$)cycloalkyloxy ; un ($C_3$-$C_7$)cycloalkylméthoxy ; un phénoxy ; un benzyloxy ; un ($C_1$-$C_7$)alkylthio ; un phénylthio ; un nitro ; un amino libre ou substitué par un ou deux ($C_1$-$C_7$)alkyles ; un cyano ; un ($C_1$-$C_7$)alkylcarbonyle ; un formyle ; un benzoyle ; un formyloxy ; un ($C_1$-$C_7$)alkylcarbonyloxy ; un benzoyloxy ; un ($C_1$-$C_7$)alkylsulfonamido ; un phénylsulfonamido ; un benzylsulfonamido ; un ($C_1$-$C_7$) alkylcarbonylamino ; un ($C_1$-$C_7$) alcoxycarbonylamino ; un uréido non substitué ou substitué par un phényle, un benzyle ou par un ou deux ($C_1$-$C_7$)alkyles ; un thiouréido non substitué ou substitué par un phényle, un benzyle ou par un ou deux ($C_1$-$C_7$)alkyles ;
- $R_3$ représente $R_4$ ; un ($C_1$-$C_8$)alkyle ; un ($C_1$-$C_8$)alkylène substitué par $R_4$ ; un ($C_1$-$C_8$)alkylène substitué par un ($C_1$-$C_4$)alcoxy ; un indanyle ; un hexahydroindanyle ; un adamantyle ; un noradamantyle ; un norbornyle ; un cyclohexyle substitué par un di($C_1$-$C_7$)alkylamino, un carboxy, un ($C_1$-$C_4$)alcoxycarbonyle, un hydroxy, un tétrahydropyran-2-yloxy, un ($C_1$-$C_4$)alcoxy($C_1$-$C_4$)alcoxy ou un phényl($C_1$-$C_2$)alcoxy($C_1$-$C_4$)alcoxy ;
- $R_4$ représente un groupe $-NR_{16}R_{17}$ ; un ($C_3$-$C_7$)cycloalkyle non substitué ou substitué une ou deux fois par un ($C_1$-$C_4$)alkyle ou un ($C_1$-$C_4$)alcoxy ; un groupe Ar ; un furyle ; un thiényle ; un pyrrolyle ; un triazolyle ; un tétrazolyle ; un pyridyle ; un pyridyle N-oxyde ; un pyrimidinyle ; un pyrazolyle ; un pyrazinyle ; un tétrahydropyran-4-yle ; un azétidin-3-yle substitué en position 1 par $R_{18}$ ; un pipérid-4-yle substitué en position 1 par $R_{18}$ ou disubstitué en position 1 par un ou deux ($C_1$-$C_7$)alkyles et/ou un ou deux benzyles ; un pyrrolidinyle ; un perhydroazépinyle ; un morpholinyle ;
- $R_5$ et $R_6$ représentent chacun indépendamment un hydrogène ; un halogène ; un ($C_1$-$C_7$)alkyle ; un trifluorométhyle ; un cyano ; un nitro ; un hydroxylamino ; un carboxy ; un guanidino non substitué ou substitué en position 1 par un ($C_1$-$C_7$)alkyle et/ou en position 3 par un ou deux ($C_1$-$C_7$)alkyles, un phényle ou un benzyle et/ou en position 2 par un cyano ; un groupe $-OR_7$ ; un groupe $-SR_7$ ; un ($C_1$-$C_7$)alkylcarbonyle ; un formyle ; un benzoyle ; un ($C_1$-$C_7$)alcoxycarbonyle ; un phénoxycarbonyle ; un benzyloxycarbonyle ; un groupe $-CONR_{19}R_{20}$ ; un groupe $CSNR_{11}R_{27}$ ; un groupe $-SO_2-NR_{21}R_{22}$ ; un ($C_1$-$C_7$)alkylsulfonamido ; un benzylsulfonamido ; un groupe $-NHSO_2-Ar$ ; un groupe $-NR_8R_9$ ; un groupe $-CO-NH-CR_{10}R_{23}-CO-R_{12}$ ; un groupe $-CH_2NR_8R_9$.
- $R_7$ représente un hydrogène ; un ($C_1$-$C_7$)alkyle ; un phényle ; un benzyle ; un ($C_3$-$C_7$)cycloalkyle ; un ($C_2$-$C_7$)alcényle ; un ω-halogéno($C_2$-$C_7$)alkyle ; un polyhalogéno($C_1$-$C_7$)alkyle ; un ω-hydroxy($C_2$-$C_7$)alkyle ; un ($C_1$-$C_7$)alkylcarbonyle ; un formyle ; un benzoyle ; un ω-carboxy($C_1$-$C_7$)alkyle ; un ω-($C_1$-$C_7$)alcoxycarbonyl($C_1$-$C_7$)alkyle ; un ω-benzyloxycarbonyl($C_1$-$C_7$)alkyle ; un ω-amino($C_2$-$C_7$)alkyle dans lequel le groupe amino est libre ou substitué par un ou deux ($C_1$-$C_7$)alkyles, ou sous forme d'ion ammonium ; un ω-carbamoyl($C_1$-$C_7$)alkyle dans lequel le carbamoyle est libre ou substitué par un ou deux ($C_1$-$C_7$)alkyles ;
- $R_5$ et $R_9$ représentent chacun indépendamment un hydrogène ; un ($C_1$-$C_7$)alkyle ; un groupe $-CH_2-Ar$ ; $R_9$ peut de plus représenter un groupe Ar ; un ($C_3$-$C_8$)alcényle ; un ($C_1$-$C_7$)alkylcarbonyle ; un formyle ; un ($C_1$-$C_7$)alkylthiocarbonyle ; un ($C_3$-$C_7$)cycloalkylcarbonyle ; un ($C_3$-$C_7$)cycloalkylthiocarbonyle ; un ω-amino($C_2$-$C_7$)alkylcarbonyle dans lequel l'amino est libre ou substitué par un ou deux ($C_1$-$C_7$)alkyles ; un ω-hydroxy($C_1$-$C_7$)alkylcarbonyle ; un ω-benzyloxy ($C_1$-$C_7$)alkylcarbonyle ; un pyridylcarbonyle ; un méthylpyridylcarbonyle ; un thiénylcarbonyle ; un groupe -CO-Ar ; un groupe $-CO-CH_2-Ar$ ; un ($C_1$-$C_7$)alcoxycarbonyle ; un phénoxycarbonyle ; un phénoxythiocarbonyle ; un benzyloxycarbonyle ; un groupe $-CO-CR_{10}R_{23}-NR_{11}R_{27}$ ; un groupe $-CR_{10}R_{23}COR_{12}$ ; un groupe $-(CH_2)_tCOR_{12}$ ; un groupe $-CO(CH_2)_u COR_{12}$ ; un groupe $-CONR_{14}R_{24}$ ; un groupe $-CSNR_{14}R_{24}$ ; un radical hétérocyclique choisi parmi pyrazolyle, imidazolyle, triazolyle, tétrazolyle, pyridyle, pyridazinyle, pyrimidinyle, thiazolyle ;
- ou bien $R_5$ et $R_9$ ensemble avec l'atome d'azote auquel ils sont liés constituent l'hydantoïne, la N-méthylhydantoïne, ou un radical hétérocyclique choisi parmi le morpholin-4-yle, le pyrrol-1-yle, le Δ3-pyrrolin-1-yle, le pyrrolidin-1-yle, l'isoindolin-2-yle dans lequel le noyau benzénique est non substitué ou substitué par un halogène, un ($C_1$-$C_7$)alkyle, un trifluorométhyle ou un méthoxy ;
- $R_{10}$ et $R_{23}$ représentent chacun indépendamment l'hydrogène ; un ($C_1$-$C_7$)alkyle ; un benzyle ;
- ou bien $R_{10}$ et $R_{23}$ ensemble avec l'atome de carbone auquel ils sont liés constituent un ($C_3$-$C_7$)cycloalkyle ;
- $R_{11}$ et $R_{27}$ représentent chacun indépendamment l'hydrogène ou un ($C_1$-$C_7$)alkyle ;
- $R_{12}$ représente un hydroxy ; un ($C_1$-$C_7$)alcoxy ; un amino libre ou substitué par un ou deux ($C_1$-$C_7$)alkyles ;
- $R_{14}$ et $R_{24}$ représentent chacun indépendamment l'hydrogène ; un ($C_1$-$C_7$)alkyle ; $R_{24}$ peut de plus représenter un ($C_1$-$C_7$)alkyle substitué par $R_{15}$ ; un groupe Ar ; un ($C_3$-$C_7$)cycloalkyle ; un adamantyle ;

- ou $R_{14}$ et $R_{24}$ ensemble avec l'atome d'azote auquel ils sont liés constituent un hétérocycle choisi parmi : la morpholine, la thiomorpholine, la pipérazine, l'azétidine, la pyrrolidine, la pipéridine ou la perhydroazépine, ledit hétérocycle étant non substitué ou substitué par un ou plusieurs groupes méthyles, par un phényle, par un groupe amino libre ou portant un groupe protecteur ;
- $R_{15}$ représente un groupe Ar ; un pyridyle ; un hydroxy ; un ($C_1$-$C_7$)alcoxy ; un groupe $-NR_{11}R_{27}$ ; un carboxy ; un ($C_1$-$C_7$)alcoxycarbonyle ;
- $R_{16}$ et $R_{17}$ représentent chacun indépendamment l'hydrogène ou un ($C_1$-$C_7$)alkyle ;
- ou bien $R_{16}$ et $R_{17}$ ensemble avec l'atome d'azote auquel ils sont liés constituent un hétérocycle choisi parmi la morpholine, la thiomorpholine, l'azétidine, la pyrrolidine, la pipéridine, la pipérazine substituée en position 4 par $R_{18}$, ou la perhydroazépine ;
- $R_{18}$ représente un hydrogène ; un ($C_1$-$C_7$)alkyle ; un phényle ; un benzyle ; un ($C_1$-$C_7$)alkylcarbonyle ; un formyle ; un benzoyle ; un ($C_1$-$C_7$)alcoxycarbonyle ; un phénoxycarbonyle ; un carbamoyle libre ou substitué par un ou deux ($C_1$-$C_7$)alkyles ;
- $R_{19}$ et $R_{20}$ représentent chacun indépendamment l'hydrogène ou un ($C_1$-$C_8$)alkyle ; $R_{20}$ peut de plus représenter un ($C_3$-$C_7$)cycloalkyle non substitué ou substitué par un ($C_1$-$C_4$)alkyle ; un groupe Ar ; un pyridyle ; un méthylpyridyle ; un pipérid-4-yle substitué en position 1 par $R_{18}$ ; un pipérid-1-yle ; un pyrrolidin-1-yle ; un morpholin-4-yle ; un thiazol-2-yle ; un indanyle ; un adamantyle ; un ($C_1$-$C_7$)alkyle substitué par un ou plusieurs halogènes ou par $R_{26}$ .
- ou bien $R_{19}$ et $R_{20}$ ensemble avec l'atome d'azote auquel ils sont liés constituent un radical hétérocyclique $R_{25}$ ;
- $R_{21}$ et $R_{22}$ représentent chacun indépendamment l'hydrogène ou un ($C_1$-$C_7$)alkyle ;
- ou bien $R_{21}$ et $R_{22}$ ensemble avec l'atome d'azote auquel ils sont liés constituent un radical hétérocyclique $R_{25}$ ;
- $R_{25}$ représente un morpholin-4-yle ; un thiomorpholine-4-yle ; un azétidin-1-yle non substitué ou substitué en position 3 par un groupe $-NR_{11}R_{27}$, un ($C_1$-$C_7$)alkyle, un phényle, un benzyle ou un ($C_1$-$C_7$)alkylcarbonyle ; un perhydroazépin-1-yle ; un pipérazin-1-yle non substitué ou substitué en position 4 par un ($C_1$-$C_7$)alkyle, un phényle, un benzyle, un ($C_1$-$C_7$)alkylcarbonyle, un ($C_1$-$C_7$)alcoxycarbonyle ou un benzyloxycarbonyle ; un pipérid-1-yle non substitué ou substitué en position 4 par un ($C_1$-$C_7$)alkyle, un phényle, un benzyle, un ($C_1$-$C_7$)alkylcarbonyle, un groupe $-NR_{11}R_{27}$ ; un pyrrolidin-1-yle non substitué ou substitué par un ($C_1$-$C_7$)alkyle, un phényle, un benzyle, un ($C_1$-$C_7$)alkylcarbonyle, un hydroxyméthyle, un carboxy, un ($C_1$-$C_7$)alcoxycarbonyle, un carbamoyle non substitué ou substitué par un ou deux ($C_1$-$C_7$)alkyles ;
- $R_{26}$ représente un hydroxy ; un ($C_1$-$C_7$)alcoxy ; un cyano ; un carboxy ; un ($C_1$-$C_7$)alcoxycarbonyle ; un benzyloxycarbonyle ; un groupe $-NR_{11}R_{27}$ ; un carbamoyle libre ou substitué par un ou deux ($C_1$-$C_7$)alkyles ; un pyrrolidin-1-ylcarbonyle ; un pipérid-1-ylcarbonyle ; un perhydroazépin-1-ylcarbonyle ; un groupe Ar ; un ($C_3$-$C_7$)cycloalkyle ; un adamantyle ; un radical hétérocyclique choisi parmi un pyridyle, un méthylpyridyle, un furanyle, un tétrahydrofuranyle, un thiényle, un méthylthiényle, un pyrrolidin-1-yle, un pipérid-1-yle, un perhydroazépin-1-yle ;
- Ar représente un phényle non substitué ou substitué une ou plusieurs fois par un substituant choisi parmi : un atome d'halogène, un ($C_1$-$C_7$)alkyle, un trifluorométhyle, un hydroxy, un ($C_1$-$C_7$)alcoxy, un carboxy, un ($C_1$-$C_7$)alcoxycarbonyle, un ($C_1$-$C_7$)alkylcarbonyloxy, un nitro, un cyano, un amino, un ($C_1$-$C_7$)alkylamino, un di($C_1$-$C_7$)alkylamino, lesdits substituants étant identiques ou différents ;
- t représente un nombre entier qui peut varier de 2 à 5 ;
- u représente un nombre entier qui peut varier de 0 à 5 ;
- m est 1 ou, lorsque $R_6$ est un halogène, un ($C_1$-$C_7$)alkyle ou un ($C_1$-$C_7$)alcoxy, m peut également être 2, 3 ou 4 ou bien $(R_6)_m$ peut représenter m substituants ayant différentes valeurs choisies parmi halogène, ($C_1$-$C_7$)alkyle ou ($C_1$-$C_7$)alcoxy ;

ainsi que leurs sels éventuels.

Avantageusement, l'invention concerne les composés de formule (I) dans laquelle :

- $R_1$ et $R_2$ représentent chacun indépendamment un hydrogène ; un halogène ; un hydroxy ; un ω-halogéno($C_1$-$C_7$)alcoxy ; un ($C_1$-$C_7$)alkyle ; un trifluorométhyle ; un ($C_1$-$C_7$)alcoxy ; un polyhalogéno($C_1$-$C_7$)alcoxy ; un ω-hydroxy($C_2$-$C_7$)alcoxy ; un ω-méthoxy($C_2$-$C_7$)alcoxy ; un ω-amino($C_2$-$C_7$)alcoxy dans lequel l'amino est libre ou substitué par un ou deux ($C_1$-$C_7$)alkyles ; un ($C_3$-$C_7$)cycloalkyloxy ; un ($C_3$-$C_7$)cycloalkylméthoxy ; un phénoxy ; un benzyloxy ; un ($C_1$-$C_7$)alkylthio ; un phénylthio ; un nitro ; un amino libre ou substitué par un ou deux ($C_1$-$C_7$)alkyles ; un cyano ; un formyle ; un ($C_1$-$C_6$)alkylcarbonyle ; un benzoyle ; un formyloxy ; un ($C_1$-$C_6$)alkyl-carbonyloxy ; un benzoyloxy ; un ($C_1$-$C_7$)alkylsulfonamido ; un phénylsulfonamido ; un benzylsulfonamido ; un ($C_1$-$C_7$) alkylcarbonylamino ; un ($C_1$-$C_7$) alcoxycarbonylamino ; un uréido non substitué ou substitué par un phényle, un benzyle ou par un ou deux ($C_1$-$C_7$)alky-

les ; un thiouréido non substitué ou substitué par un phényle, un benzyle ou par un ou deux $(C_1-C_7)$alkyles ;

- $R_3$ représente $R_4$ ; un $(C_1-C_7)$alkyle ; un $(C_1-C_7)$alkylène substitué par $R_4$ ;
- $R_4$ représente un groupe $-NR_{16}R_{17}$ ; un $(C_3-C_7)$cycloalkyle ; un phényle non substitué ou substitué une ou deux fois par un halogène, un $(C_1-C_7)$alkyle, un $(C_1-C_7)$alcoxy, un cyano, un trifluorométhyle ou un $(C_1-C_7)$alcoxycarbonyle ; un furyle ; un thiényle ; un triazolyle ; un tétrazolyle ; un pyridyle ; un pyrimidinyle ; un azétidin-3-yle ; un pipérid-4-yle ; un pyrrolidinyle ; un morpholinyle ;
- $R_5$ et $R_6$ représentent chacun indépendamment un hydrogène ; un halogène ; un $(C_1-C_7)$alkyle ; un trifluorométhyle ; un cyano ; un nitro ; un hydroxy ; un hydroxylamino ; un carboxy ; un guanidino non substitué ou substitué une ou deux fois par un $(C_1-C_7)$alkyle, un phényle ou un benzyle ; un groupe $-OR_7$; un groupe $-SR_7$ ; un $(C_1-C_6)$alkylcarbonyle ; un formyle ; un benzoyle ; un $(C_1-C_7)$alcoxycarbonyle ; un phénoxycarbonyle ; un benzyloxycarbonyle ; un groupe $-CONR_{19}R_{20}$ ; un groupe $-CSNR_{11}R_{27}$ ; un groupe $-SO_2NR_{21}R_{22}$ ; un $(C_1-C_7)$alkylsulfonamido ; un benzylsulfonamido ; un groupe -NHSO2Ar ; un groupe $-NR_8R_9$; un groupe $-CO-NH-CR_{10}R_{23}-CO-R_{12}$.
- $R_7$ représente un $(C_1-C_7)$alkyle ; un phényle ; un benzyle ; un $(C_3-C_7)$cycloalkyle ; un $(C_2-C_7)$alcényle ; un ω-halogéno$(C_2-C_7)$alkyle ; un polyhalogéno$(C_1-C_7)$alkyle ; un $(C_1-C_6)$alkylcarbonyle ; un formyle ; un benzoyle ; un ω-carboxy$(C_1-C_7)$alkyle ; un ω-$(C_1-C_7)$alcoxycarbonyl$(C_1-C_7)$alkyle ; un ω-benzyloxycarbonyl$(C_1-C_7)$alkyle ; un ω-amino$(C_2-C_7)$alkyle dans lequel le groupe amino est libre ou substitué par un ou deux $(C_1-C_7)$alkyles, ou sous forme d'ion ammonium ; un ω-carbamoyl$(C_1-C_7)$alkyle dans lequel le carbamoyle est libre ou substitué par un ou deux $(C_1-C_7)$alkyles ;
- $R_5$ et $R_9$ représentent chacun indépendamment un hydrogène ; un $(C_1-C_7)$alkyle ; un groupe $-CH_2Ar$ ; $R_9$ peut de plus représenter un groupe Ar ; un $(C_3-C_8)$alcényle ; un $(C_1-C_6)$alkylcarbonyle ; un formyle ; un $(C_1-C_6)$alkylthiocarbonyle ; un $(C_3-C_7)$cycloalkylcarbonyle ; un $(C_3-C_7)$cycloalkylthiocarbonyle ; un ω-amino$(C_2-C_6)$alkylcarbonyle ; un ω-hydroxy$(C_1-C_6)$alkylcarbonyle ; un ω-benzyloxy $(C_1-C_6)$alkylcarbonyle ; un pyridylcarbonyle ; un méthylpyridylcarbonyle ; un thiénylcarbonyle ; un groupe COAr ; un groupe $-COCH_2Ar$ ; un $(C_1-C_7)$alcoxycarbonyle ; un phénoxycarbonyle ; un groupe $-COCR_{10}R_{23}NR_{11}R_{27}$ ; un groupe $-CR_{10}R_{23}COR_{12}$; un groupe $-(CH_2)_tCOR_{12}$ ; un groupe $-CO(CH_2)_uCOR_{12}$ ; un groupe $-CONR_{14}R_{24}$ ; un groupe $-CSNR_{14}R_{24}$ ; un radical hétérocyclique choisi parmi pyrazolyle, imidazolyle, triazolyle, tétrazolyle, pyridyle, pyridazinyle, pyrimidinyle, thiazolyle ;
- ou bien $R_5$ et $R_9$ ensemble avec l'atome d'azote auquel ils sont liés constituent l'hydantoïne, la N-méthylhydantoïne, ou un radical hétérocyclique choisi parmi le pyrrol-1-yle, le Δ3-pyrrolin-1-yle, le pyrrolidin-1-yle, l'isoindolin-2-yle dans lequel le noyau benzénique est non substitué ou substitué par un halogène, un $(C_1-C_7)$alkyle, un trifluorométhyle ou un méthoxy ;
- $R_{10}$ et $R_{23}$ représentent chacun indépendamment l'hydrogène ; un $(C_1-C_7)$alkyle ; un groupe $-CH_2Ar$ ;
- ou bien $R_{10}$ et $R_{23}$ ensemble avec l'atome de carbone auquel ils sont liés constituent un $(C_3-C_7)$cycloalkyle ;
- $R_{11}$ et $R_{27}$ représentent chacun indépendamment l'hydrogène ou un $(C_1-C_7)$alkyle ;
- $R_{12}$ représente un hydroxy ; un $(C_1-C_7)$alcoxy ; un amino libre ou substitué par un ou deux $(C_1-C_7)$alkyles ;
- $R_{14}$ et $R_{24}$ représentent chacun indépendamment l'hydrogène ; un $(C_1-C_7)$alkyle ; $R_{24}$ peut de plus représenter un $(C_1-C_7)$alkyle substitué par $R_{15}$ ; un phényle non substitué ou substitué par un hydroxy ou un amino libre ou substitué par un ou deux $(C_1-C_7)$alkyles ; un $(C_3-C_7)$cycloalkyle ; un adamantyle ;
- ou $R_{14}$ et $R_{24}$ ensemble avec l'atome d'azote auquel ils sont liés constituent un hétérocycle choisi parmi : la morpholine, la thiomorpholine, la pipérazine, l'azétidine, la pyrrolidine ou la pipéridine, ledit hétérocycle étant non substitué ou substitué par un ou plusieurs groupes méthyles, par un phényle, par un groupe amino libre ou portant un groupe protecteur ;
- $R_{15}$ représente un groupe Ar ; un pyridyle ; un hydroxy ; un $(C_1-C_7)$alcoxy ; un groupe $-NR_{11}R_{27}$ ; un carboxy ; un $(C_1-C_7)$alcoxycarbonyle ;
- $R_{16}$ et $R_{17}$ représentent chacun indépendamment l'hydrogène ou un $(C_1-C_7)$alkyle ;
- ou bien $R_{16}$ et $R_{17}$ ensemble avec l'atome d'azote auquel ils sont liés constituent un hétérocycle choisi parmi la morpholine, la thiomorpholine, l'azétidine, la pyrrolidine, la pipéridine ou la pipérazine ;
- $R_{19}$ et $R_{20}$ représentent chacun indépendamment l'hydrogène ou un $(C_1-C_7)$alkyle ; $R_{20}$ peut de plus représenter un groupe Ar ; un pyridyle ; un méthylpyridyle ; un pipérid-4-yle ; un $(C_1-C_7)$alkyle substitué par un ou plusieurs halogènes ou par $R_{26}$.
- ou bien $R_{19}$ et $R_{20}$ ensemble avec l'atome d'azote auquel ils sont liés constituent un radical hétérocyclique $R_{25}$ ;
- $R_{21}$ et $R_{22}$ représentent chacun indépendamment l'hydrogène ou un $(C_1-C_7)$alkyle ;
- ou bien $R_{21}$ et $R_{22}$ ensemble avec l'atome d'azote auquel ils sont liés constituent un radical hétérocycli-

que $R_{25}$ ;

- $R_{25}$ représente un azétidin-1-yle non substitué ou substitué en position 3 par un groupe amino, un ($C_1$-$C_7$)alkyle, un phényle, un benzyle, un formyle ou un ($C_1$-$C_6$)alkylcarbonyle ; un pipérazin-1-yle non substitué ou substitué en position 4 par un ($C_1$-$C_7$)alkyle, un phényle, un benzyle, un formyle ou un ($C_1$-$C_6$)alkylcarbonyle ; un pipérid-1-yle non substitué ou substitué en position 4 par un ($C_1$-$C_7$)alkyle, un phényle, un benzyle, un formyle, un ($C_1$-$C_6$)alkylcarbonyle, un amino libre ou portant un groupe protecteur ; un pyrrolidin-1-yle non substitué ou substitué par un ($C_1$-$C_7$)alkyle, un phényle, un benzyle, un formyle, un ($C_1$-$C_6$)alkylcarbonyle, un carboxy, un ($C_1$-$C_7$)alcoxycarbonyle ;
- $R_{26}$ représente un hydroxy ; un ($C_1$-$C_7$)alcoxy ; un cyano ; un carboxy ; un ($C_1$-$C_7$)alcoxycarbonyle ; un benzyloxycarbonyle ; un groupe -$NR_{11}R_{27}$ ; un carbamoyle libre ou substitué par un ou deux ($C_1$-$C_7$)alkyles ; un pyrrolidin-1-ylcarbonyle ; un pipérid-1-ylcarbonyle ; un groupe Ar ; un ($C_3$-$C_7$)cycloalkyle ; un adamantyle ; un radical hétérocyclique choisi parmi un pyridyle, un méthylpyridyle, un furanyle, un tétrahydrofuranyle, un thiényle, un méthylthiényle, un pyrrolidin-1-yle, un pipérid-1-yle ;
- Ar représente un phényle non substitué ou substitué une ou plusieurs fois par un substituant choisi parmi : un atome d'halogène, un ($C_1$-$C_7$)alkyle, un trifluorométhyle, un ($C_1$-$C_7$)alcoxy, un carboxy, un ($C_1$-$C_7$)alcoxycarbonyle, un formyloxy, un ($C_1$-$C_6$)alkylcarbonyloxy ;
- t représente un nombre entier qui peut varier de 2 à 5 ;
- u représente un nombre entier qui peut varier de 1 à 5 ;
- m est 1 ou, lorsque $R_6$ est un halogène, un ($C_1$-$C_7$)alkyle ou un ($C_1$-$C_7$)alcoxy, m peut également être 2, 3 ou 4 ou bien $(R_6)_m$ peut représenter m substituants ayant différentes valeurs choisies parmi halogène, ($C_1$-$C_7$)alkyle ou ($C_1$-$C_7$)alcoxy ;

ainsi que ses sels éventuels.

Lorsqu'un composé selon l'invention présente un ou des carbones asymétriques, l'invention comprend tous les isomères optiques de ce composé.

Les sels des composés de formule (I) selon la présente invention comprennent ceux avec des acides minéraux ou organiques qui permettent une séparation ou une cristallisation convenable des composés de formule (I), tels que l'acide picrique, l'acide oxalique ou un acide optiquement actif, par exemple un acide mandélique ou un acide camphosulfonique, et ceux qui forment des sels physiologiquement acceptables tels que le chlorhydrate, le bromhydrate, le sulfate, l'hydrogénosulfate, le dihydrogénophosphate, le maléate, le fumarate, le 2-naphtalènesulfonate.

Les sels des composés de formule (I) comprennent également les sels avec des bases organiques ou minérales, par exemple les sels des métaux alcalins ou alcalino-terreux comme les sels de sodium, de potassium, de calcium, les sels de sodium et de potassium étant préférés, ou avec une amine, telle que le trométamol, ou bien les sels d'arginine, de lysine, ou de toute amine physiologiquement acceptable.

Selon la présente invention, par halogène on entend un atome choisi parmi le fluor, le chlore, le brome ou l'iode, de préférence le fluor ou le chlore.

Selon la présente invention par groupe protecteur de l'azote on entend un groupe tel que : un ($C_1$-$C_7$)alkyle, par exemple un méthyle ou un *tert*-butyle ; un benzyle ; un benzyle substitué tel que p-nitrobenzyle, p-chlorobenzyle, p-méthoxybenzyle ; un benzhydryle ; un trityle ; un benzoyle ; un ($C_1$-$C_4$)alkylcarbonyle, par exemple un acétyle ; un ($C_1$-$C_4$)alcoxycarbonyle, par exemple un méthoxycarbonyle, un éthoxycarbonyle ou un *tert*-butoxycarbonyle ; un benzyloxycarbonyle.

Selon la présente invention par alkyle en $C_1$-$C_7$ ou respectivement en $C_1$-$C_8$ ou en $C_1$-$C_4$ on entend un alkyle droit ou ramifié en $C_1$-$C_7$ ou respectivement en $C_1$-$C_8$ ou en $C_1$-$C_4$. Par alcoxy en $C_1$-$C_7$ ou respectivement en $C_1$-$C_4$ on entend un alcoxy droit ou ramifié en $C_1$-$C_7$ ou respectivement en $C_1$-$C_4$.

Par convention, dans la description qui va suivre et dans les revendications, l'hétérocycle 1,3-dihydro-2*H*-benzimidazol-2-one est numéroté comme suit pour les composés selon l'invention.

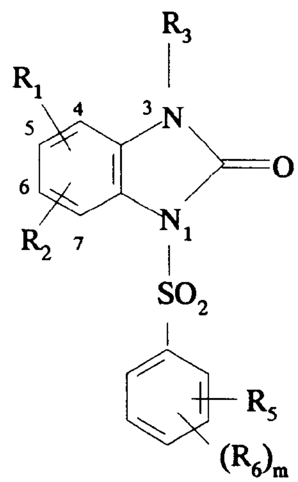

(I)

De façon avantageuse, la présente invention a pour objet des composés de formule :

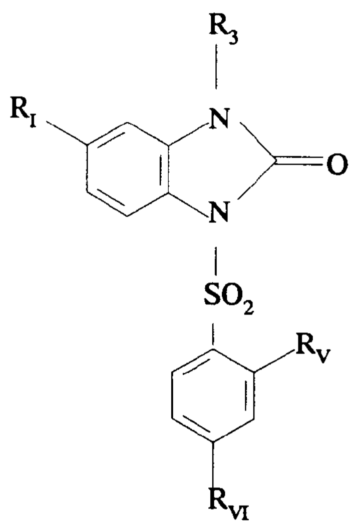

($I_a$)

dans laquelle :

- $R_I$ représente un ($C_1$-$C_4$)alcoxy, un atome de chlore ou de fluor,
- $R_V$ représente l'hydrogène ou un méthoxy,
- $R_{VI}$ représente un ($C_1$-$C_7$)alkylcarboxamido, un groupe -NHCO-Ar, un groupe $-CONR_{19}R_{20}$, un groupe $-NR_8CONR_{14}R_{24}$, un ($C_1$-$C_7$)alcoxy, un amino libre ou substitué par un ou deux ($C_1$-$C_7$)alkyles ;
- les substituants $R_3$, Ar, $R_8$, $R_{19}$, $R_{20}$ $R_{14}$, $R_{24}$ sont tels que définis ci-dessus pour les composés de formule (I) ;

ainsi que leurs sels.

De façon toute particulière, on préfère les composés de formule

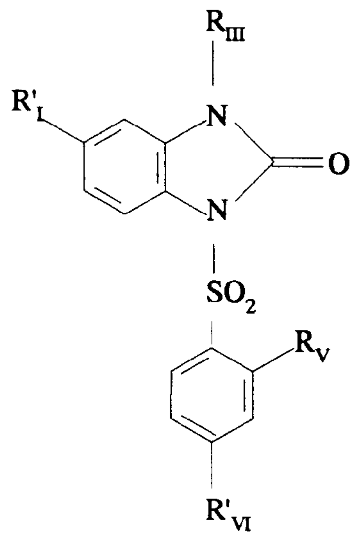

$(I_b)$

dans laquelle

- $R'_I$ représente un éthoxy ou un chlore ;
- $R_{III}$ représente un cyclohexyle ou un groupe Ar ;
- $R_V$ représente l'hydrogène ou un méthoxy ;
- $R'_{VI}$ représente un groupe $-CONR_{19}R_{20}$ ou un groupe $-NR_8CONR_{14}R_{24}$ ;
- les substituants Ar, $R_{19}$, $R_{20}$, $R_8$, $R_{14}$, $R_{24}$ sont tels que définis ci-dessus pour les composés de formule (I) ;

ainsi que leurs sels.

Dans la description et dans les exemples, les abréviations suivantes sont utilisées :

DCM : dichlorométhane
Ether : éther diéthylique
Ether iso : éther diisopropylique
MeOH : méthanol
EtOH : éthanol
Me, OMe : méthyle, méthoxy
Et, OEt : éthyle, éthoxy
iPr, nPr : isopropyle, n-propyle
nBu, iBu, tBu : n-butyle, isobutyle, *tert*-butyle
Ph : phényle
Bz : benzyle
Ac : acétyle
AcOEt : acétate d'éthyle
AcOH : acide acétique
HCl : acide chlorhydrique
DMF : diméthylformamide
THF : tétrahydrofuranne
DMSO : diméthylsulfoxyde
DIPEA : diisopropyléthylamine
NaOH : soude
$NaHCO_3$ : hydrogénocarbonate de sodium
$Na_2SO_4$ : sulfate de sodium
BOP : benzotriazol-1-yloxy-tris(diméthylamino)phosphonium hexafluorophosphate
Réactif de Lawesson : 2,4-bis-(4-méthoxyphényl)-1,3-dithia-2,4-diphosphétane-2,4-disulfure
F : point de fusion
Hg : mercure
CCM : chromatographie en couche mince
HPLC : chromatographie liquide à haute pression
Eau chlorhydrique : acide chlorhydrique dilué, environ 1N
Eb : température d'ébullition
RMN : résonance magnétique nucléaire

s : singulet
t : triplet
q : quadruplet
m : massif
TA : température ambiante

La présente invention a également pour objet un procédé de préparation des composés selon l'invention et de leurs sels, caractérisé en ce que :

1/ on fait agir sur un composé de formule :

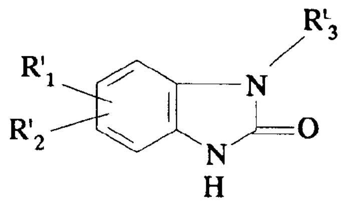
(II)

dans laquelle $R'_1$, $R'_2$ et $R'_3$ représentent, respectivement, soit $R_1$, $R_2$ et $R_3$ tels que définis pour (I), soit des groupes précurseurs de $R_1$, $R_2$ et $R_3$, un halogénure de benzènesulfonyle de formule :

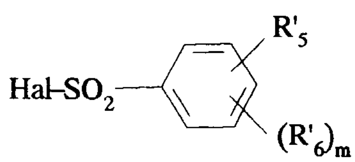
(III)

dans laquelle $R'_5$ et $R'_6$ représentent, respectivement, soit $R_5$ et $R_6$ tels que définis ci-dessus pour (I), soit des groupes précurseurs de $R_5$ et $R_6$ ; et,

2/ soit, lorsque $R'_1 = R_1$, $R'_2 = R_2$, $R'_3 = R_3$, $R'_5 = R_5$ et $R'_6 = R_6$, on isole le composé de formule (I) ainsi obtenu ;

3/ soit, lorsque l'un quelconque des groupes $R'_1$, $R'_2$, $R'_3$,$R'_5$ et/ou $R'_6$ représente respectivement un groupe précurseur de $R_1$, $R_2$, $R_3$, $R_6$ et/ou $R_6$, on soumet le composé obtenu, ci-après dénommé composé (I'), à un traitement ultérieur pour préparer le composé de formule (I) par transformation de l'un quelconque des groupes $R'_1$, $R'_2$, $R'_3$, $R'_5$ et/ou $R'_6$ en, respectivement, $R_1$, $R_2$, $R_3$, $R_6$ et/ou $R_6$ ;

4/ éventuellement, on transforme le composé obtenu à l'étape 2) ou à l'étape 3) en l'un de ses sels.

La transformation d'un substituant $R'_1$, $R'_2$, $R'_3$, $R'_5$ et/ou $R'_6$ en respectivement $R_1$, $R_2$, $R_3$, $R_6$ et/ou $R_6$ peut être effectuée soit à partir du composé de formule (I') soit à partir d'un des composés intermédiaires utiles dans la préparation de (I).

La réaction de l'étape 1 est effectuée dans un solvant anhydre tel que le DMF ou le THF, en présence d'un hydrure métallique tel que l'hydrure de sodium par exemple, ou en présence d'un alcoolate comme le tert-butylate de potassium.

Les dérivés de benzimidazol-2-ones (II) sont connus ou peuvent être préparés selon différents modes opératoires par des méthodes connues.

On peut préparer des dérivés de benzimidazol-2-ones N-substitués (II), utiles comme produits de départ pour la préparation des composés selon l'invention, d'après des procédés décrits dans les brevets GB 2 127 408, GB 1 575 386 ; demandes de brevet JP 62-249 982, JP 53-009 770, JP 51-131 875 ; brevets BE 770 911, BE 859 415, BE 830 403 ; demandes de brevet EP 477 819, EP 454 330, EP 526 434.

De même les publications suivantes décrivent des dérivés de benzimidazol-2-ones N-substitués :

Monatsh. Chem., 1985, 116 (5), 639-644.

Eur. J. Med. Chem. - Chim. Ther., 1983, 18 (6), 495-500.

On peut également préparer des dérivés de benzimidazol-2-ones N-substitués par des méthodes telles que celles décrites dans les publications suivantes:

Pharmazie, 1979, 34 (9), 576.

Pol. J. Chem., 1979, 53 (9), 1883-1887.

J. Heterocycl. Chem., 1970, 7 (4), 807-813.

Eur. J. Med. Chem. - Chim. Ther., 1981, 16 (4), 321-326.

Selon un mode de réalisation particulier, les benzimidazolones peuvent être préparées selon le procédé décrit dans Eur. J. Med. Chem., 1981, 16 (4), 321-326, de la manière suivante.

Par réaction en solution alcoolique, en présence ou non d'une base telle que la triéthylamine, à température ambiante ou à reflux, d'une amine primaire de formule :

$H_2N$ - $R'_3$ (IV)

avec des ortho-dinitro ou ortho-chloro-nitrobenzènes (V) substitués de formules

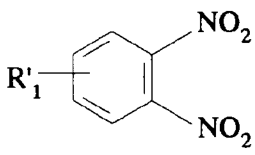

ou

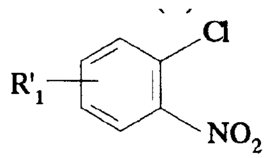

(V)

dans lesquelles $R'_1$ est différent d'un groupe nitro,

on obtient les 2-nitroanilines N-substituées de formule :

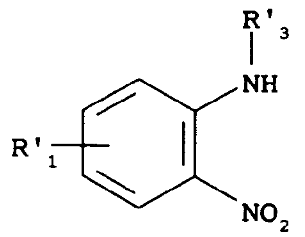

(VI)

Les composés (VI) peuvent également être obtenus par chauffage des composés (IV) et (V) dans le 2-éthoxyéthanol (J. Chem. Soc. 1960, 314-318) ou dans l'éthylèneglycol en présence d'acétate de sodium ou dans le 1,2,3,4-tétraméthylbenzène ou dans la Décaline®.

Les composés (VI) dans lesquels R'1 est un groupe ($C_1$-$C_7$)alcoxy, ($C_3$-$C_7$)cycloalkyloxy, ($C_3$-$C_7$)cycloalkylméthoxy, phénoxy, benzyloxy ou ω-méthoxy($C_2$-$C_7$)alcoxy sont obtenus par réaction d'un composé (VI) dans lequel $R'_1$ = Cl avec un alcoolate de sodium selon le procédé décrit dans J. Org. Chem. 1963, 28, 3117 ou avec un alcoolate de sodium en présence d'un catalyseur de transfert de phase tel que tris[2-(2-méthoxyéthoxy)éthyl]amine(TDA-1).

Les composés (VI) dans lesquels $R'_3$ est un groupe ($C_1$-$C_8$)alkylène substitué par un ($C_1$-$C_4$)alcoxy peuvent être préparés par réaction d'un composé (VI) dans lequel le substituant $R'_3$ est un ($C_1$-$C_8$)alkyle substitué par un hydroxy avec un halogéno ($C_1$-$C_4$)alkyle, en présence d'un hydrure métallique tel que l'hydrure de sodium par exemple, dans un solvant anhydre tel que le DMF ou le THF.

Les composés de formule (VI) sont réduits en ortho-phénylénediamines N-substituées de formules (VII) :

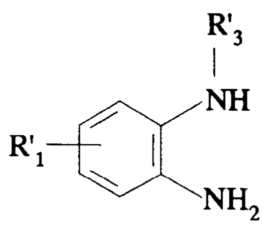

(VII)

La réduction peut être catalytique en utilisant par exemple le palladium sur charbon ou le nickel de Raney®, ou chimique en utilisant le fer, le zinc ou l'étain dans des conditions acides (J. Chem. Soc. 1960, 314).

Les composés de formule (VII), par réaction avec le chloroformiate d'éthyle ou le chloroformiate de méthyle dans un solvant tel que le chloroforme ou le dichlorométhane, en présence ou non d'une base telle que la triéthylamine, ou dans le mélange DMF/eau en présence de carbonate de potassium, conduisent aux composés de formule (VIII) et/ou (VIII'):

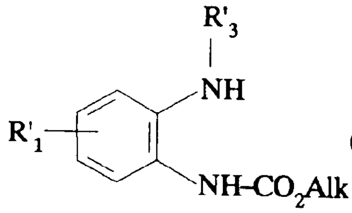 (VIII) et/ou 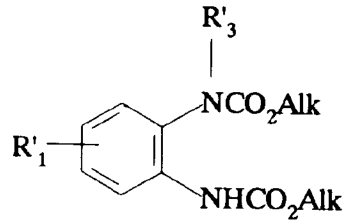 (VIII')

dans lesquelles Alk représente un éthyle ou un méthyle.

Les composés de formule (VIII) et/ou (VIII') sont cyclisés en benzimidazol-2-one (IX) par chauffage avec l'éthylate de sodium.

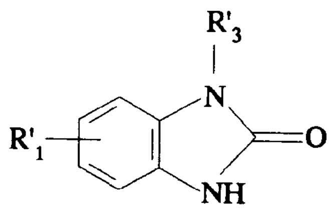 (IX)

Les composés (IX) peuvent également être obtenus par réaction d'un composé de formule (VII) avec de l'urée selon le procédé décrit dans J. Chem. Soc. 1960, 314 ou avec du 1,1'-carbonyldiimidazole selon un procédé décrit dans le brevet européen 92391.

Les composés de formule (II) portant certains substituants $R'_1$, $R'_2$ sur leur partie benzénique sont utilisés comme précurseurs pour la préparation de composés de formule (II) portant d'autres substituants $R'_1$, $R'_2$. Par exemple, les composés (II) dans lesquels $R'_1$ et/ou $R'_2$ = H peuvent être nitrés par des réactifs classiques. Ils peuvent également être acylés par action d'un chlorure d'acide de formule RCOCl dans lequel R représente un ($C_1$-$C_7$)alkyle, ou un phényl en présence d'un acide de Lewis tel que le chlorure d'aluminium pour préparer un composé (II) dans lequel $R'_1$ et/ou $R'_2$ = COR. A partir d'un composé (II) dans lequel $R'_1$ est un groupe nitro et $R'_2$ est l'hydrogène, on prépare par hydrogénation catalytique ou par réduction chimique le composé (II) dans lequel $R'_1$ est un groupe amino.

Dans le cas particulier où $R'_3$ représente un azétidin-3-yle, un pipérid-4-yle, un pipérazin-1-yle ou un groupe ($C_1$-$C_7$)alkylène substitué par un azétidin-3-yle, un pipérid-4-yle, un pipérazin-1-yle dans lequel l'atome d'azote est substitué par $R_{18}$ = un formyle, un ($C_1$-$C_7$)alkylcarbonyle, un benzoyle, un ($C_1$-$C_7$)alcoxycarbonyle, un phénoxycarbonyle ou un carbamoyle non substitué ou substitué par un ou deux ($C_1$-$C_7$)alkyles, la substitution sur l'atome d'azote peut être réalisée soit sur le composé benzimidazol-2-one (II), soit sur le composé final (I) à partir d'un composé dans lequel l'atome d'azote n'est pas substitué ($R_{18}$ = H). Ainsi lorsque l'atome d'azote est substitué par $R_{18}$ = un formyle, un($C_1$-$C_7$)alkylcarbonyle ou un benzoyle, on fait agir sur un composé (II) ou un composé (I) dans lequel l'atome d'azote du radical hétérocyclique tel que défini ci-dessus est non substitué ($R_{18}$ = H), l'acide formique en présence d'anhydride acétique ou respectivement un chlorure d'acide ou un anhydride. Lorsque l'atome d'azote est substitué par $R_{18}$ = un ($C_1$-$C_7$)alcoxycarbonyle ou un phénoxycarbonyle, on fait agir sur un composé (II) ou un composé (I) dans lequel $R_{18}$ = H le chloroformiate approprié. Par action de l'ammoniac sur un composé de formule (II) ou un composé de formule (I) dans lequel $R_{18}$ = phénoxycarbonyle, on prépare un composé (II) ou un composé (I) dans lequel $R_{18}$ est un carbamoyle ; par action sur un tel composé d'une mono ou dialkylamine en ($C_1$-$C_7$), on prépare un composé de formule (II) ou un composé de formule (I) dans lequel $R_{18}$ est un N-($C_1$-$C_7$)alkylcarbamoyle ou N,N-di($C_1$-$C_7$)alkylcarbamoyle. On peut également préparer un composé (II) ou un composé (I) dans lequel $R_{18}$ est un N-alkylcarbamoyle par action d'un isocyanate d'alkyle sur un composé (II) ou un composé (I) dans lequel $R_{18}$ = H.

Les composés de formule (IV) sont connus ou peuvent être préparés par des méthodes connues. Par exemple les cyclohexylamines différemment substituées sont préparées selon J. Org. Chem., 1962, 27, 3568-3572.

Les halogénures de benzènesulfonyle (III) sont préparés par des méthodes connues. Ainsi par exemple, le chlorure de 4-diméthylaminobenzènesulfonyle est préparé selon C.N. Sukenik et al., J. Amer. Chem. Soc., 1977, 99, 851-858. Plus généralement, les halogénures de benzènesulfonyle (III) dans lesquels le substituant $R'_6$ est un groupe diméthylamino sont connus ou préparés par des méthodes connues; le chlorure de p-benzyloxybenzènesulfonyle est préparé selon la demande de brevet européen EP 229 566.

Le chlorure d'alcoxybenzènesulfonyle est préparé à partir de l'alcoxybenzènesulfonate de sodium, lui-même préparé par action d'un halogénure d'alkyle sur l'hydroxybenzènesulfonate de sodium.

Le chlorure de 2,4-diméthoxybenzènesulfonyle est préparé selon J. Am. Chem. Soc., 1952, 74. 2008.

On peut préparer les chlorures d'halogénoalcoxybenzènesulfonyle selon le brevet US 2 540 057.

Les halogénures de benzènesulfonyle de formule :

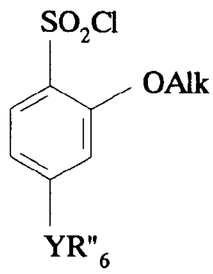

(III')

dans laquelle

- Alk représente un ($C_1$-$C_7$)alkyle ;
- Y représente O ou S ;
- R"$_6$ représente un ($C_1$-$C_7$)alkyle, un ($C_3$-$C_7$)cycloalkyle, un ($C_2$-$C_7$)alcényle, un ω-halogéno($C_2$-$C_7$)alkyle, un polyhalogéno($C_1$-$C_7$)alkyle, un benzyle, un ($C_1$-$C_7$)alkylcarbonyle, un formyle, un benzoyle, un ω-carboxy($C_1$-$C_7$)alkyle estérifié par un alkyle en $C_1$-$C_7$ ou par un benzyle,

sont préparés d'après D. Hofmann et al. dans Liebigs Ann. Chem., 1982, 282-297.

On opère sur des composés benzéniques portant les substituants YR"6 et OAlk en position -1,3 par action du chlorosulfonate de triméthylsilyle dans un solvant tel que le DCM à TA. On applique ensuite la méthode de R. Passerini et al. dans Gazz. Chim. Ital., 1960, 90, 1277-89 et on neutralise ensuite, par exemple par du carbonate alcalin, puis on fait agir un halogénure tel que $POCl_3$ pour obtenir l'halogénure de benzènesulfonyle souhaité.

Les halogénures de benzènesulfonyle (III) dans lesquels le substituant R'6 représente un alcoxycarbonyle, un phénoxycarbonyle, un benzyloxycarbonyle, un alkylthio, un phénylthio, un benzylthio ou un groupe $SR_7$, $R_7$ étant tel que défini pour (I), sont préparés selon Col. Czechoslov. Chem. Commun., 1984, 49, 1184, à partir d'un dérivé de l'aniline substitué par le même groupement R'6, ledit dérivé de l'aniline étant lui-même obtenu à partir du dérivé nitré correspondant.

Les dérivés de l'acide nitrobenzoïque sont connus ; par une réaction appropriée d'estérification de cet acide, on obtient les esters d'alkyle et de phényle correspondants.

Les dihalogénures de benzène di-sulfonyle (III, $R'_6$ = $SO_2$Hal) sont connus ou préparés par des méthodes connues. Par exemple, le dichlorure de 2,4-diméthoxybenzène-1,5-disulfonyle est décrit dans R.J.W.Cremlyn, J. Chem. Soc. C, 1969, 1341-1345.

Les chlorures d'halogénoalcoxybenzènesulfonyle (III, $R'_6$ = ω-halogénoalcoxy) sont utilisés pour la préparation de composés selon l'invention dans lesquels le substituant $R_6$ est un ω-aminoalcoxy non substitué ou substitué par un ou deux alkyles, selon le schéma suivant:

$$- O - Alk' - Hal \; + \; NHAA' \rightarrow \; - O - Alk' - NAA'$$

dans lequel Alk' représente un ($C_2$-$C_7$)alkyle et A et A' représentent chacun indépendamment l'hydrogène ou un ($C_1$-$C_7$)alkyle.

Les halogénures de benzènesulfonyle (III) dans lesquels $R'_6$ en position 4 représente un sulfamoyle substitué par $R_{21}$ et $R_{22}$ ($R'_6$ = $-S0_2NR_{21}R_{22}$) et $R'_5$ en position 2 représente un alcoxy en $C_1$-$C_7$ peuvent être préparés selon le procédé suivant: on prépare des halogénures de 3-alcoxy-4-nitrobenzènesulfonyle par action de l'acide chlorosulfonique sur des composés 2-alcoxynitrobenzène et on fait réagir le chlorure de sulfonyle ainsi obtenu avec des composés $HNR_{21}R_{22}$. On obtient les halogénures de benzènesulfonyle correspondants selon Col. Czechoslov. Chem. Commun., 1984, 49, 1184, à partir des dérivés de l'aniline substitués par le même groupement, lesdits dérivés de l'aniline étant eux-mêmes obtenus à partir des dérivés nitrés correspondants.

L'halogénure de benzènesulfonyle (III) dans lequel $R'_6$ en position 4 représente un groupe N'N'-diéthyluréido[$R'_6$ = $-NHCON(Et)_2$] peut être préparé par action de l'acide chlorosulfonique sur la N'N'-diéthyl-N-phénylurée, elle-même obtenue par réaction de l'aniline avec le chlorure de diéthylcarbamoyle.

Le chlorure de 4-(morpholin-4-yl)benzènesulfonyle est préparé selon R.J. Cremlyn et coll., dans Phosphor. Sulfur. Silicon., 1992, 73 (1-4), 107-120.

Pour certaines valeurs des substituants $R_1$, $R_2$, $R_3$, $R_5$ et/ou $R_6$ les composés selon l'invention (I) peuvent être préparés à partir d'un précurseur de formule (I') substitué par un groupe $R'_1$, $R'_2$, $R'_3$, $R'_5$ et/ou $R'_6$ appelé

groupe précurseur de $R_1$, $R_2$, $R_3$, $R_5$ et/ou $R_6$ en utilisant des méthodes connues de l'homme de l'art.

Dans la description qui va suivre, on expose la préparation des composés de formule (I) portant des substituants $R_1$ et/ou $R_5$ ; les mêmes méthodes s'appliquent à la préparation des composés dans lesquels les substituants $R_2$ et/ou $R_5$ ont les valeurs indiquées pour $R_1$ et/ou $R_6$.

Les composés (I) dans lesquels $R_1$ et/ou $R_6$ est un hydroxy peuvent être obtenus par hydrogénation catalytique, par exemple en présence de palladium sur charbon, d'un composé de formule (I') dans lequel $R'_1$ et/ou $R'_6$ est un benzyloxy. Ces composés peuvent également être préparés à partir de composés analogues de formule (I') dans lesquels $R'_1$ et/ou $R'_6$ représente un groupe amino en utilisant la méthode décrite dans J. Org. Chem., 1977, 42, 2053.

Les composés de formule (I) dans lesquels $R_1$ et/ou $R_6$ représente un ($C_1$-$C_7$) alcoxy peuvent être préparés directement par le procédé selon l'invention à partir des composés de formule (II) et (III) correctement substitués.

Les composés (I') dans lesquels $R'_1$ et/ou $R'_6$ représente un hydroxy permettent également de préparer des composés (I) dans lesquels $R_1$ et/ou $R_6$ est un ($C_1$-$C_7$)alcoxy par action d'un halogénure d'alkyle en $C_1$-$C_7$ en présence d'une base telle qu'un hydrure métallique ou un carbonate alcalin ou alcalino-terreux comme $K_2CO_3$ ou $Cs_2CO_3$ dans un solvant tel que le THF ou le DMF. De même, on prépare les composés de formule (I) dans lesquels $R_1$ et/ou $R_5$ représente un ω-aminoalkyloxy par action d'une ω-chloroalkylamine sur les composés dans lesquels $R'_1$ et/ou $R'_6$ = OH ; de même on prépare les composés dans lesquels $R_1$ et/ou $R_5$ représente un ω-hydroxyalcoxy par action d'un chloroalkylalcool; dans le cas particulier de la préparation d'un composé (I) dans lequel $R_1$ et/ou $R_6$ = $O(CH_2)_2OH$, on peut également faire agir le carbonate d'éthylène sur un composé (I') dans lequel $R'_1$ et/ou $R'_6$ = OH.

Les composés de formule (I) dans lesquels $R_1$ et/ou $R_5$ représente un formyloxy ou respectivement un ($C_1$-$C_7$)alkylcarbonyloxy ou un benzoyloxy sont obtenus par action de l'acide formique dans le dicyclohexylcarbodiimide (Huang et al., J. Chem. Res. (S), 1991, 292-293) ou respectivement d'un halogènure d'acide ou d'un anhydride sur un composé (I') dans lequel $R'_1$ et/ou $R'_6$ est un hydroxy.

Les composés de formule (I) dans lesquels $R_6$ est un groupe $OR_7$, $R_7$ représentant un ω-carbamoyl($C_1$-$C_7$)alkyle libre ou substitué par un ou deux alkyles en $C_1$-$C_7$ peuvent être préparés à partir d'un composé (I') dans lequel $R'_6$ représente un groupe $OR''_6$, $R''_6$ représentant un ω-carboxy($C_1$-$C_7$)alkyle estérifié par un alkyle en $C_1$-$C_7$. Cette préparation est effectuée de façon classique pour l'homme de l'art par action d'une amine correctement choisie.

Pour préparer des composés de formule (I) dans lesquels $R_1$ et/ou $R_6$ représente un ($C_1$-$C_7$) monoalkylamino, on fait réagir un composé de formule (I') dans lequel $R'_1$ et/ou $R'_6$ représente un groupe amino avec un aldéhyde ou une cétone, en milieu acide, en présence d'un agent réducteur tel que le cyanoborohydrure de sodium ; par une réaction identique on prépare les composés (I) dans lesquels $R_1$ et/ou $R_6$ représente un dialkylamino.

On peut préparer les composés de formule (I) dans lesquels $R_6$ représente un groupe amino substitué par un benzyle, lui même éventuellement substitué, ou par un alcène en $C_3$-$C_8$, par action d'un chlorure de benzyle ou d'un chloroalcène en $C_3$-$C_8$, sur un composé de formule (I') dans lequel $R'_6$ est un groupe amino ou ($C_1$-$C_7$)alkylamino.

Les composés de formule (I) dans lesquels $R_6$ représente un groupe Δ3-pyrrolin-1-yle se préparent par action, sous atmosphère inerte, du cis-1,4-dichlorobut-2-ène sur les composés de formule (I') dans lesquels $R'_6$ est un groupe amino en présence d'une base telle que la triéthylamine. Par hydrogénation on prépare ensuite les composés de formule (I) dans lesquels $R_6$ est un groupe pyrrolidin-1-yle.

La réaction du cis-1,4-dichlorobut-2-ène avec les composés (I') dans lesquels $R'_6$ est un groupe amino peut également s'effectuer à l'air en présence d'une base telle que $Na_2CO_3$ et conduit, dans ces conditions, à la formation d'un mélange d'un composé de formule (I) dans lequel $R_6$ représente un groupe Δ3-pyrrolin-1-yle et d'un composé de formule (I) dans lequel $R_5$ représente un groupe pyrrol-1-yle que l'on peut séparer par chromatographie.

Les composés de formule (I) dans lesquels $R_5$ représente un groupe isoindolin-2-yle se préparent par action de l'α,α'-dibromo-o-xylène sur les composés de formule (I') dans lesquels $R'_6$ est un groupe amino en présence d'une base telle que la triéthylamine, et dans un solvant tel que le diméthylformamide à reflux.

Les composés de formule (I) dans lesquels $R_5$ représente un groupe 1-méthyl-2,4-dioxoimidazolin-3-yle se préparent en deux étapes : on fait réagir la sarcosine sur un composé de formule (I') dans lequel $R'_6$ représente un phénoxycarboxamido, en présence d'une base telle que la triéthylamine, pour obtenir un composé de formule (I') dans lequel R'6 représente un N'-carboxyméthyl-N'-méthyluréido, puis par chauffage à 100°C, sous vide, le produit obtenu précédemment se cyclise.

Lorsque $R'_1$ et/ou $R'_6$ représente un amino, on peut également effectuer une nitrosation, par exemple en présence d'acide nitreux ou de nitrite de sodium pour préparer un composé (I') dans lequel $R'_1$ et/ou $R'_6$ re-

présente un sel de diazonium ; par des réactions connues de l'homme de l'art, on accède alors aux composés (I) selon l'invention dans lesquels $R_1$ et/ou $R_6$ est un cyano, un halogéno ou un thioalkyle en $C_1$-$C_7$. Enfin par des réactions classiques à partir de composés (I') dans lesquels $R'_1$ et/ou $R'_6$ = $NH_2$, on peut préparer des composés (I) dans lesquels $R_1$ et/ou $R_6$ représente l'un des groupes de formules RCONH-, ROCONH-, RNHCONH- ou $RSO_2NH$-, dans lesquels R représente un ($C_1$-$C_7$)alkyle, un groupe Ar ou un groupe -$CH_2Ar$.

Les composés de formule (I) dans lesquels $R_6$ représente un ($C_1$-$C_7$)alcoxycarbonyle peuvent se préparer directement par le procédé selon l'invention. Par des méthodes connues de l'homme de l'art, ils permettent d'obtenir les composés de formule (I) dans lesquels $R_6$ est un groupe carboxy.

Les composés de formule (I') dans lesquels $R'_6$ est un benzyloxycarbonyle permettent d'obtenir par hydrogénation catalytique les composés (I) dans lesquels $R_6$ est un carboxyle. Par action d'un halogénure de thionyle, on obtient les composés de formule (I') dans lesquels $R'_6$ est un halogénocarbonyle. A partir de tels composés, on prépare des composés de formule (I) dans lesquels $R_6$ est un carbamoyle substitué par $R_{19}$ et $R_{20}$ par action d'un composé $HNR_{19}R_{20}$. On peut également utiliser les composés de formule (I') dans lesquels le substituant $R'_6$ est un phénoxycarbonyle pour obtenir les composés (I) dans lesquels $R_6$ est un phénylcarbamoyle ou un ($C_1$-$C_7$)alkylcarbamoyle par action d'une aniline ou d'une ($C_1$-$C_7$)alkylamine. Une aniline substituée sur le phényle par l'un quelconque des substituants définis pour Ar ou, respectivement, une alkylamine substituée sur l'alkyle par $R_{26}$ permettent d'obtenir des composés de formule (I) dans lesquels $R_6$ est un phénylcarbamoyle substitué sur le phényle ou, respectivement, un alkylcarbamoyle substitué sur l'alkyle par $R_{26}$.

On peut utiliser les composés de formule (I') dans lesquels R'6 est un carboxy pour obtenir les composés de formule (I) dans lesquels $R_6$ est un groupe -$CONR_{19}R_{20}$ par action d'un composé de formule $HNR_{19}R_{20}$, en présence de BOP et d'une amine telle que la diisopropyléthylamine.

De la même façon, les composés de formule (I) dans lesquels $R_6$ est un groupe -$CONHCR_{10}R_{23}COR_{12}$ sont préparés à partir de composés de formule (I') dans lesquels $R'_6$ représente soit un groupe -COCl, soit un groupe phénoxycarbonyle, par action de $H_2NCR_{10}R_{23}COR_{12}$. Ils peuvent également être préparés à partir de composés de formule (I') dans lesquels R'6 est un carboxy par réaction avec un composé $H_2NCR_{10}R_{23}COR_{12}$ en présence de BOP et d'une amine telle que la diisopropylethylamine.

Les composés de formule (I) dans lesquels $R_6$ est un groupe -$COR_{25}$ sont préparés à partir de composés (I') correspondants dans lesquels $R'_6$ est un phénoxycarbonyle, par action de $R_{25}H$.

On peut préparer un composé (I) dans lequel $R_6$ est un thiocarbamoyle par réaction du réactif de Lawesson sur un composé (I) dans lequel $R_6$ est le carbamoyle correspondant.

Un composé (I') dans lequel $R'_6$ est un groupe nitro permet d'obtenir par hydrogénation catalytique, par exemple en présence d'oxyde de platine, de nickel de Raney® ou de palladium sur charbon, ou par réduction chimique, par exemple en présence d'étain ou de fer en milieu acide, un composé (I) dans lequel $R_6$ est un groupe amino ; on peut ensuite préparer d'autres composés dans lesquels le groupe amino est substitué en utilisant des réactions bien connues de l'homme de l'art.

Les composés (I) dans lesquels $R_6$ représente un groupe -$NR_8R_9$, $R_9$ étant un ($C_1$-$C_7$)alkylcarbonyle, un formyle, un ($C_3$-$C_7$)cycloalkylcarbonyle, un benzoyle éventuellement substitué, un pyridylcarbonyle, un méthylpyridylcarbonyle, un thiénylcarbonyle, s'obtiennent par action de l'acide formique dans l'anhydride acétique ou de l'anhydride approprié ou du chlorure d'acide approprié sur un composé (I') dans lequel $R'_6$ est un groupe amino, en présence d'une amine comme la triéthylamine.

De la même façon, on fait agir le chlorure d'acide $R_{11}R_{27}NCR_{10}R_{23}COCl$ sur un composé de formule (I') dans lequel $R'_6$ est un groupe -$NHR_6$ pour préparer un composé de formule (I) dans lequel $R_6$ est un groupe -$NR_6COCR_{10}R_{23}NR_{11}R_{27}$.

Pour préparer un composé de formule (I) dans lequel $R_6$ représente un groupe -$NR_8CO$-($C_2$-$C_7$)alkyl-NAA' dans lequel A et A' sont tels que définis ci-dessus, on fait réagir un halogénure d'halogéno($C_3$-$C_8$)acyle, tel que le chlorure de 3-chloropropionyle ou le chlorure de 4-chlorobutyryle par exemple, sur un composé de formule (I') dans lequel $R'_6$ est un groupe $R_6NH$-, en présence d'une base comme la triéthylamine ; puis par réaction du composé obtenu avec une amine HNAA' on obtient le composé de formule (I) désigné ci-dessus.

De même par action d'un halogénure d'ω-benzyloxy-($C_1$-$C_7$)alkylcarbonyle, sur un composé de formule (I') dans lequel $R'_6$ représente un groupe $R_6NH$-, on prépare un composé de formule (I) dans lequel $R_6$ représente un groupe $NR_6CO$-($C_1$-$C_7$)alkyl-O-$CH_2$-$C_6H_5$. Par hydrogénation du composé précédent, en présence d'un catalyseur tel que le palladium sur charbon à 5 %, on obtient un composé de formule (I) dans lequel $R_6$ est un groupe -$NR_6CO$-($C_1$-$C_7$)alkyl-OH.

Selon un autre exemple de préparation, un composé (I) dans lequel $R_6$ est un groupe ($C_1$-$C_7$)alkylsulfonamido ou respectivement un benzylsulfonamido ou un groupe -$NHSO_2Ar$ s'obtient par action d'un halogènure de ($C_1$-$C_7$)alkylsulfonyle ou respectivement d'un halogénure de benzylsulfonyle ou d'un composé $ArSO_2Cl$ sur un composé (I') dans lequel $R'_6$ est un groupe amino.

Les composés de formule (I') dans lesquels $R'_6$ est un groupe amino sont également utiles pour la préparation de composés dans lesquels ce groupe amino est substitué par un groupe $(CH_2)_t$-$COR_{12}$. Dans ce cas, on fait agir sur (I') en présence de chlorure cuivreux un composé de formule Hal-$(CH_2)_t$-COOAlk dans lequel Hal représente un halogène, par exemple le brome et Alk représente un alkyle en $C_1$-$C_7$ ; le cas échéant, on transforme l'ester ainsi obtenu en acide ou en amide. On peut utiliser l'action d'un anhydride, tel que l'anhydride succinique ou l'anhydride glutarique, sur un composé (I') dans lequel $R'_6$ est un amino pour préparer un composé (I) dans lequel $R_6$ est un groupe -$NHCO(CH_2)_2CO_2H$ ou -$NHCO(CH_2)_3CO_2H$. Le cas échéant on transforme l'acide ainsi obtenu en ester ou en amide.

On peut également utiliser l'action du chlorure d'éthyloxalyle sur un composé (I') dans lequel $R'_6$ est un amino pour préparer un composé (I) dans lequel $R_6$ est un groupe -$NHCOCO_2Et$.

De la même façon, les composés de formule (I) dans lesquels $R_6$ est un groupe amino substitué par un groupe $CR_{10}R_{23}COR_{12}$ sont préparés par action d'un composé de formule Hal-$CR_{10}R_{23}COR_{12}$ sur les composés (I') correspondants dans lesquels le substituant $R'_6$ est un amino.

Par action d'un chloroformiate d'alkyle en $C_1$-$C_7$, de phényle ou de benzyle sur un composé (I') dont le substituant R'6 est un amino, on prépare un composé (I) dans lequel $R_6$ est un groupe amino substitué par un alcoxycarbonyle, un phénoxycarbonyle ou un benzyloxycarbonyle.

De même, par action d'un chlorure de phénoxythiocarbonyle sur un composé de formule (I') dans lequel $R'_6$ est un groupe amino, on obtient un composé de formule (I) dans lequel $R_6$ est un phénoxythiocarbonylamino.

Par action de l'ammoniac sur un composé de formule (I') dans lequel $R'_6$ est un groupe amino substitué par un phénoxycarbonyle ou un phénoxythiocarbonyle, on prépare un composé de formule (I) dans lequel $R_6$ est un uréido ou un thiouréido; par action sur un tel composé de formule (I') d'une aniline correctement substituée ou d'une mono ou dialkylamine en $C_1$-$C_7$ correctement substituées, on prépare un composé de formule (I) dans lequel $R_6$ est un N'-phényluréido correctement substitué, un N'-alkyluréido ou N',N'-dialkyluréido dans lesquels l'alkyle est en $C_1$-$C_7$ correctement substitués.

On peut également préparer d'autres composés (I) dans lequel $R_6$ est un uréido (-$NHCONR_{14}R_{24}$) ou respectivement, un thiouréido (-$NHCSNR_{14}R_{24}$) par action d'un composé $NHR_{14}R_{24}$, sur un composé (I') dans lequel $R'_6$ est un groupe phénoxycarbonylamino ou, respectivement, phénoxythiocarbonylamino.

On peut aussi préparer un composé (I) dans lequel $R_6$ est un uréido (-$NHCONR_{14}R_{24}$) ou respectivement un thiouréido par action d'un chlorure de carbamoyle ($ClCONR_{14}R_{24}$), ou respectivement d'un chlorure de thiocarbamoyle, sur un composé de formule (I') dans lequel $R'_6$ est un groupe amino.

On peut encore préparer un composé (I) dans lequel $R_6$ est un thiouréido par action du réactif de Lawesson sur un composé (I') dans lequel $R'_6$ est l'uréido correspondant.

Les composés (I) dans lesquels $R_6$ est un groupe guanidino non substitué ou substitué une ou deux fois par un alkyle en $C_1$-$C_7$, un phényle ou un benzyle peuvent être préparés à partir des composés (I') dans lesquels $R'_6$ est un groupe phénoxyamido par action du cyanamide ou d'un de ses dérivés correctement substitué sur l'azote.

Les composés (I) dans lesquels $R_6$ est un groupe guanidino substitué en position 2 par un cyano se préparent en deux étapes : on fait réagir le diméthyl N-cyanodithioiminocarbonate sur un composé (I') dans lequel $R'_6$ est un amino, dans un solvant tel que le n-butanol à reflux, pour obtenir un composé (I') dans lequel $R'_6$ est un groupe -$NHC(SCH_3)$=N-CN ; par réaction du composé précédent avec une amine appropriée, on obtient le composé (I) attendu.

On peut également préparer un composé (I) dans lequel $R_6$ est un groupe amino substitué par un ($C_1$-$C_7$)alkylcarbamoyle ou par un phénylcarbamoyle par action d'un isocyanate d'alkyle ou de phényle sur un composé (I') dont le substituant R'6 est un amino.

Par ailleurs, un composé (I) dans lequel $R_6$ est un groupe sulfamoyle substitué par $R_{21}R_{22}$ est préparé par action d'un composé $HNR_{21}R_{22}$ sur un composé (I') dans lequel R'6 est un groupe halogénosulfonyle.

L'affinité des composés selon l'invention pour les récepteurs de la vasopressine a été déterminée in vitro en utilisant la méthode décrite dans C. J. Lynch et al., J. Biol. Chem., 1985, 260 (5), 2844-2851. Cette méthode consiste à étudier le déplacement de la vasopressine tritiée fixée aux sites $V_1$ de membranes de foie de rats. Les concentrations inhibitrices de 50 % ($CI_{50}$) de la fixation de la vasopressine tritiée des composés selon l'invention sont faibles, allant jusqu'à $10^{-7}$M.

L'affinité des composés (I) selon l'invention pour les récepteurs $V_2$ a été mesurée sur une préparation membranaire de rein de boeuf selon une méthode adaptée de P. Crause et al., Molecular and Cellular Endocrinology, 1982, 28, 529-541 et de F.L. Stassen et al., J. Pharmacol. Exp. Ther., 1982, 223, 50-54. Les composés selon l'invention inhibent la fixation de l'arginine-vasopressine tritiée aux récepteurs de la préparation membranaire. Les $CI_{50}$ des composés selon l'invention sont faibles, allant jusqu'à $10^{-9}$M.

L'activité antagoniste des récepteurs $V_2$ des composés selon l'invention a été montrée par le test de dosage de l'activité adénylate cyclase effectué selon une méthode adaptée de M. Laburthe et al., Molecular Pharmacol., 1986, 29, 23-27. On utilise une préparation membranaire de rein de boeuf et chaque produit est incubé 10 minutes à 37°C, seul ou en présence d'AVP (arginine vasopressine) à la concentration de $3.10^{-8}$ M. L'AMP cyclique (adénosine monophosphate cyclique) produite est mesurée par dosage radioimmunologique. On détermine la concentration inhibant de 50 %($CI_{50}$) la stimulation de l'adénylate cyclase induite par $3.10^{-8}$M d'AVP. Les $CI_{50}$ déterminées sont de l'ordre de $10^{-7}$M, allant jusqu'à $10^{-8}$M.

L'activité agoniste ou antagoniste des récepteurs de la vasopressine des composés selon l'invention, administrés par voie orale, est évaluée chez le rat (Souche OFA, Sprague-Dawley) en surcharge hydrique, traité à la vasopressine. L'activité antagoniste des composés, selon l'invention, a été également évaluée chez le rat normalement hydraté (souche OFA, Sprague-Dawley) selon la technique décrite dans Br. J. Pharmacol., 1992, 105, 787-791. L'effet diurétique a été observée pour certains composés à la dose de 10 mg/kg.

De même, l'affinité des composés (I) selon l'invention pour les récepteurs de l'ocytocine a été déterminée in vitro par déplacement d'un analogue radioiodé de l'ocytocine fixé aux récepteurs d'une préparation membranaire de glandes mammaires de rates en gestation, selon une technique proche de celle décrite par J. Eland et al. dans Eur. J. Pharmacol., 1987, 147, 197-207. Les $CI_{50}$ des composés selon l'invention atteignent $10^{-8}$M.

Les composés selon l'invention sont actifs après administration par différentes voies, notamment par voie orale.

Aucun signe de toxicité n'est observé avec ces composés aux doses pharmacologiquement actives.

Ainsi les composés selon l'invention peuvent être utilisés dans le traitement ou la prévention de différentes affections vasopressine-dépendantes ou ocytocine dépendantes, les affections cardiovasculaires, comme l'hypertension, l'hypertension pulmonaire, l'insuffisance cardiaque, l'infarctus du myocarde, ou le vasospasme coronaire, en particulier chez le fumeur, les angines instables et PTCA (percutaneous transluminal coronary angioplasty), l'ischémie cardiaque, les dérèglements de l'hémostase notamment l'hémophilie, le syndrome de Von Willebrand ; les affections du système nerveux central, la migraine, le vasospasme cérébral, l'hémorragie cérébrale, les oedèmes cérébraux, la dépression, l'anxiété, les états psychotiques, les troubles de la mémoire par exemple ; les affections du système rénal comme les oedèmes, le vasospasme rénal, la nécrose du cortex rénal, l'hyponatrémie, l'hypokaliémie, le syndrome de Schwartz Bartter ; les affections du système gastrique comme le vasospasme gastrique, l'hépatocirrhose, les ulcères, la pathologie des vomissements, par exemple la nausée y compris la nausée due à une chimiothérapie, le mal des transports, ou encore le syndrome de la sécrétion inappropriée de l'hormone antidiurétique (SIADH), le diabète insipide et l'énurésie. Les composés selon l'invention peuvent également être utilisés dans le traitement des troubles du comportement sexuel ; chez la femme, les composés selon l'invention peuvent être utilisés pour traiter la dysménorrhée ou le travail prématuré. On peut également utiliser les composés selon l'invention dans le traitement des cancers pulmonaires à petites cellules, des encéphalopathies hyponatrémiques, de la maladie de Raynaud, le syndrome pulmonaire, le glaucome et dans les traitements post-opératoires, notamment après une chirurgie abdominale.

La présente invention a également pour objet des compositions pharmaceutiques contenant une dose efficace d'un composé selon l'invention ou d'un sel pharmaceutiquement acceptable de celui-ci et des excipients convenables.

Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intra-veineuse, topique, intratrachéale, intranasale, transdermique ou rectale, les principes actifs de formule (I) ci-dessus, ou leurs sels éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,01 et 50 mg par kg de poids du corps et par jour.

Chaque dose unitaire peut contenir de 0,5 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule (I) ci-dessus ou d'un de leurs sels pharmaceutiquement acceptables, d'autres principes actifs qui peuvent être utiles dans le traitement des troubles ou maladies indiqués ci-dessus.

Ainsi, la présente invention a également pour objet des compositions pharmaceutiques contenant plusieurs principes actifs en association dont l'un est un composé selon l'invention.

Ainsi, selon la présente invention, on peut préparer des compositions pharmaceutiques contenant un composé selon l'invention associé à un composé agissant sur le système rénine-angiotensine tel qu' un inhibiteur de l'enzyme de conversion, un antagoniste de l'angiotensine II, un inhibiteur de la rénine. On peut également associer un composé selon l'invention, par exemple, avec un vasodilatateur périphérique, un inhibiteur calcique, un béta-bloquant, un alpha-1-bloquant ou un diurétique. De telles compositions seront utiles en particulier dans le traitement de l'hypertension ou de la défaillance cardiaque.

On peut également associer deux composés selon l'invention : un antagoniste spécifique du récepteur $V_1$ à un antagoniste spécifique du récepteur $V_2$ ou bien un antagoniste spécifique du récepteur $V_1$ à un antagonïste spécifique de l'ocytocine.

Ces associations permettront de renforcer les activités thérapeutiques des composés selon l'invention.

L'invention va être décrite maintenant plus en détail par les préparations et exemples illustratifs non limitatifs ci-après.

PREPARATIONS

Préparations des 1,3-dihydro-2*H*-benzimidazol-2-ones.

Préparation 1

5-Chloro-1,3-dihydro-3-phényl-2*H*-benzimidazol-2-one.

On prépare ce composé selon le mode opératoire décrit dans Eur. J. Med. Chem. - Chimica Therapeutica, 1981, 16 (4), 321-326.

Préparation 2

5-chloro-3-cyclohexyl-1,3-dihydro-2*H*-benzimidazol-2-one.

A) 4-chloro-2-cyclohexylamino-1-nitrobenzène

On maintient au reflux pendant 12 heures le mélange constitué de 19,4 g de 2,4-dichloro-1-nitrobenzène, 40 g de cyclohexylamine et 100 ml de 2-éthoxyéthanol.

On évapore sous vide le solvant et reprend le résidu par de l'éther éthylique, et lave avec $H_2O$, séche sur $Na_2SO_4$ et évapore sous vide le solvant. On obtient 11,7 g du produit attendu après cristallisation dans l'éther iso, F = 125°C.

B) 1-Amino-4-chloro-2-cyclohexylaminobenzène

On porte au reflux 12 g du composé obtenu à l'étape A), 8 g de fer en poudre, 15 ml d'eau et 15 ml d'éthanol. On introduit ensuite goutte à goutte en 30 minutes, 30 ml d'acide chlorhydrique concentré dans 20 ml d'eau et 20 ml d'éthanol. On maintient ensuite le milieu réactionnel au reflux pendant 1 heure 30 minutes.

Après refroidissement, on verse le milieu réactionnel sur de la glace et on y ajoute une solution sa-

turée de $NaHCO_3$. On extrait avec AcOEt, lave à l'eau puis avec une solution saturée de $NaHCO_3$, ensuite à l'eau, sèche sur $Na_2SO_4$ et évapore sous vide le solvant. On chromatographie le résidu sur silice en éluant avec de l'éther isopropylique. On obtient 9,4 g du produit attendu, qui est utilisé tel quel à l'étape suivante.

C) 5-chloro-3-cyclohexyl-1,3-dihydro-2*H*-benzimidazol-2-one.

On chauffe à 170-180°C pendant 90 minutes un mélange de 4,5 g du composé obtenu à l'étape B) avec 2,5 g d'urée et 10 ml de 1, 2, 3, 4-tétraméthylbenzène.

Après refroidissement on reprend le milieu réactionnel par de l'acétate d'éthyle. On lave à l'eau, sèche sur sulfate de sodium et évapore sous vide le solvant. Après cristallisation dans de l'heptane et recristallisation dans AcOEt on obtient 3,5 g du produit attendu, F = 213°C.

On peut également préparer ce composé selon Eur. J. Med. Chem. - Chimica Therapeutica, 1981, 16 (4), 321-326, F = 206-208°C.

Préparation 3

3-(1-Benzylpipérid-4-yl)-5-chloro-1,3-dihydro-2*H*-benzimidazol-2-one.

A) 2-[(1-Benzylpipérid-4-yl)amino]-4-chloro-1-nitrobenzène.

On chauffe à 100°C une solution de 38,4 g de 2,4-dichloro-1-nitrobenzène dans 160 ml de 2-éthoxyéthanol. Puis on ajoute lentement une solution de 152,23 g de N-benzyl-4-aminopipéridine dans 40 ml de 2-éthoxyéthanol. On porte au reflux pendant 5 heures. On évapore sous vide le solvant et reprend le résidu par $H_2O$, on extrait par AcOEt, lave à l'eau, sèche sur $Na_2SO_4$ et évapore sous vide le solvant. On chromatographie le résidu sur silice en éluant avec de l'éther isopropylique. On obtient 24,4 g du produit attendu après cristallisation dans l'éther isopropylique, F = 84°C.

B) 1-Amino-2-[(1-benzylpipérid-4-yl)amino]-4-chlorobenzène.

On porte au reflux un mélange de 20,75 g du composé obtenu à l'étape A et de 10 g de fer en poudre dans 19 ml d'eau et 19 ml d'éthanol. On ajoute goutte à goutte à ce mélange une solution de 37,5 ml d'acide chlorhydrique concentré dans 25 ml d'eau et 25 ml d'éthanol et on maintient le reflux pendant 1 heure 30 minutes. Après refroidissement on coule le mélange réactionnel sur de la glace, puis on traite avec une solution saturée de $NaHCO_3$ et extrait avec AcOEt. On lave à l'eau, sèche sur $Na_2SO_4$ et évapore sous vide le solvant. On chromatographie le résidu sur silice en éluant avec un mélange DCM/MeOH (92/8 ; v/v). Après cristallisation dans l'éther isopropylique, on obtient 12,71 g du produit attendu, F = 108°C.

C) 3-(1-Benzylpipérid-4-yl)-5-chloro-1,3-dihydro-2*H*-benzimidazol-2-one.

On maintient au reflux pendant 3 heures un mélange de 12,71 g du composé obtenu à l'étape B, de 8,9 g de 1,1'-carbonyldiimidazole dans 130 ml d'acétonitrite. On évapore le solvant sous vide et reprend le résidu par de l'eau, extrait au DCM, lave par une solution saturée de $NaHCO_3$, lave à l'eau, sèche sur $Na_2SO_4$ et évapore sous vide le solvant. On chromatographie le résidu sur silice en éluant avec un mélange DCM/MeOH (92/8 ; v/v). Après recristallisation dans l'éthanol absolu, on obtient 8,9 g du produit attendu, F = 204-206°C.

Préparation 4

5-Chloro-3-cyclohexylméthyl-1,3-dihydro-2*H*-benzimidazol-2-one.

A) 4-Chloro-1-nitro-2-(cyclohexylméthyl)aminobenzène

A une solution de 8,1 g, de 1,2-dinitro-4-chlorobenzène dans 20 ml d'éthanol 95 on ajoute goutte à goutte une solution de 13,6 g de cyclohexylméthylamine dans 10 ml d'éthanol 95. La température s'élève à 50°C. On maintient le milieu réactionnel sous agitation pendant 2 heures, puis on évapore sous vide le solvant. On reprend le résidu au DCM, lave à l'eau, puis avec de l'acide chlorhydrique 2N, et ensuite à l'eau, sèche sur $Na_2SO_4$ puis évapore sous vide le solvant. On chromatographie le résidu sur silice en éluant avec de l'éther isopropylique. Après recristallisation dans l'heptane on obtient 4,21 g du produit attendu, F = 73°C.

B) 4-Chloro-1-amino-2-(cyclohexylméthyl)aminobenzène.

On porte au reflux une solution de 21,5 g du produit obtenu à l'étape A) et de 13,4 g de fer en poudre dans un mélange de 25 ml d'eau et de 25 ml d'éthanol. Puis on ajoute lentement une solution de 50 ml d'acide chlorhydrique concentré dans un mélange de 34 ml d'eau et 34 ml d'éthanol. On maintient le reflux pendant 2 heures. Puis on coule le milieu réactionnel sur de la glace et on traite avec une solution saturée de $NaHCO_3$, puis on extrait au DCM, lave à l'eau, sèche sur $Na_2SO_4$ et évapore sous vide le solvant. On chromatographie le résidu sur silice en éluant avec DCM. Après cristallisation dans l'heptane on obtient 7,2 g du produit attendu, F = 62°C.

C) 5-Chloro-3-cyclohexylméthyl-1,3-dihydro-2*H*-benzimidazol-2-one.

On chauffe à reflux pendant 5 minutes un mélange de 7,2 g du composé obtenu à l'étape B), 6,7 g de 1,1'-carbonyldiimidazole dans 100 ml d'acétonitrile. On évapore sous vide le solvant, reprend le résidu à l'eau, extrait au DCM, lave par une solution saturée de $NaHCO_3$, sèche sur $Na_2SO_4$ et évapore sous vide. On chromatographie le résidu sur silice en éluant par le mélange DCM/MeOH (95/5 ; v/v). On obtient 5,6 g du produit attendu, F = 173°C.

Préparation 5

5-Chloro-3-cycloheptyl-1,3-dihydro-2*H*-benzimidazol-2-one.

A) 4-Chloro-2-cycloheptylamino-1-nitrobenzène.

On laisse 15 heures sous agitation à TA un mélange de 18,2 g de 4-chloro-1,2-dinitrobenzène, 31 g de cycloheptylamine dans 55 ml d'EtOH 95. On concentre sous vide le mélange réactionnel, extrait le résidu à l'AcOEt, lave par une solution d'HCl 1N, à l'eau, sèche sur $Na_2SO_4$ et évapore sous vide le solvant. On chromatographie le résidu sur silice en éluant à l'éther de pétrole. On obtient 13 g du produit attendu après cristallisation dans l'isopropanol.

B) 1-Amino-4-chloro-2-cycloheptylaminobenzène.

On chauffe à reflux un mélange de 12,9 g du composé obtenu à l'étape précédente, 8 g de fer en poudre dans 15 ml d'eau et 15 ml d'EtOH. Puis on ajoute goutte à goutte une solution de 30 ml d'HCl concentré dans 20 ml d'EtOH et 20 ml d'eau et on maintient à reflux pendant 1 heure et 30 minutes. Après refroidissement, on filtre le mélange réactionnel sur Célite®, lave au MeOH et concentre sous vide le filtrat. On reprend le résidu par de la glace, alcalinise par ajout d'une solution saturée de $NaHCO_3$, extrait au DCM, lave à l'eau, sèche sur $Na_2SO_4$ et évapore sous vide le solvant. On chromatographie le résidu sur silice en éluant à l'éther de pétrole puis à l'éther iso. On obtient 10 g du produit attendu sous forme d'huile que l'on utilise tel quel à l'étape suivante.

C) 5-Chloro-3-cycloheptyl-1,3-dihydro-2*H*-benzimidazol-2-one.

On chauffe à reflux pendant 10 minutes un mélange de 9,9 g du composé obtenu à l'étape précédente, 9,3 g de 1,1'-carbonyldiimidazole dans 150 ml d'acétonitrile. On évapore sous vide le solvant, reprend le résidu par une solution saturée de $NaHCO_3$, extrait au DCM, lave à l'eau, sèche sur $Na_2SO_4$ et évapore sous vide le solvant. On reprend le résidu dans 100 ml d'éther iso et essore le précipité formé. On chromatographie le précipité sur silice en éluant au DCM puis avec le mélange DCM/AcOEt (80/20 ; v/v). On obtient 8,1 g du produit attendu, F = 201°C.

Préparation 6

3-Cyclohexyl-5-éthoxy-1,3-dihydro-2*H*-benzimidazol-2-one.

A) 4-Ethoxy-1-nitro-2-cyclohexylaminobenzène.

On prépare une solution d'éthylate de sodium en ajoutant 0,5 g de sodium dans 60 ml d'éthanol. Puis on ajoute 5,1 g de 4-chloro-2-cyclohexylamino-1-nitrobenzène décrit à la préparation 2 étape A. Ensuite on ajoute 7,5 ml de tris [2-(2-méthoxyéthoxy)éthyl]amine et porte au reflux pendant 3 heures. On évapore sous vide le solvant et reprend le résidu par de l'eau, extrait au DCM, lave à l'eau, sèche sur $Na_2SO_4$ et évapore sous vide le solvant. Après chromatographie sur silice en éluant au DCM on obtient 4,26 g du produit attendu sous la forme d'une huile jaune qui a cristallisé dans l'éther iso, F = 80-82°C.

B) 4-Ethoxy-1-amino-2-cyclohexylaminobenzène.

On porte au reflux un mélange de 4,26 g du composé obtenu à l'étape A), de 2,7 g de fer en poudre dans 5,1 ml d'eau et 5,1 ml d'éthanol. Puis on ajoute goutte à goutte une solution de 10 ml d'HCl concentré dans 7 ml d'eau et 7 ml d'éthanol et on maintient encore le reflux pendant 2 heures. Après refroidissement, on verse le mélange réactionnel sur de la glace et on traite par une solution saturée de $NaHCO_3$, extrait au DCM, filtre sur Célite® un insoluble gris. Après décantation de la phase organique, on lave à l'eau, sèche sur $Na_2SO_4$ et évapore sous vide le solvant. On obtient 3,05g d'une huile noire qui a été utilisée telle que dans l'étape suivante.

C) 3-Cyclohexyl-5-éthoxy-1,3-dihydro-2*H*-benzimidazol-2-one.

On chauffe à 170-180°C pendant 1 heure 30 minutes un mélange de 3,05 g de l'huile obtenue à l'étape B:, de 1,6 g d'urée dans 8 ml de 1, 2, 3, 4-tétraméthylbenzène. Après refroidissement, on reprend dans AcOEt, lave à l'eau, sèche sur sulfate de sodium et évapore sous vide. On reprend le résidu dans de l'hexane et essore le précipité marron formé. On chromatographie le précipité sur silice en éluant avec un mélange DCM/AcOEt (50/50 ; v/v). On obtient 1,33 g du produit attendu qui a été précipité par de l'éther isopropylique. F = 203°C.

Préparation 7

3-Cyclohexyl-1,3-dihydro-5-méthoxy-2*H*-benzimidazol-2-one.

A) 2-Cyclohexylamino-4-méthoxy-1-nitrobenzène.

On prépare une solution de méthylate de sodium en ajoutant 0,5 g de sodium dans 60 ml de MeOH. Puis on ajoute successivement 5,1 g du composé obtenu à la préparation 2 étape A, 7,5 ml de tris [2-(2-méthoxyéthoxy)éthyl]amine et chauffe à reflux pendant 24 heures. On évapore sous vide le solvant, reprend le résidu à l'eau, extrait au DCM, lave à l'eau, sèche sur $Na_2SO_4$ et évapore sous vide. On obtient 3,86 g du produit attendu sous forme d'huile qui cristallise, F = 78-80°C.

B) 1-Amino-2-cyclohexylamino-4-méthoxybenzène.

On chauffe à reflux un mélange de 11,86 g du composé obtenu à l'étape précédente, 7,9 g de fer en poudre, 15 ml d'eau et 15 ml d'EtOH, puis on ajoute, goutte à goutte, une solution de 29,4 ml d'acide chlorhydrique concentré dans 20 ml d'eau et 20 ml d'EtOH. On laisse le mélange réactionnel à reflux pendant 2 heures. Après refroidissement, on verse le mélange réactionnel sur de la glace, ajoute une solution saturée de $NaHCO_3$, extrait au DCM et filtre un insoluble gris sur Célite®. Après décantation du filtrat, on lave la phase organique par une solution saturée de $NaHCO_3$, à l'eau, sèche sur $Na_2SO_4$ et évapore sous vide. On chromatographie le résidu sur silice en éluant par le mélange DCM/MeOH (98/2 ; v/v). On obtient 7,5 g du produit attendu sous forme d'une huile noire que l'on utilise tel quel à l'étape suivante.

C) 3-Cyclohexyl-1,3-dihydro-5-méthoxy-2*H*-benzimidazol-2-one.

On chauffe à 170-180°C pendant 1 heure 30 minutes un mélange de 7,5 g du composé obtenu à l'étape précédente, 4,1 g d'urée dans 20 ml de 1, 2, 3, 4-tetraméthylbenzène. Après refroidissement, on extrait à l'AcOEt, lave à l'eau, sèche sur $Na_2SO_4$ et évapore sous vide. On reprend le résidu à l'hexane et essore le précipité marron formé. On chromatographie le précipité sur silice en éluant par le mélange DCM/AcOEt (50/50 ; v/v). On obtient 2,76 g du produit attendu après cristallisation dans l'AcOEt, F = 163-165°C.

Préparation 8

5-Ethoxy-1,3-dihydro-3-(4(a,e)-méthylcyclohexyl)-2*H*-benzimidazol-2-one.

A) 4-Chloro-2-[(4(a,e)-méthylcyclohexyl)amino]-1-nitrobenzène.

A une solution de 20 g de 4-chloro-1,2-dinitrobenzène dans 80 ml d'EtOH 95 on ajoute goutte à goutte 30 g de 4-méthylcyclohexylamine (mélange d'isomères) et laisse 24 heures sous agitation à TA. On évapore sous vide le mélange réactionnel, extrait le résidu à l'heptane et évapore sous vide le solvant. On obtient 25 g du produit attendu sous forme d'huile rouge et que l'on utilise tel quel à l'étape suivante.

B) 4-Ethoxy-2-[(4(a,e)-méthylcyclohexyl)amino]-1-nitrobenzène.

On prépare ce composé selon le mode opératoire décrit à la préparation 6 étape A à partir de 25 g du composé obtenu à l'étape précédente. On obtient 14 g du produit attendu, F = 85°C.

C) 1-Amino-4-éthoxy-2-[(4(a,e)-méthylcyclohexyl)amino]benzène et 1-amino-4-éthoxy-2-[(4(a)-méthylcyclohexyl)amino]benzène.

On hydrogène à TA, sous une pression de 2 bars, un mélange de 14 g du composé obtenu à l'étape précédente, 0,8 g de palladium sur charbon à 5 % dans 150 ml d'EtOH 95. On filtre le catalyseur sur Célite® et évapore sous vide le filtrat. On reprend le résidu dans l'heptane à chaud et après refroidissement on essore le solide formé. On obtient 6 g du produit attendu sous forme du mélange d'isomères axial-équatorial, F = 92°C. On concentre sous vide les jus d'essorage précédents et on obtient 3 g de l'isomère axial du produit attendu.

D) 4-Ethoxy-1-éthoxycarboxamido-2-[(4(a,e)-méthylcyclohexyl)amino] benzène.

On porte à reflux pendant 1 heure un mélange de 5,8 g du composé obtenu à l'étape précédente, 10 g de chloroformiate d'éthyle dans 100 ml de chloroforme. On évapore sous vide le solvant et chromatographie le résidu sur silice en éluant au DCM. On obtient 5 g du produit attendu qui est utilisé tel quel à l'étape suivante.

E) 5-Ethoxy-1,3-dihydro-3-(4(a,e)-méthylcyclohexyl)-2*H*-benzimidazol-2-one.

A une solution d'éthylate de sodium, préparée en ajoutant 0,75 g de sodium dans 30 ml d'EtOH absolu, on ajoute 5 g du composé obtenu à l'étape précédente et chauffe à reflux pendant 6 heures. On évapore sous vide le mélange réactionnel, extrait le résidu au DCM, lave à l'eau, sèche sur $Na_2SO_4$ et évapore sous vide. On obtient 3 g du produit attendu après cristallisation dans l'éther iso, F = 190°C.

Préparation 9

5-Ethoxy-1,3-dihydro-3-(4(a)-méthylcyclohexyl)-2*H*-benzimidazol-2-one, isomère axial.

A) 4-Ethoxy-1-éthoxycarboxamido-2-[(4(a)-méthylcyclohexyl)amino]benzène.

On prépare ce composé selon le mode opératoire décrit dans la Préparation 8 étape D à partir de 3 g de l'isomère axial du composé obtenu à la Préparation 8 étape C. On chromatographie sur silice en éluant par le mélange DCM/AcOEt (90/10 ; v/v). On obtient 2,8 g du produit attendu après cristallisation dans l'éther iso, F = 183°C.

B) 5-Ethoxy-1,3-dihydro-3-(4(a)-méthylcyclohexyl)-2*H*-benzimidazol-2-one, isomère axial.

On prépare ce composé selon le mode opératoire décrit à la Préparation 8 étape E à partir de 2,8 g du composé obtenu à l'étape précédente. On obtient 1,3 g du produit attendu après cristallisation dans l'éther iso, F = 170°C.

Préparation 10

5-Ethoxy-1,3-dihydro-3-(4(a,e)-méthoxycyclohexyl)-2*H*-benzimidazol-2-one.

A) 4(a,e)-méthoxycyclohexylamine.

On hydrogène pendant 3 heures, à la température de 75-80°C, sous 45 bars de pression, un mélange de 100 g de 4-méthoxyaniline, 48 g de palladium sur charbon à 5% dans 400 ml d'AcOH. On filtre le catalyseur, ajoute 20 ml d'eau au filtrat et évapore sous vide le filtrat. On reprend le résidu avec 100 ml d'eau, refroidit à 0°C, alcalinise par ajout de NaOH concentrée, extrait à l'éther, sèche sur $Na_2SO_4$ et évapore le solvant à pression atmosphérique. On distille l'huile obtenue à pression atmosphérique. On obtient 31 g du produit attendu sous forme d'huile, Eb = 183-188°C.

B) 4-Chloro-2-[(4(a,e)-méthoxycyclohexyl)amino]-1-nitrobenzène.

On laisse 15 heures sous agitation un mélange de 12 g de 4-chloro-1,2-dinitrobenzène, 7 g du composé obtenu à l'étape précédente dans 30 ml d'EtOH. On évapore sous vide le solvant, extrait le résidu à l'éther, lave à l'eau, par une solution 1N de NaOH, par une solution 1N d'HCl, à l'eau, sèche sur $Na_2SO_4$ et évapore sous vide le solvant. On chromatographie le résidu sur silice en éluant au DCM. On obtient 6,6 g du produit attendu, sous forme d'huile, qui est utilisé tel quel à l'étape suivante.

C) 4-Ethoxy-2-[(4(a,e)-méthoxycyclohexyl)amino]-1-nitrobenzène.

On prépare ce composé selon le mode opératoire décrit à la Préparation 6 étape A à partir de 15,4 g du composé obtenu à l'étape précédente. On obtient 12 g du produit attendu après cristallisation dans l'éther iso, F = 93°C.

D) 1-Amino-4-éthoxy-2-[(4(a,e)-méthoxycyclohexyl)amino]benzène.

On hydrogène à TA, à pression atmosphérique, pendant 4 heures, un mélange de 10 g du composé obtenu à l'étape précédente, 3 g de palladium sur charbon à 5 % dans 100 ml d'EtOH. On filtre le catalyseur et évapore sous vide le filtrat. On obtient 8,5 g du produit attendu, sous forme d'huile rouge, qui est utilisé tel quel à l'étape suivante.

E) 4-Ethoxy-1-éthoxycarboxamido-2-[(4(a,e)-méthoxycyclohexyl)amino] benzène.

On refroidit à 10°C une solution de 8,4 g du composé obtenu à l'étape précédente, 13 g de triéthylamine dans 100 ml de DCM et ajoute, goutte à goutte, une solution de 5 ml de chloroformiate d'éthyle dans 15 ml de THF. On laisse 3 heures sous agitation en laissant remonter la température à TA et évapore sous vide les solvants. On extrait le résidu à l'éther iso, lave à l'eau, par une solution à 10 % de $Na_2CO_3$, sèche sur $Na_2SO_4$ et évapore sous vide le solvant. On obtient 12 g du produit attendu qui est utilisé tel quel à l'étape suivante.

F) 5-Ethoxy-1,3-dihydro-3-(4(a,e)-méthoxycyclohexyl)-2*H*-benzimidazol-2-one.

On chauffe à reflux pendant 4 heures un mélange de 12 g du composé obtenu à l'étape précédente, 4,1 g d'éthylate de sodium dans 150 ml de THF. On évapore sous vide le mélange réactionnel, dissout le résidu dans 50 ml d'eau, acidifie à pH = 1 par ajout d'HCl 2N, essore le précipité formé et lave ce dernier à l'eau. On chromatographie le précipité sur silice en éluant au DCM puis par le mélange DCM/AcOEt (70/30 ; v/v). On obtient 7,8 g du produit attendu, F = 201°C.

Préparation 11.

5-Ethoxy-1,3-dihydro-3-[4(a,e)-(2-méthoxyéthoxy)cyclohexyl]-2*H*-benzimidazol-2-one.

A) 4-(2-méthoxyéthoxy)-1-nitrobenzène.

On chauffe à reflux pendant 20 heures un mélange de 40 g de 4-nitrophénol, 41 g de 1-bromo-2-méthoxyéthane, 45 g de $K_2CO_3$, 80 ml de tris [2-(2-méthoxyéthoxy)éthyl]amine dans 80 ml d'acétone. On filtre un insoluble et concentre sous vide le filtrat. On reprend le résidu dans l'eau, essore le précipité formé et lave à l'eau. On dissout le précipité dans l'AcOEt, lave par une solution 1N de NaOH, à l'eau, par une solution 1N d'HCl, à l'eau, sèche sur $Na_2SO_4$ et évapore sous vide le solvant. On obtient 59 g du produit attendu qui est utilisé tel quel à l'étape suivante.

B) 4-(2-méthoxyéthoxy)aniline.

On hydrogène à 40°C, à pression atmosphérique, pendant 5 heures, un mélange de 59 g du composé obtenu à l'étape précédente, 6 g de palladium sur charbon à 5 % dans 400 ml d'EtOH. On filtre le catalyseur et évapore sous vide le filtrat. On obtient 43 g du produit attendu qui est utilisé tel quel à l'étape suivante.

C) 4(a,e)-(2-méthoxyéthoxy)cyclohexylamine.

On prépare ce composé selon le mode opératoire décrit à la Préparation 10 étape A à partir de 43 g du composé obtenu à l'étape précédente. On distille l'huile obtenue sous pression réduite. On obtient 19 g du produit attendu sous forme d'huile, Eb = 123-127°C sous 15 mm de Hg.

D) 4-Chloro-2-[ [4(a,e)-(2-méthoxyéthoxy)cyclohexyl]amino]1-nitrobenzène.

On laisse 15 heures sous agitation à TA un mélange de 19 g du composé obtenu à l'étape précédente, 22,2 g de 4-chloro-1,2-dinitrobenzène, 20 ml de triéthylamine dans 30 ml d'EtOH. On évapore sous vide le mélange réactionnel, reprend le résidu à l'eau, extrait à l'éther, lave par une solution 1N d'HCl, à l'eau, par une solution 1N de NaOH, à l'eau, sèche sur $Na_2SO_4$ et évapore sous vide. On chromatographie le résidu sur silice en éluant à l'éther iso puis au DCM. On obtient 20 g du produit attendu, sous forme d'huile orange, que l'on utilise tel quel à l'étape suivante.

E) 4-Ethoxy-2-[ [4(a,e)-(2-méthoxyéthoxy)cyclohexyl]amino]-1-nitrobenzène.

On prépare une solution d'éthylate de sodium en ajoutant 1,8 g de sodium dans 50 ml d'EtOH, puis on ajoute 19,9 g du composé obtenu à l'étape précédente, 30 ml de tris [2-(2-méthoxyéthoxy)éthyl]amine et 80 ml d'EtOH et chauffe à reflux pendant 5 heures. On évapore sous vide le mélange réactionnel, reprend le résidu par une solution 2N d'HCl, extrait à l'éther, lave à l'eau, sèche sur $Na_2SO_4$ et évapore sous vide. On obtient 17,5 g du produit attendu que l'on utilise tel quel à l'étape suivante.

F) 5-Ethoxy-1,3-dihydro-3-[4(a,e)-(2-méthoxyéthoxy)cyclohexyl]-2*H*-benzimidazol-2-one.

On prépare ce composé selon les modes opératoires décrits à la Préparation 10 étapes D, E puis F à partir de 17,4 g du composé obtenu à l'étape précédente. On obtient 11,5 g du produit attendu, F = 118-120°C.

Préparation 12

5-Ethoxy-1,3-dihydro-3-(2-méthoxy-1,1-diméthyléthyl)-2*H*-benzimidazol-2-one.

A) 4-Chloro-2-[(2-hydroxy-1,1-diméthyléthyl)amino]-1-nitrobenzène.

On chauffe à reflux pendant 36 heures un mélange de 20 g de 4-chloro-1,2-dinitrobenzène, 36 g de 2-amino-2-méthylpropane-1-ol dans 100 ml d'EtOH. On évapore sous vide le mélange réactionnel, extrait le résidu à l'AcOEt, lave par une solution 1N d'HCl, à l'eau, sèche sur $Na_2SO_4$ et évapore sous vide. On chromatographie le résidu sur silice en éluant au DCM. On obtient 16 g du produit attendu que l'on utilise tel quel à l'étape suivante.

B) 4-Chloro-2-[(2-méthoxy-1,1-diméthyléthyl)amino]-1-nitrobenzène.

A une solution de 15 g du composé obtenu à l'étape précédente dans 200 ml de THF, on ajoute, par portions, 1,6 g d'hydrure de sodium et laisse 30 minutes sous agitation à TA. Puis on ajoute 6 ml d'iodure de méthyle et laisse 2 heures sous agitation à TA. On évapore sous vide le solvant, reprend le résidu dans 300 ml d'eau, extrait à l'AcOEt, sèche sur $Na_2SO_4$ et évapore sous vide. On chromatographie le résidu sur silice en éluant au DCM. On obtient 12,5 g du produit attendu que l'on utilise tel quel à l'étape suivante.

C) 4-Ethoxy-2-[(2-méthoxy-1,1-diméthyléthyl)amino]-1-nitrobenzène.

On prépare une solution d'éthylate de sodium en ajoutant 2 g de sodium dans 100 ml d'EtOH, puis ajoute 12,5 g du composé obtenu à l'étape précédente et chauffe à reflux pendant 5 heures. On concentre sous vide le mélange réactionnel, reprend le résidu dans 300 ml d'eau, extrait à l'AcOEt, sèche sur $Na_2SO_4$ et évapore sous vide le solvant. On obtient 12 g du produit attendu que l'on utilise tel quel à l'étape suivante.

D) 1-Amino-4-éthoxy-2-[(2-méthoxy-1,1-diméthyléthyl)amino]benzène.

On hydrogène pendant 24 heures, à 40°C et à pression atmosphérique, un mélange de 12 g du composé obtenu à l'étape précédente, 1,2 g de palladium sur charbon à S % dans 250 ml d'AcOEt. On filtre le catalyseur sur Célite® et évapore sous vide le filtrat. On obtient 12 g du produit attendu que l'on utilise tel quel à l'étape suivante.

E) 4-Ethoxy-1-éthoxycarboxamido-2-[(2-méthoxy-1,1-diméthyléthyl)amino] benzène.

On chauffe 2 heures à reflux un mélange de 12 g du composé obtenu à l'étape précédente, 14 g de chloroformiate d'éthyle dans 200 ml de chloroforme. Après refroidissement on lave par une solution 1N de NaOH, sèche sur $Na_2SO_4$ et évapore sous vide. On chromatographie le résidu sur silice en éluant par le mélange DCM/MeOH (99/1 ; v/v). On obtient 8,4 g du produit attendu, F = 138°C.

F) 5-Ethoxy-1,3-dihydro-3-(2-méthoxy-1,1-diméthyléthyl)-2*H*-benzimidazol-2-one.

On prépare ce composé selon le mode opératoire décrit à la Préparation 8 étape E à partir de 8,4 g du

composé obtenu à l'étape précédente. On obtient 4,9 g du produit attendu après cristallisation dans l'EtOH, F = 149°C.

Préparation 13

5-Ethoxy-1,3-dihydro-3-(1,1,3,3-tétraméthylbutyl)-2*H*-benzimidazol-2-one.

A) 4-Chloro-2-[(1,1,3,3-tetraméthylbutyl)amino]-1-nitrobenzène.

On chauffe à reflux pendant 16 heures un mélange de 20 g de 4-chloro-1,2-dinitrobenzène, 30 g de tert-octylamine dans 300 ml d'EtOH 95. On évapore sous vide le solvant. On obtient 13 g du produit attendu après cristallisation dans le mélange éther iso/heptane (40/60 ; v/v), F = 108°C.

B) 4-Ethoxy-2-[(1,1,3,3-tetraméthylbutyl)amino]-1-nitrobenzène.

On prépare ce composé selon le mode opératoire décrit dans la Préparation 12 étape C à partir de 18 g du composé obtenu à l'étape précédente. On chromatographie sur silice en éluant à l'heptane. On obtient 4 g du produit attendu que l'on utilise tel quel à l'étape suivante.

C) 1-Amino-4-éthoxy-2-[(1,1,3,3-tetraméthylbutyl)amino]benzène.

On hydrogène à TA et à pression atmosphérique un mélange de 4 g du composé obtenu à l'étape précédente, 0,2 g de palladium sur charbon à 5 % dans 150 ml d'AcOEt. On filtre le catalyseur sur Célite® et évapore sous vide le filtrat. On obtient 3,6 g du produit attendu que l'on utilise tel quel à l'étape suivante.

D) 4-Ethoxy-1-éthoxycarboxamido-2-[(1,1,3,3-tétraméthylbutyl)amino] benzène.

On laisse 1 heure sous agitation un mélange de 3,6 g du composé obtenu à l'étape précédente, 2 ml de chloroformiate d'éthyle, 2 ml de triéthylamine dans 100 ml de chloroforme. On lave par une solution 1N de NaOH, à l'eau, une solution 1N d'HCl, à l'eau, sèche sur $Na_2SO_4$ et évapore sous vide le solvant. On obtient 4 g du produit attendu que l'on utilise tel quel à l'étape suivante.

E) 5-Ethoxy-1,3-dihydro-3-(1,1,3,3-tétraméthylbutyl)-2*H*-benzimidazol-2-one.

On prépare une solution d'éthylate de sodium à partir de 0,6 g de sodium et 100 ml d'EtOH, ajoute 4 g du composé obtenu à l'étape précédente et chauffe 3 heures à reflux. On évapore sous vide le solvant, reprend dans 100 ml d'eau, filtre le précipité formé, lave à l'eau puis à l'éther iso. On obtient 2,6 g du produit attendu après séchage, F = 157°C.

Préparation 14

3-(2-Chlorophényl)-5-éthoxy-1,3-dihydro-2*H*-benzimidazol-2-one.

A) 4-Chloro-2-[(2-chlorophényl)amino]-1-nitrobenzène.

On chauffe à reflux pendant 96 heures un mélange de 101 g de 4-chloro-1,2-dinitrobenzène, 191 g de 2-chloroaniline dans 750 ml d'EtOH 95. On évapore sous vide le solvant, extrait le résidu au DCM, lave par une solution 3N d'HCl, à l'eau, sèche sur $Na_2SO_4$ et évapore sous vide le solvant. On chromatographie le résidu sur silice en éluant par le mélange DCM/hexane (50/50 ; v/v). On obtient 7 g du produit attendu après cristallisation dans l'EtOH, F = 97°C.

B) 2-[(2-Chlorophényl)amino]-4-éthoxy-1-nitrobenzène.

On prépare ce composé selon le mode opératoire décrit dans la Préparation 6 étape A à partir de 7 g du composé obtenu à l'étape précédente. On obtient 3,3 g du produit attendu après cristallisation dans l'éther iso.

C) 1-Amino-2-[(2-chlorophényl)amino]-4-éthoxybenzène.

On chauffe à reflux un mélange de 3,3 g du composé obtenu à l'étape précédente, 2 g de fer en poudre, dans 3 ml d'eau et 3 ml d'EtOH, puis on ajoute goutte à goutte une solution de 0,17 ml d'HCl concentré dans 0,7 ml d'eau et 0,7 ml d'EtOH. On laisse deux heures à reflux, puis après refroidissement, on alcalinise par ajout de NaOH concentré, filtre le mélange réactionnel sur Célite®, et lave abondamment à l'AcOEt. Après décantation du filtrat, on sèche la phase organique sur $Na_2SO_4$ et évapore sous vide le solvant. On chromatographie le résidu sur silice en éluant par le mélange DCM/AcOEt (90/10 ; v/v). On obtient 1,75 g du produit attendu que l'on utilise tel quel à l'étape suivante.

D) 2-[(2-Chlorophényl)amino]-4-éthoxy-1-méthoxycarboxamidobenzène.

On chauffe à reflux pendant 3 heures un mélange de 1,75 g du composé obtenu à l'étape précédente, 3 g de chloroformiate de méthyle dans 30 ml de chloroforme. On évapore sous vide le solvant, extrait le résidu au DCM, lave à l'eau, sèche sur $Na_2SO_4$ et évapore sous vide le solvant. On obtient 1,2 g du produit attendu que l'on utilise tel quel à l'étape suivante.

E) 3-(2-Chlorophényl)-5-éthoxy-1,3-dihydro-2*H*-benzimidazol-2-one.

On prépare ce composé selon le mode opératoire décrit à la Préparation 8 étape E à partir de 1,2 g du composé obtenu à l'étape précédente. Après évaporation sous vide du mélange réactionnel, on reprend le résidu à l'AcOEt, lave à l'eau et le produit précipite. On filtre le précipité et on obtient 1 g du produit attendu après séchage, F = 213°C.

Préparation 15

5-Ethoxy-3-(tétrahydropyran-4-yl)-1,3-dihydro-2*H*-benzimidazol-2-one.

A) Tétrahydro-4*H*-pyran-4-one oxime.

A une solution de 35 g de tétrahydro-4*H*-pyran-4-one dans 225 ml de pyridine on ajoute une solution de 29 g de chlorhydrate d'hydroxylamine dans 90 ml d'EtOH et laisse 48 heures sous agitation à TA. On concentre le mélange réactionnel jusqu'à 50 ml, ajoute 500 ml d'eau glacée, extrait 6 fois à l'AcOEt, sèche sur $Na_2SO_4$ et évapore sous vide le solvant. On obtient 26 g du produit attendu, F = 94°C.

B) 4-Aminotétrahydropyrane.

On hydrogène pendant 3 heures à 60°C et sous 20 bars de pression un mélange de 32 g du composé obtenu à l'étape précédente dans 300 ml d'EtOH, en présence de nickel de Raney®. On filtre le catalyseur, évapore sous vide le solvant et distille l'huile obtenue à pression atmosphérique. On obtient 18 g du produit attendu, Eb = 150-175°C.

C) 4-Chloro-1-nitro-2-[(tétrahydropyran-4-yl)amino]benzène.

On chauffe à 60°C pendant 48 heures un mélange de 33 g de 4-chloro-1,2-dinitrobenzène, 18 g du composé obtenu à l'étape précédente, 22 g de triéthylamine dans 250 ml d'EtOH 96. Après refroidissement, on filtre le précipité formé, lave à l'EtOH puis à l'éther iso. On obtient 24,4 g du produit attendu, F = 155°C.

D) 4-Ethoxy-1-nitro-2-[(tétrahydropyran-4-yl)amino]benzène.

On prépare ce composé selon le mode opératoire décrit à la Préparation 12 étape C à partir de 24,4 g du composé obtenu à l'étape précédente. On obtient 21,4 g du produit attendu après cristallisation dans l'éther iso, F = 117°C.

E) 1-Amino-4-éthoxy-2-[(tetrahydropyran-4-yl)amino]benzène.

On hydrogène à 40°C et à la pression atmosphérique un mélange de 21,4 g du composé obtenu à l'étape précédente, 2 g de palladium sur charbon à 5 % dans 500 ml d'AcOEt. On filtre le catalyseur sur Célite® et évapore sous vide le filtrat. On obtient 18 g du produit attendu, F = 101°C.

F) 4-Ethoxy-2-[(tetrahydropyran-4-yl)amino]-1-methoxycarboxamido-benzène.

A une solution de 19 g du composé obtenu à l'étape précédente, 15 ml de triéthylamine dans 500 ml de chloroforme, on ajoute goutte à goutte 30 ml de chloroformiate de méthyle et laisse 3 heures sous agitation à TA. On lave par une solution 1N d'HCl, par une solution 1N de NaOH, sèche sur $Na_2SO_4$ et évapore sous vide le solvant. On obtient 13,8 g du produit attendu après cristallisation dans le mélange éther iso/EtOH(80/20 ; v/v), F = 185°C.

G) 5-Ethoxy-3-(tétrahydropyran-4-yl)-1,3-dihydro-2*H*-benzimidazol-2-one.

On chauffe à reflux pendant 3 heures un mélange de 13,8 g du composé obtenu à l'étape précédente avec une solution d'éthylate de sodium préparée à partir de 3,5 g de sodium dans 300 ml d'EtOH. On évapore sous vide le solvant, reprend le résidu à l'eau, filtre le précipité formé, lave ce dernier à l'AcOEt. On obtient 8,4 g du produit attendu, F = 222°C.

Préparation 16

3-Cyclohexyl-5-cyclopentyloxy-1,3-dihydro-2*H*-benzimidazol-2-one.

A) 2-Cyclohexylamino-4-cyclopentyloxy-1-nitrobenzène.

On chauffe à 50°C une solution de cyclopentylate de sodium, préparée à partir de 0,9 g de sodium dans 150 ml de cyclopentanol, ajoute 10 g du composé obtenu à la Préparation 2 étape A et 12 ml de tris [2-(2-méthoxyéthoxy)èthyl]amine puis chauffe à 100°C pendant 30 heures. On distille sous vide le cyclopentanol, reprend le résidu à l'eau, extrait à l'AcOEt, lave à l'eau, sèche sur $Na_2SO_4$ et évapore sous vide le solvant. On chromatographie le résidu sur silice en éluant à l'heptane. On obtient 4,7 g du produit attendu, F = 98°C.

B) 1-Amino-2-cyclohexylamino-4-cyclopentyloxybenzène.

On hydrogène pendant 3 heures, à TA et sous 2 bars de pression, un mélange de 4,7 g du composé obtenu à l'étape précédente, 0,3 g de palladium sur charbon à 5 % dans 120 ml d'EtOH 95. On filtre le catalyseur sur Célite® et évapore sous vide le filtrat. On obtient 4 g du produit attendu, F = 80°C.

C) 2-Cyclohexylamino-4-cyclopentyloxy-1-éthoxycarboxamidobenzène.

On chauffe à reflux pendant 2 heures un mélange de 4 g du composé obtenu à l'étape précédente, 6 g de chloroformiate d'éthyle dans 50 ml de chloroforme. On lave à l'eau le mélange réactionnel, sèche

sur $Na_2SO_4$ et évapore sous vide le solvant. On chromatographie le résidu sur silice en éluant par le mélange DCM/AcOEt (75/25 ; v/v). On obtient 2,6 g du produit attendu, après cristallisation dans l'éther iso, F = 202°C.

D) 3-Cyclohexyl-5-cyclopentyloxy-1,3-dihydro-2*H*-benzimidazol-2-one.

A une solution d'éthylate de sodium préparée à partir de 0,2 g de sodium dans 50 ml d'EtOH, on ajoute 2,5 g du composé obtenu à l'étape précédente et chauffe à reflux pendant 18 heures. On évapore sous vide le solvant, extrait le résidu à l'AcOEt, lave à l'eau, sèche sur $Na_2SO_4$ et évapore sous vide le solvant. On obtient 1,7 g du produit attendu après cristallisation dans l'éther iso, F = 242°C.

Préparation 17

3-Cyclohexyl-1,3-dihydro-5-(2-méthoxyéthoxy)-2*H*-benzimidazol-2-one.

A) 2-Cyclohexylamino-4-(2-méthoxyéthoxy)-1-nitrobenzène.

A une solution de 2-méthoxyéthylate de sodium préparée à partir de 0,9 g de sodium dans 100 ml de 2-méthoxyéthanol, on ajoute 10 g du composé obtenu à la Préparation 2 étape A et 12 ml de tris [2-(2-méthoxyéthoxy)éthyl]amine puis chauffe à reflux pendant 5 heures. On évapore sous vide le mélange réactionnel, extrait le résidu à l'AcOEt, lave à l'eau, sèche sur $Na_2SO_4$ et évapore sous vide le solvant. On chromatographie le résidu sur silice en éluant par le mélange DCM/heptane (50/50 ; v/v). On obtient 7 g du produit attendu qui est utilisé tel quel à l'étape suivante.

B) 1-Amino-2-cyclohexylamino-4-(2-méthoxyéthoxy)benzène.

On hydrogène à TA et sous 2 bars de pression, un mélange de 7 g du composé obtenu à l'étape précédente, 0,5 g de palladium sur charbon à 5 % dans 200 ml d'EtOH 95. On filtre le catalyseur sur Célite® et évapore sous vide le filtrat. On obtient 5 g du produit attendu qui est utilisé tel quel à l'étape suivante.

C) 2-Cyclohexylamino-1-éthoxycarboxamido-4-(2-méthoxyéthoxy)benzène.

On chauffe à reflux pendant 3 heures un mélange de 5 g du composé obtenu à l'étape précédente, 6 g de chloroformiate d'éthyle dans 50 ml de chloroforme. On évapore sous vide le solvant et chromatographie le résidu sur silice en éluant au DCM. On obtient 6 g du produit attendu, F = 145°C.

D) 3-Cyclohexyl-1,3-dihydro-5-(2-méthoxyéthoxy)-2*H*-benzimidazol-2-one.

A une solution d'éthylate de sodium, préparée à partir de 0,45 g de sodium dans 100 ml d'EtOH, on ajoute 6 g du composé obtenu à l'étape précédente et chauffe à reflux pendant 25 heures. On évapore sous vide le solvant, extrait le résidu à l'AcOEt, lave à l'eau, sèche sur $Na_2SO_4$ et évapore sous vide le solvant. On obtient 2,2 g du produit attendu après cristallisation dans l'éther iso, F = 182°C.

Préparation 18

5-Chloro-3-(3-chlorophényl)-1,3-dihydro-2*H*-benzimidazol-2-one.

A) 4-Chloro-2-[(3-chlorophényl)amino]-1-nitrobenzène.

On chauffe à reflux pendant 72 heures un mélange de 50 g de 2,4-dichloro-1-nitrobenzène, 40 ml de 3-chloroaniline, 43 g d'acétate de sodium anhydre dans 220 ml d'éthylène glycol. Après refroidissement, on filtre le précipité formé et le lave à l'eau. On obtient 39 g du produit attendu après cristallisation dans l'éther iso, F = 112°C.

B) 1-Amino-4-chloro-2-[(3-chlorophényl)amino]benzène.

A un mélange de 38 g du composé obtenu à l'étape précédente, 140 ml d'HCl concentré et 390 ml d'EtOH, on ajoute par portions 64 g d'étain en poudre, en maintenant la température inférieure à 50°C. On laisse 1 heure sous agitation, on filtre le mélange réactionnel sur Célite® et évapore sous vide le filtrat. On extrait le résidu au DCM, lave à l'eau, sèche sur $Na_2SO_4$ et évapore sous vide le solvant. On obtient 38 g du produit attendu, F = 82°C.

C) 4-Chloro-2-[(3-chlorophényl)amino]-1-éthoxycarboxamidobenzène.

A un mélange de 18 g du composé obtenu à l'étape précédente, 10 g de carbonate de potassium dans 160 ml de DMF et 55 ml d'eau, on ajoute lentement 6,8 ml de chloroformiate d'éthyle, en maintenant la température à 20°C. On laisse 1 heure sous agitation, ajoute 300 ml d'eau, extrait au DCM, lave à l'eau, sèche sur $Na_2SO_4$ et évapore sous vide le solvant. On chromatographie le résidu sur silice en éluant par le mélange cyclohexane/DCM (80/20 ; v/v). On obtient 19 g du produit attendu, F = 96°C.

D) 5-Chloro-3-(3-chlorophényl)-1,3-dihydro-2*H*-benzimidazol-2-one.

A une solution d'éthylate de sodium préparée à partir de 2,6 g de sodium dans 100 ml d'EtOH, on ajoute 18 g du composé obtenu à l'étape précédente et chauffe à 60°C pendant 1 heure. On évapore sous vide le solvant, reprend le résidu par 200 ml d'eau, acidifie à pH = 1 par ajout d'HCl concentré, filtre le précipité formé. On obtient 12,6 g du produit attendu, F = 245°C.

Préparation 19

5-Ethoxy-1,3-dihydro-3-[4(a,e)-(tétrahydropyran-2(R,S)-yloxy)cyclohexyl]-2*H*-benzimidazol-2-one.

A) 4-Chloro-2-[(4(a,e)-hydroxycyclohexyl)amino]-1-nitrobenzène.

On laisse 15 heures sous agitation à TA un mélange de 19,8 g de 4-chloro-1,2-dinitrobenzène, 45 g de 4-aminocyclohexanol (mélange d'isomères) dans 75 ml d'EtOH. On évapore sous vide le mélange réactionnel, extrait le résidu à l'éther, lave par une solution 1N d'HCl, à l'eau, sèche sur $Na_2SO_4$ et évapore sous vide le solvant. On chromatographie le résidu sur silice en éluant à l'éther iso. On obtient 14,8 g du produit attendu sous forme d'huile que l'on utilise tel quel à l'étape suivante.

B) 4-Chloro-2-[[4(a,e)-(tétrahydropyran-2(R,S)-yloxy)cyclohexyl]amino]-1-nitrobenzène.

On laisse 20 heures sous agitation à TA un mélange de 15,5 g du composé obtenu à l'étape précédente, 10,5 g de 3,4-dihydro-2*H*-pyrane, 0,1 g d'acide paratoluènesulfonique dans 250 ml d'éther. On évapore sous vide le solvant et obtient 23 g du produit attendu sous forme d'huile que l'on utilise tel quel à l'étape suivante.

C) 4-Ethoxy-2-[[4(a,e)-(tétrahydropyran-2(R,S)-yloxy)cyclohexyl]amino]-1-nitrobenzène.

A une solution d'éthylate de sodium préparée à partir de 1,8 g de sodium dans 30 ml d'EtOH on ajoute 21 g du composé obtenu à l'étape précédente, 10 ml de tris [2-(2-méthoxyéthoxy)éthyl]amine et chauffe à reflux pendant 5 heures. On évapore sous vide le mélange réactionnel, reprend le résidu à l'eau, refroidit à 0°C, acidifie à pH = 1 par ajout d'HCl 1N, extrait rapidement à l'éther, lave à l'eau, sèche sur $Na_2SO_4$ et évapore sous vide le solvant. On obtient 23 g du produit attendu sous forme d'huile que l'on utilise tel quel à l'étape suivante.

D) 1-Amino-4-éthoxy-2-[[4(a,e)-(tétrahydropyran-2(R,S)-yloxy)cyclohexyl] amino]benzène.

On hydrogène à 35-40°C, à pression atmosphérique pendant 6 heures un mélange de 23 g du produit obtenu à l'étape précédente, 3 g de palladium sur charbon à 5 % et 90 ml d'EtOH. On filtre le catalyseur et évapore sous vide le filtrat. On obtient 20 g du produit attendu sous forme d'huile que l'on utilise tel quel à l'étape suivante.

E) 4-Ethoxy-1-éthoxycarboxamido-2-[[4(a,e)-(tétrahydropyran-2(R,S)-yloxy) cyclohexyl]amino]benzène.

On refroidit à 5°C un mélange de 19,9 g du composé obtenu à l'étape précédente, 27 g de triéthylamine dans 150 ml de DCM, ajoute goutte à goutte une solution de 6,5 ml de chloroformiate d'éthyle dans 25 ml de THF et laisse 3 heures sous agitation en laissant remonter la température à TA. On évapore sous vide le mélange réactionnel, extrait le résidu à l'éther, lave à l'eau, sèche sur $Na_2SO_4$ et évapore sous vide le solvant. On chromatographie le résidu sur alumine en éluant à l'éther iso, puis rechromatographie le produit obtenu sur silice en éluant à l'éther iso puis au DCM. On obtient 4 g du produit attendu sous forme d'huile que l'on utilise tel quel à l'étape suivante.

F) 5-Ethoxy-1,3-dihydro-3-[4(a,e)-(tétrahydropyran-2(R,S)-yloxy)cyclohexyl]-2*H*-benzimidazol-2-one.

A une solution d'éthylate de sodium préparée à partir de 0,3 g de sodium dans 50 ml d'EtOH, on ajoute 4 g du composé obtenu à l'étape précédente et chauffe 3 heures à reflux. On évapore sous vide le mélange réactionnel, extrait le résidu à l'AcOEt, lave à l'eau, sèche sur $Na_2SO_4$ et évapore sous vide le solvant. On obtient 1,5 g du produit attendu après cristallisation dans l'éther iso, F = 135-145°C.

Préparation 20

5-Ethoxy-1,3-dihydro-3-[2-(N,N-diisopropylamino)éthyl]-2*H*-benzimidazol-2-one.

A) 4-Chloro-2-[N-[2-(N',N'-diisopropylamino)éthyl]amino]-1-nitrobenzène.

A une solution de 19,5 g de 4-chloro-1,2-dinitrobenzène dans 150 ml d'EtOH on ajoute 39 g de N,N-diisopropyléthylènediamine. La température s'élève à 50°C. On maintient le milieu réactionnel sous agitation pendant 3 heures puis évapore sous vide le solvant. On reprend le résidu dans 200 ml d'isopropanol, refroidit à 0°C, laisse au repos à cette température puis filtre le précipité formé. On obtient 12 g du produit attendu après recristallisation dans l'isopropanol.

B) 4-Ethoxy-2-[N-[2-(N',N'-diisopropylamino)éthyl]amino]-1-nitrobenzène.

On prépare ce composé selon le mode opératoire décrit à la Préparation 6 étape A à partir de 11,3 g du composé obtenu à l'étape précédente. On chromatographie sur silice en éluant par le mélange DCM/MeOH (97/3 ; v/v). On obtient 6,7 g du produit attendu après cristallisation dans l'heptane, F = 89°C.

C) 1-Amino-4-éthoxy-2-[N-[2-(N',N'-diisopropylamino)éthyl]amino]benzène.

On hydrogène pendant 8 heures à TA et à pression atmosphérique un mélange de 6,7 g du composé obtenu à l'étape précédente, 0,55 g de palladium sur charbon à 5 % dans 550 ml d'EtOH. On filtre le catalyseur sur Célite® et évapore sous vide le filtrat. On obtient 5,8 g du produit attendu que l'on utilise tel quel à l'étape suivante.

D) 4-Ethoxy-1-éthoxycarboxamido-2-[N-éthoxycarbonyl-N-[2-(N',N'-diisopropylamino)éthyl]amino]benzène.

A une solution de 5,8 g du composé obtenu à l'étape précédente dans 25 ml de chloroforme, on ajoute goutte à goutte, 5,5 ml de chloroformiate d'éthyle et chauffe à reflux pendant 20 heures. On évapore sous vide le solvant, reprend le résidu par une solution saturée de $NaHCO_3$, extrait au DCM, sèche sur $Na_2SO_4$ et évapore sous vide le solvant. On chromatographie le résidu sur silice en éluant par le mélange DCM/MeOH (95/5 ; v/v). On obtient 5,9 g du produit attendu que l'on utilise tel quel à l'étape suivante.

E) 5-Ethoxy-1,3-dihydro-3-[2-(N,N-diisopropylamino)éthyl]-2*H*-benzimidazol-2-one.

A une solution d'éthylate de sodium préparée à partir de 0,8 g de sodium dans 35 ml d'EtOH, on ajoute 5,9 g du composé obtenu à l'étape précédente et chauffe à reflux pendant 3 heures. On évapore sous vide le solvant, reprend le résidu à l'eau, extrait au DCM, sèche sur $Na_2SO_4$ et évapore sous vide le solvant. On reprend le résidu dans l'éther iso, essore le précipité formé. On obtient 2,4 g du produit attendu, F = 120°C.

Préparation 21

5-Ethoxy-1,3-dihydro-3-[2-(morpholin-4-yl)éthyl]-2*H*-benzimidazol-2-one.

A) 4-Chloro-2-[N-[2-(morpholin-4-yl)éthyl]amino]-1-nitrobenzène.

On laisse 20 heures sous agitation à TA un mélange de 19,5 g de 4-chloro-1,2-dinitrobenzène, 35 g de 4-(2-aminoéthyl)morpholine dans 180 ml d'EtOH. On essore le précipité formé, le lave à l'éther iso. On obtient 17,1 g du produit attendu après deux cristallisations successives dans l'isopropanol.

B) 4-Ethoxy-2-[N-[2-(morpholin-4-yl)éthyl]amino]-1-nitrobenzène.

On prépare ce composé selon le mode opératoire décrit à la Préparation 6 étape A à partir de 8,6 g du composé obtenu à l'étape précédente. On chromatographie sur silice en éluant par le mélange DCM/MeOH (99/1; v/v). On obtient 4,3 g du produit attendu, F = 107°C.

C) 1-Amino-4-éthoxy-2-[N-[2-(morpholin-4-yl)éthyl]amino]-1-nitrobenzène.

On hydrogène pendant 1 heure à TA et à pression atmosphérique un mélange de 4,3 g du composé obtenu à l'étape précédente, 0,4 g de palladium sur charbon à 5 % dans 400 ml d'EtOH. On filtre le catalyseur sur Célite® et évapore sous vide le filtrat. On chromatographie le résidu sur silice en éluant par le mélange DCM/MeOH (95/5 ; v/v). On obtient 3,7 g du produit attendu sous forme d'huile que l'on utilise tel quel à l'étape suivante.

D) 4-Ethoxy-1-éthoxycarboxamido-2-[N-[2-(morpholin-4-yl)éthyl]amino] benzène.

A une solution de 3,7 g du composé obtenu à l'étape précédente dans 20 ml de chloroforme, on ajoute à TA, goutte à goutte, 3 ml de chloroformiate d'éthyle et chauffe à reflux pendant 4 heures. On évapore sous vide le solvant, reprend le résidu par une solution saturée de $NaHCO_3$, extrait au DCM, sèche sur $Na_2SO_4$ et évapore sous vide le solvant. On obtient 4,5 g du produit attendu que l'on utilise tel quel à l'étape suivante.

E) 5-Ethoxy-1,3-dihydro-3-[2-(morpholin-4-yl)éthyl]-2*H*-benzimidazol-2-one.

A une solution d'éthylate de sodium préparée à partir de 0,6 g de sodium dans 25 ml d'EtOH, on ajoute 4,5 g du composé obtenu à l'étape précédente et chauffe à reflux pendant 3 heures. On évapore sous vide le solvant, reprend le résidu à l'eau, extrait au DCM, sèche sur $Na_2SO_4$ et évapore sous vide le solvant. On chromatographie le résidu sur silice en éluant par le mélange DCM/MeOH (95/5 ; v/v). On reprend le produit obtenu dans l'éther iso, essore le précipité formé. On obtient 0,85 g du produit attendu, F = 160°C.

Préparation 22

5-Ethoxy-1,3-dihydro-3-(4(a,e)-diméthylaminocyclohexyl)-2*H*-benzimidazol-2-one.

A) 4-Diméthylaminocyclohexylamine.

On hydrogène à la température de 75-90°C, sous 50 bars de pression, un mélange de 68g de 4-diméthylaminoaniline, 34 g de palladium sur charbon à 5 % dans 250 ml d'AcOH. On filtre le catalyseur, lave à l'eau et évapore sous vide le filtrat. On reprend la résidu à l'eau, alcalinise par ajout de NaOH concentrée, extrait à l'AcOEt, sèche sur $Na_2SO_4$ et évapore sous vide le solvant. On distille l'huile obtenue sous pression réduite. On obtient 16,2 g du produit attendu sous forme d'huile, Eb = 102-110°C sous 20 mm de Hg.

B) 4-Chloro-2-[(4(a,e)-diméthylaminocyclohexyl)amino]-1-nitrobenzène.

On laisse 20 heures sous agitation à TA un mélange de 22 g de 4-chloro-1,2-dinitrobenzène, 16 g du composé obtenu à l'étape précédente, 20 ml de triéthylamine dans 30 ml d'EtOH. On évapore sous vide le mélange réactionnel, reprend le résidu à l'eau, extrait à l'AcOEt, lave à l'eau, sèche sur $Na_2SO_4$ et évapore sous vide le solvant. On chromatographie le résidu sur alumine en éluant au DCM. On obtient 14,9 g du produit attendu sous forme d'huile qui cristallise, F = 85°C.

C) 4-Ethoxy-2-[(4(a,e)-diméthylaminocyclohexyl)amino]-1-nitrobenzène.

A une solution d'éthylate de sodium préparée à partir de 1,5 g de sodium dans 80 ml d'EtOH, on ajoute 14,9 g du composé obtenu à l'étape précédente, 15 ml de tris[2-(2-méthoxyéthoxy)éthyl]amine et chauffe à reflux pendant 5 heures. On évapore sous vide le mélange réactionnel, reprend le résidu à l'eau, extrait à l'éther iso, lave à l'eau, sèche sur $Na_2SO_4$ et évapore sous vide le solvant. On obtient 9 g du produit attendu après cristallisation dans l'éther iso, F = 75°C.

D) 1-Amino-4-éthoxy-2-[(4(a,e)-diméthylaminocyclohexyl)amino]benzène.

On hydrogène pendant 3 heures à TA et à pression atmosphérique un mélange de 9 g du composé obtenu à l'étape précédente, 2 g de palladium sur charbon à 5 % et 40 ml d'EtOH. On filtre le catalyseur, lave au MeOH et évapore sous vide le filtrat. On obtient 7,3 g du produit attendu sous forme d'huile que l'on utilise tel quel à l'étape suivante.

E) 4-Ethoxy-1-éthoxycarboxamido-2-[(4(a,e)-diméthylaminocyclohexyl) amino] benzène.

On refroidit à 10°C un mélange de 7,2 g du composé obtenu à l'étape précédente, 12 g de triéthylamine dans 70 ml de DCM et ajoute goutte à goutte une solution de 2,9 ml de chloroformiate d'éthyle dans 10 ml de DCM. On laisse 3 heures sous agitation en laissant remonter la température à TA et évapore sous vide. On reprend le résidu à l'eau, extrait au DCM, sèche sur $Na_2SO_4$ et évapore sous vide le solvant. On obtient 3,7 g du produit attendu après cristallisation dans l'éther iso, F = 193-195°C.

F) 5-Ethoxy-1,3-dihydro-3-(4(a,e)-diméthylaminocyclohexyl)-2*H*-benzimidazol-2-one.

A une solution d'éthylate de sodium préparée à partir de 0,3 g de sodium dans 30 ml d'EtOH, on ajoute 3,6 g du composé obtenu à l'étape précédente et chauffe à reflux pendant 5 heures. On reprend le résidu à l'eau, essore le précipité formé et lave ce dernier à l'eau, à l'isopropanol et au pentane. On obtient 2,15 g du produit attendu, F = 215-217°C.

Préparation 23

5-Ethoxy-1,3-dihydro-3-(4-méthylpipérazin-1-yl)-2*H*-benzimidazol-2-one.

A) 4-Chloro-2-[(4-méthylpipérazin-1-yl)amino]-1-nitrobenzène.

On laisse 24 heures sous agitation à TA un mélange de 35 g de 4-chloro-1,2-dinitrobenzène, 20 g de 1-amino-4-méthylpipérazine dans 200 ml d'EtOH 96. On évapore sous vide le mélange réactionnel et chromatographie le résidu sur silice en éluant par le mélange DCM/AcOEt (80/20 ; v/v). On obtient 17,5 g du produit attendu après cristallisation dans le mélange éther iso/heptane (50/50 ; v/v), F = 108°C.

B) 4-Ethoxy-2-[(4-méthylpipérazin-1-yl)amino]-1-nitrobenzène.

A une solution d'éthylate de sodium préparée à partir de 1,5 g de sodium dans 85 ml d'EtOH, on ajoute 17,5 g du composé obtenu à l'étape précédente, 13 ml de tris[2-(2-méthoxyéthoxy)éthyl]amine et chauffe à reflux pendant 4 heures. On évapore sous vide le mélange réactionnel, reprend le résidu à l'eau, extrait au DCM, sèche sur $Na_2SO_4$ et évapore sous vide le solvant. On obtient 13,5 g du produit attendu après cristallisation dans l'éther iso, F = 145°C.

C) 1-Amino-4-éthoxy-2-[(4-méthylpipérazin-1-yl)amino]benzène.

On hydrogène pendant 3 heures à TA et à pression atmosphérique un mélange de 13,5 g du composé obtenu à l'étape précédente, 0,75 g de palladium sur charbon à 5 % et 350 ml d'EtOH 96. On filtre le catalyseur et évapore sous vide le filtrat. On obtient 10,5 g du produit attendu que l'on utilise tel quel à l'étape suivante.

D) 4-Ethoxy-1-éthoxycarboxamido-2-[(4-méthylpipérazin-1-yl)amino] benzène.

On refroidit au bain de glace un mélange de 5 g du composé obtenu à l'étape précédente, 2,2 ml de triéthylamine dans 60 ml de DCM et ajoute goutte à goutte une solution de 2,2 ml de chloroformiate d'éthyle dans 20 ml de DCM. On laisse 16 heures sous agitation en laissant remonter la température à TA et évapore sous vide le solvant. On chromatographie le résidu sur silice en éluant par le mélange DCM/MeOH (90/10; v/v). On obtient 2,5 g du produit attendu que l'on utilise tel quel à l'étape suivante.

E) 5-Ethoxy-1,3-dihydro-3-(4-méthylpipérazin-1-yl)-2*H*-benzimidazol-2-one.

A une solution d'éthylate de sodium préparée à partir de 0,36 g de sodium dans 15 ml d'EtOH on ajoute une solution de 2,5 g du composé obtenu précédemment dans 20 ml d'EtOH et chauffe à reflux pendant 8 heures. On évapore sous vide, reprend le résidu à l'eau, extrait à l'AcOEt, lave à l'eau, sèche sur $Na_2SO_4$ et évapore sous vide le solvant. On chromatographie le résidu sur silice en éluant par le mélange DCM/AcOEt (90/10 ; v/v). On obtient 0,75 g du produit attendu après cristallisation dans l'éther iso, F = 203°C.

Préparation 24

5-Ethoxy-1,3-dihydro-3-(morpholin-4-yl)-2*H*-benzimidazol-2-one.

A) 4-Chloro-2-[(morpholin-4-yl)amino]-1-nitrobenzène.

On laisse 48 heures sous agitation à TA un mélange de 45 g de 4-chloro-1,2-dinitrobenzène, 25 g de 4-aminomorpholine, 30 g de triéthylamine dans 250 ml d'EtOH 96. On filtre le précipité formé et lave ce dernier à l'éther iso. On obtient 30,2 g du produit attendu, F = 155°C.

B) 4-Ethoxy-2-[(morpholin-4-yl)amino]-1-nitrobenzène.

A une solution d'éthylate de sodium préparée à partir de 3,5 g de sodium dans 250 ml d'EtOH, on ajoute 30,2 g du composé obtenu à l'étape précédente et chauffe à reflux pendant 5 heures. On évapore sous vide le solvant, extrait le résidu à l'AcOEt, lave à l'eau, sèche sur $Na_2SO_4$ et évapore sous vide le solvant. On obtient 19 g du produit attendu après cristallisation dans l'EtOH, F = 152°C.

C) 1-Amino-4-éthoxy-2-[(morpholin-4-yl)amino]benzène.

On hydrogène à TA et pression atmosphérique un mélange de 19 g du composé obtenu à l'étape précédente, 2 g du palladium sur charbon à 5 % dans 1000 ml d'AcOEt. On filtre le catalyseur et évapore sous vide le filtrat. On obtient 14 g du produit attendu, sous forme d'huile, utilisé tel quel à l'étape suivante.

D) 4-Ethoxy-1-éthoxycarboxamido-2-[(morpholin-4-yl)amino]benzène.

On refroidit au bain de glace un mélange de 14 g du composé obtenu à l'étape précédente, 5 ml de triéthylamine dans 300 ml de chloroforme et ajoute 10 ml de chloroformiate d'éthyle. On laisse 30 minutes sous agitation à TA, lave le mélange réactionnel par une solution de NaOH 1N, sèche la phase organique sur $Na_2SO_4$ et évapore sous vide le solvant. On reprend le résidu dans l'éther iso, filtre un insoluble et chromatographie le filtrat sur silice en éluant par le mélange DCM/MeOH (99/1 ; v/v). On obtient 5 g du produit attendu que l'on utilise tel quel à l'étape suivante.

E) 5-Ethoxy-1,3-dihydro-3-(morpholin-4-yl)-2*H*-benzimidazol-2-one.

A une solution d'éthylate de sodium préparée à partir de 1 g de sodium dans 50 ml d'EtOH, on ajoute 5 g du composé obtenu à l'étape précédente et chauffe à reflux pendant 5 heures. On évapore sous vide, extrait le résidu à l'AcOEt, lave à l'eau, sèche sur $Na_2SO_4$ et évapore sous vide le solvant. On obtient 1,87 g du produit attendu après cristallisation dans le DCM et recristallisation dans l'EtOH, F = 228°C.

Préparation 25

5-Ethoxy-1,3-dihydro-3-(4-méthoxyphényl)-2*H*-benzimidazol-2-one.

A) 4-Chloro-2-[(4-méthoxyphényl)amino]-1-nitrobenzène.

On chauffe 15 heures à reflux un mélange de 10 g de 4-chloro-1,2-dinitrobenzène, 6,5 g de 4-méthoxyaniline et 16 g de 1,2,3,4-tétraméthylbenzène. Après refroidissement, on ajoute de l'eau, extrait à l'AcOEt, lave par une solution d'HCl 1N, par une solution de NaOH 1N, à l'eau, sèche sur $Na_2SO_4$ et évapore sous vide le solvant. On reprend le résidu à l'éther iso, sépare un insoluble gommeux et chromatographie le filtrat sur alumine en éluant à l'éther iso. On obtient 4,6 g du produit attendu après cristallisation dans l'isopropanol, F = 98°C.

B) 4-Ethoxy-2-[(4-méthoxyphényl)amino]-1-nitrobenzène.

A une solution d'éthylate de sodium préparée à partir de 0,45 g de sodium dans 20 ml d'EtOH, on ajoute 4,5 g du composé obtenu à l'étape précédente, 5 ml de tris[2-(2-méthoxyéthoxy)éthyl]amine et chauffe à reflux pendant 3 heures. On évapore sous vide, reprend le résidu à l'eau, extrait à l'éther, lave par une solution d'HCl 1N, à l'eau, sèche sur $Na_2SO_4$ et évapore sous vide. On obtient 2,2 g du produit attendu après cristallisation dans l'éther iso, F = 109°C.

C) 1-Amino-4-éthoxy-2-[(4-méthoxyphényl)amino]benzène.

On hydrogène pendant 8 heures à TA et pression atmosphérique un mélange de 2,2 g du composé obtenu à l'étape précédente, 0,5 g de palladium sur charbon à 5 % et 20 ml d'EtOH. On filtre le catalyseur, lave à l'EtOH et évapore sous vide le filtrat. On obtient 1,9 g du produit attendu que l'on utilise tel quel à l'étape suivante.

D) 4-Ethoxy-1-éthoxycarboxamido-2-[(4-méthoxyphényl)amino]benzène.

On refroidit à 5 °C un mélange de 1,9 g du composé obtenu à l'étape précédente, 3 g de triéthylamine dans 20 ml de DCM et ajoute 1,1 g de chloroformiate d'éthyle. On laisse 3 heures sous agitation en laissant remonter la température à TA et évapore sous vide. On reprend le résidu à l'eau, sèche sur $Na_2SO_4$ et évapore sous vide le solvant. On obtient 2,4 g du produit attendu que l'on utilise tel quel à l'étape suivante.

E) 5-Ethoxy-1,3-dihydro-3-(4-méthoxyphényl)-2*H*-benzimidazol-2-one.

A une solution d'éthylate de sodium préparée à partir de 0,17 g de sodium dans 10 ml d'EtOH on ajoute une solution de 2,4 g du composé obtenu à l'étape précédente dans 15 ml d'EtOH et chauffe à reflux pendant 4 heures. On évapore sous vide, reprend le résidu par une solution d'HCl 1N, essore le précipité formé, le lave à l'eau puis au DCM. On obtient 1,7 g du produit attendu, F = 204°C.

Préparation 26

5-Ethoxy-1,3-dihydro-3-(4-isopropylphényl)-2*H*-benzimidazol-2-one.

A) 4-Chloro-2-[(4-isopropylphényl)amino]-1-nitrobenzène.

On chauffe à reflux pendant 15 heures un mélange de 15 g de 4-chloro-1,2-dinitrobenzène, 10 g de 4-isopropylaniline et 25 ml de Décaline®. On concentre le mélange réactionnel sous 0,01 mm de Hg, reprend le résidu à l'eau, extrait à l'éther, lave par une solution d'HCl 1N, à l'eau, sèche sur $Na_2SO_4$ et évapore sous vide le solvant. On chromatographie le résidu sur silice en éluant à l'éther iso. On obtient 13 g du produit attendu que l'on utilise tel quel à l'étape suivante.

B) 4-Ethoxy-2-[(4-isopropylphényl)amino]-1-nitrobenzène.

On prépare ce composé selon le mode opératoire décrit à la Préparation 25 étape B à partir de 13 g du composé obtenu à l'étape précédente. On chromatographie sur silice en éluant au pentane. On obtient 4,4 g du produit attendu, F = 100,5°C.

C) 1-Amino-4-éthoxy-2-[(4-isopropylphényl)amino]benzène.

On prépare ce composé selon le mode opératoire décrit à la Préparation 25 étape C à partir de 4,3 g du composé obtenu à l'étape précédente. On obtient 4 g du produit attendu que l'on utilise tel quel à l'étape suivante.

D) 4-Ethoxy-1-éthoxycarboxamido-2-[(isopropylphényl)amino]benzène.

On prépare ce composé selon le mode opératoire décrit à la Préparation 25 étape D à partir de 3,9 g du composé obtenu à l'étape précédente. On chromatographie sur silice en éluant à l'éther iso. On obtient 5 g du produit attendu que l'on utilise tel quel à l'étape suivante.

E) 5-Ethoxy-1,3-dihydro-3-(4-isopropylphényl)-2*H*-benzimidazol-2-one.

On prépare ce composé selon le mode opératoire décrit à la Préparation 25 étape E à partir de 4,9 g du composé obtenu à l'étape précédente. On obtient 2,8 g du produit attendu après cristallisation dans l'EtOH, F = 202°C.

Préparation 27

5-Ethoxy-1,3-dihydro-3-(indan-2-yl)-2*H*-benzimidazol-2-one.

A) 4-Chloro-2-(indan-2-yl)amino-1-nitrobenzène.

A une solution d'éthylate de sodium préparée à partir de 0,61 g de sodium dans 200 ml d'EtOH, on ajoute 26 g de chlorhydrate de 2-aminoindane puis 20 g de 4-chloro-1,2-dinitrobenzène et laisse 48 heures sous agitation à TA. On évapore sous vide le solvant, extrait le résidu à l'AcOEt, lave à l'eau, sèche sur $Na_2SO_4$ et évapore sous vide le solvant. On obtient 10,3 g du produit attendu après cristallisation dans l'EtOH, F = 108°C.

B) 4-Ethoxy-2-(indan-2-yl)amino-1-nitrobenzène.

A une solution d'éthylate de sodium préparée à partir de 2 g de sodium dans 100 ml d'EtOH, on ajoute 10,3 g du composé obtenu à l'étape précédente et chauffe à reflux pendant 4 heures. On évapore sous vide le solvant, extrait à l'AcOEt, lave à l'eau, sèche sur $Na_2SO_4$ et évapore sous vide le solvant. On chromatographie le résidu sur silice en éluant au DCM. On obtient 8,5 g du produit attendu, F = 151°C.

C) 1-Amino-4-éthoxy-2-(indan-2-yl)aminobenzène.

On hydrogène à TA et à pression atmosphérique un mélange de 8,5 g du composé obtenu à l'étape précédente, 1 g de palladium sur charbon à 5 % dans 500 ml d'AcOEt. On filtre le catalyseur et évapore sous vide le filtrat. On obtient 7,2 g du produit attendu que l'on utilise tel quel à l'étape suivante.

D) 4-Ethoxy-1-éthoxycarboxamido-2-(indan-2-yl)aminobenzène.

On chauffe à reflux pendant 2 heures un mélange de 7,2 g du composé obtenu à l'étape précédente, 8,4 g de chloroformiate d'éthyle dans 100 ml de chloroforme. On extrait au chloroforme, lave par une solution de NaOH 1N, sèche sur $Na_2SO_4$ et évapore sous vide le solvant. On chromatographie le résidu sur silice en éluant au DCM. On obtient 3,5 g du produit attendu, F = 122°C.

E) 5-Ethoxy-1,3-dihydro-3-(indan-2-yl)-2*H*-benzimidazol-2-one.

On prépare une solution d'éthylate de sodium à partir de 0,7 g de sodium dans 50 ml d'EtOH, ajoute 3,5 g du composé obtenu à l'étape précédente et chauffe 3 heures à reflux. On évapore sous vide le solvant, reprend le résidu à l'eau, extrait au DCM, sèche sur $Na_2SO_4$ et évapore sous vide le solvant. On obtient 2,6 g du produit attendu après cristallisation dans l'EtOH, F = 225°C.

Préparation 28

3-(Adamant-1-yl)-5-éthoxy-1,3-dihydro-2*H*-benzimidazol-2-one.

A) 2-(Adamant-1-yl)amino-4-chloro-1-nitrobenzène.

A une solution de 20 g de 4-chloro-1,2-dinitrobenzène dans 80 ml d'EtOH 96 on ajoute 15,5 ml de triéthylamine puis une suspension de 15 g de 1-aminoadamantane dans 50 ml d'EtOH 96. On chauffe à reflux pendant 7 heures et après refroidissement on essore le précipité formé. On obtient 7 g du produit attendu, F = 146°C.

B) 2-(Adamant-1-yl)amino-4-éthoxy-1-nitrobenzène.

A une solution d'éthylate de sodium préparée à partir de 0,6 g de sodium dans 70 ml d'EtOH, on ajoute 7 g du composé obtenu à l'étape précédente, 8 ml de tris[2-(2-méthoxyéthoxy)éthyl]amine et chauffe à reflux pendant 3 heures. On évapore sous vide le solvant, reprend le résidu à l'eau, extrait au DCM, sèche sur $Na_2SO_4$ et évapore sous vide le solvant. On obtient 6 g du produit attendu, F = 147°C.

C) 2-(Adamant-1-yl)amino-1-amino-4-éthoxybenzène.

On hydrogène à TA et sous 2 bars de pression un mélange de 6 g du composé obtenu à l'étape précédente, 0,85 g de palladium sur charbon à 5 % et 70 ml d'EtOH 96. On filtre le catalyseur, le lave à l'AcOEt et évapore sous vide le filtrat. On obtient 4,7 g du produit attendu que l'on utilise tel quel à l'étape suivante.

D) 2-(Adamant-1-yl)amino-4-éthoxy-1-éthoxycarboxamidobenzène.

On chauffe à reflux pendant 1 heure 30 minutes un mélange de 4,7 g du composé obtenu à l'étape précédente, 6 ml de chloroformiate d'éthyle dans 70 ml de chloroforme. On évapore sous vide le solvant, reprend le résidu dans le mélange éther iso/AcOEt (50/50 ; v/v) et essore le précipité formé. On chromatographie le précipité sur silice en éluant au DCM puis par le mélange DCM/AcOEt (80/20 ; v/v). On obtient 5 g du produit attendu que l'on utilise tel quel à l'étape suivante.

E) 3-(Adamant-1-yl)-5-éthoxy-1,3-dihydro-2*H*-benzimidazol-2-one.

On prépare ce composé selon le mode opératoire décrit à la Préparation 27 étape E à partir de 5 g du composé obtenu précédemment. Après évaporation sous vide du solvant, on reprend le résidu dans l'AcOEt et essore le précipité formé. On obtient 2,5 g du produit attendu, F = 264°C.

Préparation 29

3-Cycloheptyl-5-éthoxy-1,3-dihydro-2*H*-benzimidazol-2-one.

A) 2-Cycloheptylamino-4-éthoxy-1-nitrobenzène.

A une solution d'éthylate de sodium préparée à partir de 2,2 g de sodium dans 250 ml d'EtOH, on ajoute 25 g du composé obtenu à la Préparation 5 étape A, 30 ml de tris[2-(2-méthoxyéthoxy)éthyl]amine et chauffe à reflux pendant 24 heures. On concentre sous vide et chromatographie le résidu sur silice en éluant par le mélange DCM/heptane (50/50 ; v/v). On obtient 14 g du produit attendu, F = 83°C.

B) 1-Amino-2-cycloheptylamino-4-éthoxybenzène.

On hydrogène pendant 24 heures à TA et sous 2 bars de pression, un mélange de 13 g du composé obtenu à l'étape précédente, 0,3 g de palladium sur charbon à 5 % et 250 ml d'EtOH 95. On filtre le catalyseur et évapore sous vide le filtrat. On chromatographie le résidu sur silice en éluant par le mélange DCM/heptane (50/50 ; v/v) puis au DCM et enfin par le mélange DCM/AcOEt (90/10 ; v/v). On obtient 6 g du produit attendu que l'on utilise tel quel à l'étape suivante.

C) 4-Ethoxy-1-éthoxycarboxamido-2-cycloheptylaminobenzène.

On chauffe à reflux pendant 5 heures un mélange de 6 g du composé obtenu à l'étape précédente, 7,5 g de chloroformiate d'éthyle dans 50 ml de chloroforme, On concentre sous vide et chromatographie le résidu sur silice en éluant au DCM. On obtient 4,7 g du produit attendu après cristallisation dans l'éther iso, F = 172°C.

D) 3-Cycloheptyl-5-éthoxy-1,3-dihydro-2*H*-benzimidazol-2-one.

A une solution d'éthylate de sodium préparée à partir de 0,35 g de sodium dans 100 ml d'EtOH on ajoute 4,5 g du composé obtenu à l'étape précédente et chauffe à reflux pendant 24 heures. On concentre sous vide, extrait le résidu à l'AcOEt, lave à l'eau, sèche sur $Na_2SO_4$ et évapore sous vide le solvant. On obtient 2,9 g du produit attendu après cristallisation dans l'éther iso, F = 204°C.

Préparation 30

5-Chloro-3-(2-chlorophényl)-1,3-dihydro-2*H*-benzimidazol-2-one.

A) 1-Amino-4-chloro-2-[(2-chlorophényl)amino]benzène.

On hydrogène pendant 4 heures à TA et sous 2 bars de pression un mélange de 3 g du composé obtenu à la Préparation 14 étape A, 0,5 g de nickel de Raney® et 100 ml d'EtOH 95. On filtre le catalyseur et évapore sous vide le filtrat. On obtient 2 g du produit attendu que l'on utilise tel quel à l'étape suivante.

B) 5-Chloro-3-(2-chlorophényl)-1,3-dihydro-2*H*-benzimidazol-2-one.

On chauffe à reflux pendant 30 minutes un mélange de 2 g du composé obtenu à l'étape précédente, 2 g de 1,1'-carbonyldiimidazole dans 50 ml d'acétonitrile. On concentre sous vide le mélange réactionnel, extrait le résidu à l'AcOEt, lave par une solution d'HCl 1N, à l'eau, par une solution saturée de $NaHCO_3$, à l'eau, sèche sur $Na_2SO_4$ et évapore sous vide le solvant. On obtient 1 g du produit attendu après cristallisation dans l'éther iso, F = 239°C.

Préparation 31

5-Ethoxy-1,3-dihydro-3-(pyrid-2-yl)-2*H*-benzimidazol-2-one.

A) 4-Chloro-1-nitro-2-(pyrid-2-yl)aminobenzène.

On prépare ce composé selon le mode opératoire décrit dans Eur. J. Med. Chem. - Chim. Ther., 1983, 18 (6), 495-500.

B) 4-Ethoxy-1-nitro-2-(pyrid-2-yl)aminobenzène.

A une solution d'éthylate de sodium préparée à partir de 0,8 g de sodium dans 50 ml d'EtOH, on ajoute 6,2 g du composé obtenu à l'étape précédente et chauffe à reflux pendant 3 heures. On concentre sous vide le mélange réactionnel, extrait le résidu à l'AcOEt, lave à l'eau, sèche sur $Na_2SO_4$ et évapore sous vide le solvant. On chromatographie le résidu sur silice en éluant par le mélange DCM/pentane (50/50 ; v/v). On obtient 3,5 g du produit attendu que l'on utilise tel quel à l'étape suivante.

C) 1-Amino-4-éthoxy-2-(pyrid-2-yl)aminobenzène.

On hydrogène à TA et à pression atmosphérique un mélange de 3,5 g du composé obtenu à l'étape précédente, 0,2 g de palladium sur charbon à 5 % dans 250 ml d'AcOEt. On filtre le catalyseur et évapore sous vide le filtrat. On obtient 2,6 g du produit attendu, F = 95°C.

D) 4-Ethoxy-1-éthoxycarboxamido-2-(pyrid-2-yl)aminobenzène et 4-éthoxy-1-éthoxycarboxamido-2-[N-éthoxycarbonyl-N-(pyrid-2-yl)amino]benzène.

A une solution de 3,6 g du composé obtenu à l'étape précédente, 2 ml de triéthylamine dans 100 ml de DCM, on ajoute 2 ml de chloroformiate d'éthyle et laisse 2 heures sous agitation à TA. On concentre sous vide le mélange réactionnel, extrait le résidu à l'AcOEt, lave à l'eau, sèche sur $Na_2SO_4$ et évapore sous vide le solvant. On obtient 3,2 g du mélange des deux composés cités en titre de l'étape D) et que l'on utilise tel quel à l'étape suivante.

E) 5-Ethoxy-1,3-dihydro-3-(pyrid-2-yl)-2*H*-benzimidazol-2-one.

A une solution d'éthylate de sodium préparée à partir de 0,6 g de sodium dans 100 ml d'EtOH, on ajoute 3,2 g du mélange des composés obtenus à l'étape précédente, et chauffe à reflux pendant 3 heures. On concentre sous vide le mélange réactionnel, extrait le résidu à l'AcOEt, lave à l'eau, sèche sur $Na_2SO_4$ et évapore sous vide le solvant. On obtient 2,7 g du produit attendu après cristallisation dans l'éther iso, F = 205°C.

Préparation 32

3-Cyclopentyl-5-éthoxy-1,3-dihydro-2*H*-benzimidazol-2-one.

A) 4-Chloro-2-cyclopentylamino-1-nitrobenzène.

On laisse une nuit sous agitation à TA, un mélange de 20 g de 4-chloro-1,2-dinitrobenzène, 20 g de cyclopentylamine dans 50 ml d'EtOH 95. On essore le précipité formé, le lave à l'EtOH 95. On obtient 14 g du produit attendu, F = 75°C.

B) 2-Cyclopentylamino-4-éthoxy-1-nitrobenzène.

A une solution d'éthylate de sodium préparée à partir de 2 g de sodium dans 150 ml d'EtOH, on ajoute 20 ml de tris[2-(2-méthoxyéthoxy)éthyl]amine et 14 g du composé obtenu à l'étape précédente puis chauffe à reflux pendant 24 heures. On concentre sous vide le mélange réactionnel, extrait le résidu à l'AcOEt, lave à l'eau, sèche sur $Na_2SO_4$ et évapore sous vide le solvant. On chromatographie le résidu sur silice en éluant par le mélange DCM/heptane (50/50 ; v/v). On obtient 10 g du produit attendu que l'on utilise tel quel à l'étape suivante.

C) 1-Amino-2-cyclopentylamino-4-éthoxybenzène.

On hydrogène pendant 3 heures à TA et sous 2 bars de pression, un mélange de 10 g du composé obtenu à l'étape précédente, 0,6 g de palladium sur charbon à 5 % dans 200 ml d'EtOH 95. On filtre le catalyseur et évapore sous vide le filtrat. On obtient 8,2 g du produit attendu que l'on utilise tel quel à l'étape suivante.

D) 2-Cyclopentylamino-4-éthoxy-1-éthoxycarboxamidobenzène.

On chauffe à reflux pendant 5 heures un mélange de 8 g du composé obtenu à l'étape précédente, 8 ml de chloroformiate d'éthyle dans 50 ml de chloroforme. On concentre sous vide le mélange réactionnel, reprend le résidu dans le mélange, à chaud, éther iso/AcOEt (75/25 ; v/v), essore le précipité et le lave à

l'éther iso. On obtient 9,5 g du produit attendu, F = 173°C.

E) 3-Cyclopentyl-5-éthoxy-1,3-dihydro-2*H*-benzimidazol-2-one.

A une solution d'éthylate de sodium préparée à partir de 0,9 g de sodium dans 225 ml d'EtOH, on ajoute 9,5 g du composé obtenu à l'étape précédente, et chauffe à reflux pendant 18 heures. On concentre sous vide le mélange réactionnel, extrait le résidu à l'AcOEt, lave à l'eau, sèche sur $Na_2SO_4$ et évapore sous vide le solvant. On obtient 4 g du produit attendu après cristallisation dans le mélange AcOEt/éther iso (50/50 ; v/v), F = 178°C.

Par ailleurs, en utilisant le mode opératoire décrit dans la partie générale, on a préparé les chlorures de benzènesulfonyle décrits dans le tableau 1 ci-après :

## TABLEAU 1

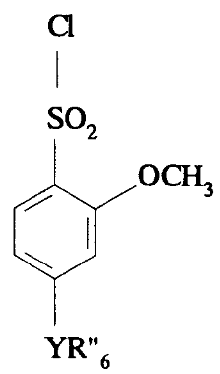

| Y | R"6 | F°C |
|---|---|---|
| S | $CH_3$ | 85 |
| O | $CH_2Bz$ | 95 |
| O | $CH_2CO_2Et$ | 89 |
| O | $(CH_2)_3Br$ | 106-108 |

EXEMPLE 1

5-chloro-1,3-dihydro-1-(2-méthoxy-4-nitrobenzènesulfonyl)-3-phényl-2*H*-benzimidazol-2-one.

A une solution de 1,223 g de 5-chloro-1,3-dihydro-3-phényl-2*H*-benzimidazol-2-one dans 12 ml de DMF à la température ambiante on a ajouté par portions et sous agitation 0,15 g d'hydrure de sodium à 80 % dans de l'huile ; puis on a introduit 1,384 g de chlorure de 2-méthoxy-4-nitrobenzènesulfonyle et on a maintenu le milieu réactionnel sous agitation à la température ambiante pendant 24 heures. On a évaporé ensuite le solvant sous vide et on a repris le résidu obtenu dans de l'eau. On a extrait ensuite par DCM et on a lavé à l'eau, puis on a séché sur sulfate de sodium et on a évaporé à sec. Le résidu obtenu a été chromatographié sur silice en éluant avec un mélange DCM/AcOEt (92/8 v/v). On a obtenu le produit attendu sous la forme d'un produit jaune qui a cristallisé dans EtOH absolu, m = 1,870 g, F > 250° C

EXEMPLE 2

3-Cyclohexyl-1,3-dihydro-5-méthoxy-1-(4-nitrobenzènesulfonyl)-2*H*-benzimidazol-2-one.

A une solution de 0,493 g de 3-cyclohexyl-1,3-dihydro-5-méthoxy-2*H*-benzimidazol-2-one dans 5 ml de DMF, on a ajouté par portions et sous agitation 0,066 g d'hydrure de sodium à 80 % dans de l'huile. On a laissé sous agitation pendant 30 minutes ; puis on a refroidi au bain de glace et on a ajouté 0,490 g de chlorure de

4-nitrobenzènesulfonyle. On a maintenu 24 heures sous agitation tout en laissant remonter la température à la température ambiante. On a évaporé sous vide le solvant et on a repris le résidu dans de l'eau. On a extrait ensuite par DCM et on a lavé à l'eau, puis on a séché sur sulfate de sodium et évaporé à sec. Le résidu a été chromatographié sur silice en éluant avec un mélange DCM/AcOEt (95/5 v/v).

On a obtenu le produit attendu sous la forme d'un solide jaune qui a été cristallisé dans EtOH absolu, m = 0,580 g, F = 160° C

EXEMPLE 3

5-chloro-1,3-dihydro-1-(2-méthoxy-4-nitrobenzènesulfonyl)-3-cyclohexyl--2*H*-benzidimidazol-2-one.

A une solution de 1 g de 5-chloro-3-cyclohexyl-1,3-dihydro-2*H*-benzimidazol-2-one dans 10 ml de DMF à la température ambiante on a ajouté par portions et sous agitation 0,13 g d'hydrure de sodium à 80 % dans de l'huile ; puis on a introduit 1,1 g de chlorure de 2-méthoxy-4-nitrobenzènesulfonyle et on a maintenu le milieu réactionnel sous agitation à la température ambiante pendant 4 heures. On a évaporé ensuite le solvant sous vide et on a repris le résidu obtenu dans de l'eau. On a extrait ensuite par DCM et on a lavé à l'eau, puis on a séché sur sulfate de sodium et on a évaporé à sec. Le résidu obtenu a été chromatographié sur silice en éluant avec un mélange DCM/AcOEt (95/5 v/v). On a obtenu le produit attendu sous la forme d'un solide légèrement jaune qui a cristallisé dans EtOH absolu, m = 1,52 g, F = 185° C

EXEMPLE 4

5-chloro-1,3-dihydro-1-(4-nitrobenzènesulfonyl)-3-cyclohexyl-2*H*-benzimidazol-2-one.

A une solution de 0,5 g de 5-chloro-3-cyclohexyl-1,3-dihydro-2*H*-benzimidazol-2-one dans 5 ml de DMF, on a ajouté par portions et sous agitation 0,066 g d'hydrure de sodium à 80 % dans de l'huile. On a laissé sous agitation pendant 30 minutes ; puis on a introduit 0,49 g de chlorure de 4-nitrobenzènesulfonyle. On a maintenu le milieu réactionnel 24 heures sous agitation à la température ambiante. On a évaporé sous vide le solvant et on a repris le résidu dans de l'eau. On a extrait ensuite par DCM et on a lavé à l'eau, puis on a séché sur sulfate de sodium et évaporé à sec. Le résidu a été chromatographié sur silice en éluant avec un mélange DCM/AcOEt (95/5 v/v).

On a obtenu le produit attendu sous la forme d'un solide jaune pâle qui a été cristallisé dans EtOH absolu, m = 0,57 g, F = 162° C

EXEMPLE 5

5-éthoxy-1,3-dihydro-1-[2-méthoxy-4-(N,N-diméthylamino)benzènesulfonyl] -3-cyclohexyl-2*H*-benzimidazol-2-one.

A) 5-éthoxy-1,3-dihydro-1-(2-méthoxy-4-nitrobenzènesulfonyl)-3-cyclohexyl-2*H*-benzimidazol-2-one.

A une solution de 4,95 g de 5-éthoxy-3-cyclohexyl-1,3-dihydro-2*H*-benzimidazol-2-one dans 40 ml de THF et 20 ml de DMF on a ajouté 0,5 g d'hydrure de sodium à 60 % dans de l'huile.

On a agité le milieu réactionnel pendant 30 minutes à 20° C puis on a ajouté en 5 minutes à 20° C 5,3 g de chlorure de 2-méthoxy-4-nitrobenzènesulfonyle dans 15 ml de THF. On a maintenu le milieu réactionnel sous agitation pendant 4 heures à 20° C, puis on a concentré sous vide et repris le résidu dans de l'eau. On a filtré le précipité, qui a été cristallisé dans de l'éthanol absolu puis recristallisé dans de l'isopropanol. On a obtenu 9,1 g du produit attendu sous la forme d'un solide jaune, F = 162° C

B) 5-éthoxy-1,3-dihydro-1-(2-méthoxy-4-aminobenzènesulfonyl)-3-cyclohexyl-2*H*-benzimidazol-2-one.

On a porté au reflux une suspension contenant 8,9 g du composé obtenu à l'étape précédente, 11 g de fer en poudre, 35 ml d'éthanol 95. Puis on a ajouté en 15 minutes au mélange réactionnel résultant, une solution de 4 ml d'acide chlorhydrique concentré dans 15 ml d'éthanol, puis 10 ml d'AcOH. On a maintenu le mélange réactionnel au reflux pendant 3 heures. On a refroidi et on a lavé avec une solution aqueuse de $NaHCO_3$ saturée, extrait au DCM, séché sur $Na_2SO_4$ et concentré. On a recristallisé dans de l'éther isopropylique. On a obtenu 6,7 g du produit attendu, F = 228° C

C) 5-éthoxy-1,3-dihydro-1-[2-méthoxy-4-(N,N-diméthylamino)benzènesulfonyl]-3-cyclohexyl-2*H*-benzimidazol-2-one.

A une suspension de 0,5 g du composé obtenu à l'étape précédente dans 10 ml de $CH_3CN$ et 4 ml de formaldéhyde en solution aqueuse (37 %) on a ajouté à 20° C 0,5 g de cyanoborohydrure de sodium dans 0,15

ml de AcOH, puis on a agité le mélange réactionnel pendant 48 heures à 20° C. Le milieu réactionnel a ensuite été repris par de l'éther éthylique, lavé à l'eau puis séché sur sulfate de sodium et concentré. Le résidu a été chromatographié sur silice en éluant avec du DCM. On a obtenu 0,1 g du produit attendu sous la forme d'un solide jaune, qui a été cristallisé dans de l'éthanol absolu et recristallisé dans de l'isopropanol, F = 220° C.

EXEMPLE 6

5-éthoxy-1,3-dihydro-1-[2-méthoxy-4-(Δ3-pyrrolin-1-yl)benzènesulfonyl]-3-cyclohexyl-2*H*-benzimidazol-2-one.

On a porté au reflux pendant 2 heures une suspension contenant 2 g du composé obtenu à l'étape B) de l'exemple 5, 1,5 g de cis-1,4-dichlorobut-2-ène dans 30 ml de DMF et 1,5 ml de triéthylamine. Puis on a versé le milieu réactionnel sur un mélange eau/glace et on a extrait à l'AcOEt. La phase organique a été lavée à l'eau, séchée sur sulfate de sodium, puis évaporée sous vide. Le résidu a été chromatographié sur gel de silice en éluant avec du DCM. On a obtenu 0,64 g du produit attendu qui cristallise dans un mélange acétate d'éthyle/éther iso (50/50 v/v), F = 212° C.

EXEMPLE 7

5-chloro-1,3-dihydro-1-(2-méthoxy-4-aminobenzènesulfonyl)-3-cyclohexyl-2*H*-benzimidazol-2-one.

On a soumis à une hydrogénation sous 50 bars 1,8 g ($4.10^{-3}$ mole) du composé obtenu à l'exemple 3, dans EtOH 95° en présence de nickel de Raney®. On a filtré sur Célite® le catalyseur et on a rincé avec de l'EtOH 95° chaud et on a évaporé sous vide le filtrat. On a obtenu 1,22 g du produit attendu sous la forme d'un solide blanc. Le produit a cristallisé dans l'éthanol absolu, F = 211°C.

EXEMPLE 8

5-chloro-1,3-dihydro-1-(2-méthoxy-4-aminobenzènesulfonyl)-3-phényl-2*H*-benzimidazol-2-one.

On a soumis à une hydrogénation sous 50 bars de pression et à température ambiante, pendant 24 heures, un mélange de 1,8 g ($4.10^{-3}$ mole) de 5-chloro-1,3-dihydro-1-(2-méthoxy-4-nitrobenzènesulfonyl)-3-phényl-2*H*-benzimidazol-2-one obtenu à l'exemple 1 et de 1 g de nickel de Raney® dans 70 ml d'éthanol 95. On a ensuite filtré sur Celite® le catalyseur et évaporé sous vide le filtrat. Le résidu a été chromatographié sur silice en éluant avec un mélange de DCM/MeOH (99/1 v/v). On a obtenu 0,6 g du produit attendu sous la forme d'un solide ocre, F = 205° C.

EXEMPLE 9

5-éthoxy-1,3-dihydro-1-[2-méthoxy-4-(2-éthylbutyrylamino)benzènesulfonyl]-3-cyclohexyl-2*H*-benzimidazol-2-one.

On a mélangé 0,5 g du composé obtenu à l'étape B, exemple 5 dans 20 ml de DCM avec 0,5 g de chlorure de l'acide 2-éthylbutyrique et 0,5 g de pyridine. On a concentré sous vide, extrait à l'éther éthylique, puis lavé à l'eau, à l'acide chlorhydrique 1N, puis à l'eau. Le résidu a été chromatographié sur silice en éluant avec DCM. On a obtenu le produit attendu qui a cristallisé dans l'éther isopropylique, m = 0,17 g, F = 168°C.

EXEMPLE 10

5-éthoxy-1,3-dihydro-1-[2-méthoxy-4-(2-méthylphénylcarbonyl-amino)benzènesulfonyl]-3-cyclohexyl-2*H*-benzimidazol-2-one.

On a mélangé à 20° C, 0,46 g du composé préparé à l'étape B/ de l'exemple 5, 20 ml de DCM et 0,5 g de pyridine, puis on a ajouté 0,18 g de chlorure de 2-méthylbenzoyle dans 10 ml de DCM. On a agité le mélange réactionnel pendant 16 heures, puis on a concentré sous vide. Le résidu a été lavé à l'eau, à l'acide chlorhydrique 1N, au carbonate de sodium 10 %, puis à l'eau. On a séché sur sulfate de sodium puis on a évaporé à sec. On a obtenu le produit attendu sous la forme d'un solide blanc qui a cristallisé dans l'éther isopropylique, m = 0,3 g, F = 180° C.

EXEMPLE 11

Fumarate de 5-éthoxy-1,3-dihydro-1-[2-méthoxy-4-(diéthylaminoacétamido) benzènesulfonyl]-3-cyclohexyl-2*H*-benzimidazol-2-one.

A) 5-éthoxy-1,3-dihydro-1-(2-méthoxy-4-chloroacétamidobenzènesulfonyl)-3-cyclohexyl-2*H*-benzimidazol-2-one.

On a mélangé 0,52 g du composé obtenu à l'étape B/ de l'exemple 5, 25 ml de DCM et 0,5 g de pyridine. Puis on y a ajouté en 5 minutes à 20° C, 0,54 ml de chlorure de chloroacétyle dans 10 ml de DCM. On a agité le mélange réactionnel pendant 2 heures à 20°C, puis on a concentré sous vide à 20° C. On a extrait avec AcOEt, lavé à l'eau puis avec de l'acide chlorhydrique 1N, puis de l'eau. On a séché sur sulfate de sodium et évaporé le solvant. On a obtenu le produit attendu qui a cristallisé dans de l'éther isopropylique, m = 0,6 g, F = 232° C.

B)Furamate de 5-éthoxy-1,3-dihydro-1-[2-méthoxy-4-(diéthylamino-acétamido)benzènesulfonyl]-3-cyclohexyl-2*H*-benzimidazol-2-one.

On a mélangé à 20° C, 0,55 g du composé obtenu à l'étape A) ci-dessus, avec 20 ml de THF et 10 ml de diéthylamine. On a laissé le mélange réactionnel au repos pendant 15 heures puis on a concentré sous vide. On a repris à l'eau, extrait à AcOEt, lavé par HCl 1N, à l'eau ; on a séché sur $Na_2SO_4$ et évaporé sous vide le solvant. Le résidu est chromatographié sur silice en éluant avec DCM puis DCM/AcOEt (95/5, v/v). On a obtenu 0,5 g de cire que l'on a salifié par 0,1 g d'acide fumarique dans 20 ml d'acétone. Le sel obtenu est soluble dans l'éther éthylique, m = 0,2 g, F = 124° C.

EXEMPLE 12

5-Ethoxy-1,3-dihydro-1-(2-méthoxy-4-benzyloxycarbonylbenzènesulfonyl)-3-cyclohexyl-2*H*-benzimidazol-2-one.

A une solution de 2,4 g de 5-éthoxy-3-cyclohexyl-1,3-dihydro-2*H*- benzimidazol-2-one dans 50 ml de THF et 50 ml de DMF, on a ajouté par petites fractions, à température ambiante 0,5 g d'hydrure de sodium à 60 % dans l'huile et on a agité pendant une demi-heure, toujours à température ambiante. Puis on a ajouté 3,4 g de chlorure de 2-méthoxy-4-benzyloxycarbonylbenzènesulfonyle et on a laissé le milieu réactionnel sous agitation pendant 4 heures à température ambiante. On a concentré sous vide pour éliminer le THF résiduel et on a repris le résidu avec de l'eau. On a extrait ensuite avec AcOEt, lavé à l'eau, sèché la phase organique sur sulfate de sodium et a on concentré sous vide. Le résidu obtenu a ensuite été purifié par chromatographie sur silice en éluant avec DCM. Par cristallisation dans l'éther iso, on a obtenu 4,2 g du produit attendu sous forme d'un solide blanc, F = 131° C.

EXEMPLE 13

5-éthoxy-1,3-dihydro-1-(2-méthoxy-4-carboxybenzènesulfonyl)-3-cyclohexyl-2*H*-benzimidazol-2-one.

On a soumis à une hydrogénation, à la température ambiante et à la pression atmosphérique, 4 g de 5-éthoxy-1,3-dihydro-1-(2-méthoxy-4-benzyloxycarbonylbenzènesulfonyl)-3-cyclohexyl-2*H*-benzimidazol-2-one dans 100 ml d'AcOEt en présence de 200 mg de Pd/C. On a filtré le catalyseur sur Célite® et on a concentré le filtrat sous vide. Par cristallisation dans l'éther iso, on a obtenu 2,47 g du composé attendu sous forme d'un solide blanc, F = 205° C.

EXEMPLE 14

5-Ethoxy-1,3-dihydro-1-[2-méthoxy-4-(N-(tert-butyl)carbamoyl)benzènesulfonyl]-3-cyclohexyl-2*H*-benzimidazol-2-one.

On a mélangé 700 mg du composé obtenu à l'exemple précédent, 780 mg de BOP et 1 ml de *tert*-butylamine, dans 50 ml de DCM et 2 ml de DIPEA et on a laissé le milieu réactionnel sous agitation pendant 30 minutes à température ambiante. On a concentré sous vide, on a repris le résidu avec de l'eau et on extrait avec DCM. On a lavé avec HCl 1N puis avec NaOH 1N, on a séché la phase organique sur sulfate de sodium et on a concentré sous vide. Le résidu obtenu a été ensuite purifié par chromatographie sur silice en éluant avec un mélange DCM/AcOEt 97,5/2,5 (v/v). On a obtenu 500 mg du composé attendu sous forme d'un solide

blanc, m = 2,47 g, F = 252°C.

EXEMPLE 15

5-éthoxy-1,3-dihydro-1-[2-méthoxy-4-(N-(1-éthoxycarbonyl-1-méthyléthyl)carbamoyl)benzènesulfonyl]-3-cyclohexyl-2*H*-benzimidazol-2-one

On a mélangé 500 mg du composé obtenu à l'exemple 13, 300 mg de chlorhydrate de 1-éthoxycarbonyl-1-méthyléthylamine, et 550 mg de BOP, dans 2 ml de DIPEA et 50 ml de DCM, et on a laissé le milieu réactionnel sous agitation pendant 30 minutes à température ambiante. Puis on a concentré sous vide et on a repris le résidu avec de l'eau. On a extrait ensuite avec AcOEt, lavé successivement avec HCl 1N, NaOH 1N puis avec de l'eau, on a sèché sur sulfate de sodium et on a concentré sous vide. Le résidu a été purifié par chromatographie sur colonne de silice en éluant avec un mélange DCM/AcOEt 97/3 (v/v). Par cristallisation dans un mélange AcOEt/éther iso 10/90 (v/v), on a obtenu 170 mg du produit attendu sous forme d'un solide blanc, F = 202° C.

EXEMPLE 16

5-éthoxy-1,3-dihydro-1-[2-méthoxy-4-(N',N'-diéthyluréido)benzènesulfonyl]-3-cyclohexyl-2*H*-benzimidazol-2-one.

A) 5-éthoxy-1,3-dihydro-1-[2-méthoxy-4-phénoxycarbonylaminobenzènesulfonyl]-3-cyclohexyl-2*H*-benzimidazol-2-one.

A une solution de 1,1 g de 5-éthoxy-1,3-dihydro-1-(2-méthoxy-4-aminobenzènesulfonyl)-3-cyclohexyl-2*H*-benzimidazol-2-one, obtenu à l'exemple 5 (étape B), dans 25 ml de THF, on a ajouté sous agitation et à 5° C, 0,29 g de NaOH en pastilles dans 2 ml d'eau. On a ajouté ensuite 1 ml de chloroformiate de phényle, en maintenant l'agitation. Le mélange réactionnel a alors été laissé sous agitation pendant 5 heures en laissant la température remonter jusqu'à 20° C. Puis on a concentré sous vide, on a repris le résidu avec de l'eau et on a extrait avec de l'éther. On a lavé à l'eau, on a sèché la phase organique sur sulfate de sodium et on a concentré sous vide. Par cristallisation dans l'éther iso, on a obtenu 1 g du produit attendu, F = 197° C.

B) 5-éthoxy-1,3-dihydro-1-[2-méthoxy-4-(N',N'-diéthyluréido)benzènesulfonyl]-3-cyclohexyl-2*H*-benzimidazol-2-one.

On a mélangé, à 20° C et sous agitation, 0,5 g du produit obtenu à l'étape précédente dans 15 ml d'EtOH, et 0,5 g de diéthylamine dans 10 ml de DCM. On a laissé alors reposer le mélange réactionnel pendant 15 heures puis on a concentré sous vide. On a repris le résidu avec de l'eau, on a extrait avec de l'éther, puis on a lavé successivement avec de l'eau, une solution de NaOH 1N et encore de l'eau. On a sèché la phase organique sur sulfate de sodium et on a concentré sous vide. Le produit a cristallisé dans l'éther iso, pour donner 0,33 g du composé attendu sous forme d'un solide jaunâtre, F = 165°C.

EXEMPLE 17

5-chloro-1,3-dihydro-1-[2-méthoxy-4-(N',N'-diéthyluréido)benzènesulfonyl]-3-cyclohexyl-2*H*-benzimidazol-2-one.

A) 5-chloro-1,3-dihydro-1-[2-méthoxy-4-phénoxycarbonylaminobenzène-sulfonyl]-3-cyclohexyl-2*H*-benzimidazol-2-one.

A une solution de 1,05 g du composé obtenu à l'exemple 7 dans 25 ml de THF, on a ajouté sous agitation et à 5° C 0,24 g de NaOH en pastilles dans 2 ml d'eau. On a ajouté ensuite 1 ml de chloroformiate de phényle, en maintenant l'agitation. Le mélange réactionnel a alors été laissé pendant 5 heures sous agitation en laissant la température remonter jusqu'à 20° C. Puis on a concentré sous vide, on a repris le résidu avec de l'eau et on a extrait à l'éther. On a lavé à l'eau, sèché la phase organique sur sulfate de sodium et concentré sous vide. Par cristallisation dans l'éther iso, on a obtenu 1,35 g du produit attendu, F = 236° C.

B) 5-chloro-1,3-dihydro-1-[2-méthoxy-4-(N',N'-diéthyluréido)benzènesulfonyl]-3-cyclohexyl-2*H*-benzimidazol-2-one.

On a mélangé à 20° C et sous agitation, 0,5 g du produit obtenu à l'étape précédente dans 15 ml d'EtOH, et 0,5 g de diéthylamine dans 10 ml de DCM. On a laissé alors reposer le mélange réactionnel pendant 20

heures, puis on a concentré sous vide. On a repris le résidu avec une solution de NaOH 1N, on a extrait avec AcOEt, on a lavé à l'eau, on a sèché la phase organique sur sulfate de sodium et on a concentré sous vide. Par cristallisation dans l'éther iso, on a obtenu 0,45 g du produit attendu sous forme d'un solide blanc, F = 250° C.

EXEMPLE 18

5-éthoxy-1,3-dihydro-1-[2-méthoxy-4-(pipérid-1-ylcarbonylamino)benzènesulfonyl]-3-cyclohexyl-2*H*-benzimidazol-2-one.

A une solution de 500 mg du composé obtenu à l'exemple 16 (étape A) dans 25 ml de DCM, on a ajouté 2 ml de pipéridine. On a agité ensuite le mélange réactionnel pendant 18 heures à température ambiante. On a concentré à sec et on a passé le produit ainsi obtenu sur silice en éluant avec DCM puis avec un mélange DCM/AcOEt 95/5 (v/v). On a obtenu 0,36 g du produit attendu sous forme de cristaux blancs, F = 232° C.

EXEMPLE 19

5-chloro-1,3-dihydro-1-[2-méthoxy-4-(N',N'-diéthyluréido)benzènesulfonyl]-3-cycloheptyl-2*H*-benzimidazol-2-one.

A) 5-chloro-1,3-dihydro-1-[2-méthoxy-4-nitrobenzènesulfonyl]-3-cycloheptyl-2*H*-benzimidazol-2-one.

A une solution de 3 g de 5-chloro-3-cycloheptyl-1,3-dihydro-2*H*-benzimidazol-2-one dans 30 ml de THF, on a ajouté par portions et sous agitation 600 mg d'hydrure de sodium à 60 % dans l'huile. Puis on a ajouté 3 g de chlorure de 4-nitro-2-méthoxybenzènesulfonyle, et on a laissé le mélange réactionnel sous agitation pendant 18 heures. On a coulé alors le mélange réactionnel sur un mélange eau + glace ; on a extrait à l'acétate d'éthyle ; on a lavé à l'eau ; on a sèché la phase organique sur sulfate de sodium et on a concentré à sec. Par cristallisation dans un mélange éther iso/AcOEt 60/40 (v/v), on a obtenu 3,5 g du produit attendu, F = 193° C.

B) 5-chloro-1,3-dihydro-1-[2-méthoxy-4-aminobenzènesulfonyl]-3-cycloheptyl-2*H*-benzimidazol-2-one.

On a porté au reflux pendant 4 heures un mélange de 1,5 g de produit obtenu à l'étape précédente, de 6 ml d'alcool, 6 ml d'eau, 2 g de fer en poudre et de 1 ml d'HCl concentré dans 3 ml d'eau. On a dilué ensuite le milieu réactionnel dans un mélange eau + glace, on a filtré l'insoluble et on a lavé le précipité obtenu avec AcOEt. On a extrait le filtrat avec AcOEt, on a lavé à l'eau, on a sèché la phase organique sur sulfate de sodium et on a concentré à sec. Par cristallisation dans l'éthanol, on a obtenu 0,81 g du produit attendu, F = 219° C.

C) 5-chloro-1,3-dihydro-1-[2-méthoxy-4-(phénoxycarbonylamino)benzènesulfonyl]-3-cycloheptyl-2*H*-benzimidazol-2-one.

A une solution de 0,5 g du produit obtenu à l'étape précédente dans 15 ml de THF, on a ajouté sous agitation et à une température inférieure ou égale à 10° C, 60 mg de soude en pastilles dans 1 ml d'eau. Puis on a ajouté 0,6 ml de chloroformiate de phényle, et on a agité le mélange réactionnel pendant 3 heures. On a concentré à sec et on a repris à l'acétate d'éthyle. On a lavé à l'eau, on a sèché la phase organique sur sulfate de sodium et on a concentré à sec. Par cristallisation dans l'éther iso, on a obtenu 0,59 g du produit attendu, F = 234° C.

D) 5-chloro-1,3-dihydro-1-[2-méthoxy-4-(N',N'-diéthyluréido)benzènesulfonyl]-3-cycloheptyl-2*H*-benzimidazol-2-one.

On a mélangé 500 mg du produit obtenu à l'étape précédente et 2 ml de diéthylamine dans 30 ml de DCM et on a agité le mélange réactionnel pendant 2 heures à la température ambiante. Puis on a concentré à sec et on a cristallisé le résidu dans 30 ml d'un mélange AcOEt/éther iso 50/50 (v/v) pour donner 0,27 g du composé attendu sous forme de cristaux blancs, F = 218° C.

EXEMPLE 20

5-chloro-1,3-dihydro-1-(3,4-diméthoxybenzènesulfonyl)-3-phényl-2*H*-benzimidazol-2-one.

A une solution de 490 mg de 5-chloro-1,3-dihydro-3-phényl-2*H*-benzimidazol-2-one dans 5 ml de DMF on a ajouté par portions 66 mg d'hydrure de sodium à 80 % dans l'huile, et on a maintenu le milieu réactionnel sous agitation pendant 30 minutes. On a ensuite introduit 520 mg de chlorure de 3,4-diméthoxybenzènesulfonyle et on a maintenu le milieu réactionnel sous agitation pendant une nuit à la température ambiante. On a ensuite évaporé le solvant sous vide et on a repris le résidu obtenu dans de l'eau. On a extrait ensuite par

DCM et on a lavé à l'eau, puis on a séché sur sulfate de sodium et on a évaporé à sec. Le résidu obtenu a été chromatographié sur silice en éluant avec un mélange DCM/AcOEt (95/5 v/v). On a obtenu le produit attendu sous la forme d'un solide blanc qui a cristallisé dans EtOH absolu, m = 0,71 g, F = 154° C.

En procédant selon le mode opératoire décrit ci-dessus on a préparé les composés de formule (I) selon l'invention décrits dans le tableau 2 ci-après :

**TABLEAU 2**

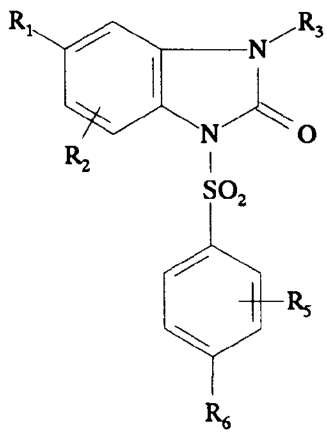

| EX | $R_1$ | $R_2$ | $R_3$ | $R_5$ | $R_6$ | F° C |
|---|---|---|---|---|---|---|
| 21 | $CH_3O$ | H | 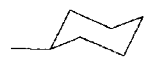 | 2-$CH_3O$ | 4-$CH_3O$ | 118 |
| 22 | $CH_3O$ | H | 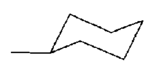 | 3-$CH_3O$ | 4-$CH_3O$ | 139 |
| 23 | Cl | H | 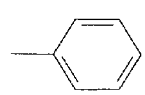 | 2-$CH_3O$ | 4-$CH_3O$ | 196 |
| 24 | $C_2H_5O$ | H | 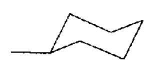 | 2-$CH_3O$ | 4-$CH_3O$ | 163 |
| 25 | Cl | H | 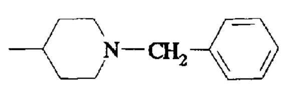 | 2-$CH_3O$ | 4-$CH_3O$ | 88 |
| 26 | Cl | H | 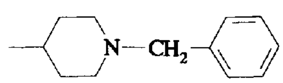 | 3-$CH_3O$ | 4-$CH_3O$ | 95 |

TABLEAU 2 (suite)

| EX | $R_1$ | $R_2$ | $R_3$ | $R_5$ | $R_6$ | F° C |
|---|---|---|---|---|---|---|
| 27 | Cl | H | 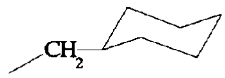 | 3-$CH_3O$ | 4-$CH_3O$ | 142 |
| 28 | Cl | H | 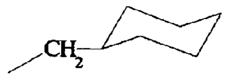 | 2-$CH_3O$ | 4-$CH_3$ | 136 |
| 29 | Cl | H | 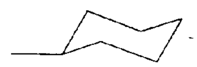 | 3-$CH_3O$ | 4-$CH_3O$ | 148 |
| 30 | Cl | H | 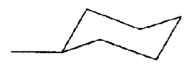 | 2-$CH_3O$ | 4-$CH_3O$ | 156 |
| 31 | Cl | H | 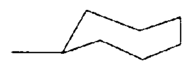 | 2-$CH_3O$ | 4-$CH_3O$ | 153 |

EXEMPLE 32

Iodure de N-benzyl-N-méthyl-4-[5-chloro-2,3-dihydro-1-(2,4-diméthoxybenzènesulfonyl)-2-oxo-1*H*-benzimidazol-3-yl]pipéridinium.

On a mélangé 0,75 g du composé de l'exemple 25 avec 10 ml d'iodure de méthyle dans 200 ml d'éther éthylique. On a maintenu le milieu réactionnel pendant 5 jours à 20° C. Le précipité formé a été séparé, lavé à l'éther éthylique et séché. On a obtenu 0,35 g de l'iodure attendu, F = 178° C.

EXEMPLE 33

5-Ethoxy-1,3-dihydro-3-[2-(N,N-diisopropylamino)éthyl]-1-(2,4-diméthoxybenzènesulfonyl)-2*H*-benzimidazol-2-one.

A une solution de 0,5 g de 5-éthoxy-1,3-dihydro-3-[2-(N,N-diisopropylamino) éthyl]-2*H*-benzimidazol-2-one dans 7 ml de THF, on ajoute par portions 0,05 g d'hydrure de sodium à 60 % dans l'huile et laisse 30 minutes sous agitation à TA. On ajoute ensuite 0,42 g de chlorure de 2,4-diméthoxybenzènesulfonyle et laisse 1 heure sous agitation à TA. On évapore le solvant sous vide, reprend le résidu à l'eau, extrait à l'AcOEt, lave à l'eau, sèche sur $Na_2SO_4$ et évapore sous vide le solvant. On obtient 0,7 g du produit attendu après cristallisation dans l'éther iso, F = 132°C.

EXEMPLE 34

5-Ethoxy-1,3-dihydro-1-(2,4-diméthoxybenzènesulfonyl)-3-[2-(morpholin-4-yl)éthyl]-2*H*-benzimidazol-2-one.

On prépare ce composé selon le mode opératoire décrit à l'EXEMPLE 33 à partir de 0,6 g de 5-éthoxy-1,3-dihydro-3-[2-(morpholin-4-yl)éthyl]-2*H*-benzimidazol-2-one. On obtient 0,75 g du produit attendu après cristallisation dans l'éther, F = 146-149°C.

EXEMPLE 35

1-[4-(N'-*tert*-Butyluréido)-2-méthoxybenzènesulfonyl]-3-cyclohexyl-5-éthoxy-1,3-dihydro-2*H*-benzimidazol-2-one.

On laisse 48 heures sous agitation à TA un mélange de 0,7 g du composé obtenu à l'EXEMPLE 16 étape A, 1 ml de *tert*-butylamine et 25 ml de DCM. On évapore sous vide le solvant et chromatographie le résidu sur silice en éluant par le mélange DCM/AcOEt (90/10 ; v/v). On obtient 0,41 g du produit attendu après cristallisation dans le mélange AcOEt/éther iso (50/50 ; v/v), F = 237°C.

EXEMPLE 36

1-[4-(N'-*tert*-Butyl-N'-méthyluréido)-2-méthoxybenzènesulfonyl]-3-cyclohexyl-5-éthoxy-1,3-dihydro-2*H*-benzimidazol-2-one.

On laisse 2 heures sous agitation à TA un mélange de 0,5 g du composé obtenu à l'EXEMPLE 16 étape A, 1 ml de N-méthyl-*tert*-butylamine et 20 ml de DCM. On évapore sous vide le solvant et chromatographie le résidu sur silice en éluant par le mélange DCM/AcOEt (95/5 ; v/v). On obtient 0,13 g du produit attendu après cristallisation dans le mélange éther iso/AcOEt (65/35 ; v/v), F = 208°C.

EXEMPLE 37

5-Ethoxy-1-[4-(N',N'-diéthyluréido)-2-méthoxybenzènesulfonyl]-1,3-dihydro-3-(1,1,3,3-tétraméthylbutyl)-2*H*-benzimidazol-2-one.

A) 5-Ethoxy-1,3-dihydro-1-(2-méthoxy-4-nitrobenzènesulfonyl)-3-(1,1,3,3-tétraméthylbutyl)-2*H*-benzimidazol-2-one.

A une solution de 1 g de 5-éthoxy-1,3-dihydro-3-(1,1,3,3-tétraméthylbutyl)2*H*-benzimidazol-2-one dans 10 ml de THF et 20 ml de DMF on ajoute par portions 0,2 g d'hydrure de sodium à 60 % dans l'huile et laisse 30 minutes sous agitation à TA. Puis on ajoute 1,2 g de chlorure de 2-méthoxy-4-nitrobenzènesulfonyle et laisse 5 heures sous agitation à TA. On verse le mélange réactionnel dans 200 ml d'eau, extrait à l'AcOEt, lave à l'eau, sèche sur $Na_2SO_4$ et évapore sous vide le solvant. On obtient 1,1 g du produit attendu après cristallisation dans l'éther iso, F = 158°C.

B) 1-(4-Amino-2-méthoxybenzènesulfonyl)-5-éthoxy-1,3-dihydro-3-(1,1,3,3-tétraméthylbutyl)-2*H*-benzimidazol-2-one.

On hydrogène à TA et à pression atmosphérique un mélange de 1,1 g du composé obtenu à l'étape précédente, 0,2 g de palladium sur charbon à 5 % dans 200 ml d'AcOEt. On filtre le catalyseur sur Célite® et évapore sous vide le filtrat. On obtient 0,56 g du produit attendu, F = 216°C.

C) 5-Ethoxy-1,3-dihydro-1-[2-méthoxy-4-(phénoxycarboxamido)benzènesulfonyl]-3-(1,1,3,3-tétraméthylbutyl)-2*H*-benzimidazol-2-one.

A une solution de 0,52 g du composé obtenu à l'étape précédente dans 30 ml de THF, on ajoute une solution de 0,25 g de NaOH en pastilles dans 10 ml d'eau. Puis on ajoute 1 ml de chloroformiate de phényle et laisse 5 heures sous agitation à TA. On évapore sous vide le mélange réactionnel, reprend le résidu à l'eau, extrait à l'AcOEt, lave à l'eau, sèche sur $Na_2SO_4$ et évapore sous vide le solvant. On obtient 0,69 g du produit attendu après cristallisation dans l'éther iso, F = 168°C.

D) 5-Ethoxy-1-[4-N',N'-diéthyluréido)-2-méthoxybenzènesulfonyl]-1,3-dihydro-3-(1,1,3,3-tétraméthylbutyl)-2*H*-benzimidazol-2-one.

A une solution de 0,67 g du composé obtenu à l'étape précédente dans 20 ml de DCM, on ajoute 1 ml de diéthylamine et laisse 6 heures sous agitation à TA. On dilue le mélange réactionnel avec du DCM, lave par une solution d'HCl 1N, par une solution de NaOH 1N, à l'eau, sèche sur $Na_2SO_4$ et évapore sous vide le solvant. On chromatographie le résidu sur silice en éluant par le mélange DCM/MeOH (99/1 ; v/v). On obtient 0,33 g du produit attendu après cristallisation dans l'éther iso, F = 225°C.

EXEMPLE 38

5-Ethoxy-1-[4-(N',N'-diéthyluréido)-2-méthoxybenzènesulfonyl]-1,3-dihydro-3-(4(a,e)-méthylcyclohexyl)-2*H*-benzimidazol-2-one.

A) 5-Ethoxy-1,3-dihydro-1-(2-méthoxy-4-nitrobenzènesulfonyl)-3-(4(a,e) méthylcyclohexyl)-2*H*-benzimidazol-2-one.

On prépare ce composé selon le mode opératoire décrit à l'EXEMPLE 37 étape A à partir de 1,4 g de 5-éthoxy-1,3-dihydro-3-(4(a,e)-méthylcyclohexyl)-2*H*-benzimidazol-2-one et de 1,5 ml de chlorure de 2-méthoxy-4-nitrobenzènesulfonyle dans 30 ml de DMF. On obtient 2 g du produit attendu après cristallisation dans l'éther iso, F = 147°C.

B) 1-(4-Amino-2-méthoxybenzènesulfonyl)-5-éthoxy-1,3-dihydro-3-(4(a,e)-méthylcyclohexyl)-2*H*-benzimidazol-2-one.

On hydrogène à TA et sous 30 bars de pression un mélange de 2 g du composé obtenu à l'étape précédente dans 50 ml d'EtOH 95 et 1,5 g de nickel de Raney®. On filtre le catalyseur sur Célite® et évapore sous vide le filtrat. On obtient 0,5g du produit attendu, F = 126°C.

C) 5-Ethoxy-1,3-dihydro-1-[2-méthoxy-4-(phénoxycarboxamido)benzènesulfonyl]-3-(4(a,e)-méthylcyclohexyl)-2*H*-benzimidazol-2-one.

On prépare ce composé selon le mode opératoire décrit à l'EXEMPLE 37 étape C à partir de 0,5 g du composé obtenu à l'étape précédente et de 1,5 ml de chloroformiate de phényle. On obtient 0,44 g du produit attendu après cristallisation dans l'éther iso, F = 193°C.

D) 5-Ethoxy-1-[4-(N',N'-diéthyluréido)-2-méthoxybenzènesulfonyl]-1,3-dihydro-3-(4(a,e)-méthylcyclohexyl)-2*H*-benzimidazol-2-one.

A une solution de 0,43 g du composé obtenu à l'étape précédente dans 30 ml de DCM, on ajoute 2 ml de diéthylamine et laisse une nuit sous agitation à TA. On évapore sous vide le mélange réactionnel et chromatographie le résidu sur silice en éluant par le mélange DCM/AcOEt (80/20 ; v/v). On obtient 0,345 g du produit attendu après cristallisation dans l'éther iso, F = 206°C.

EXEMPLE 39

5-Ethoxy-1-[4-(N',N'-diéthyluréido)-2-méthoxybenzènesulfonyl]-1,3-dihydro-3-(4(a,e)-hydroxycyclohexyl)-2*H*-benzimidazol-2-one.

A) 5-Ethoxy-1,3-dihydro-3-[4(a,e)-(tétrahydropyran-2(R,S)-yloxy)cyclohexyl]-1-(2-méthoxy-4-nitrobenzènesulfonyl)-2*H*-benzimidazol-2-one.

On prépare ce composé selon le mode opératoire décrit à l'EXEMPLE 37 étape A à partir de 1,45 g de 5-éthoxy-1,3-dihydro-3-[4(a,e)-(tétrahydropyran-2(R,S)-yloxy)cyclohexyl]-2*H*-benzimidazol-2-one et 1,1 g de chlorure de 2-méthoxy-4-nitrobenzènesulfonyle. On obtient 2,5 g d'huile qui cristallise, F = 132-136°C.

B) 1-(4-Amino-2-méthoxybenzènesulfonyle)-5-éthoxy-1,3-dihydro-3-[4(a,e)-(tétrahydropyran-2(R,S)-yloxy)cyclohexyl] -2*H*-benzimidazol-2-one.

On hydrogène à 30°C et à pression atmosphérique un mélange de 2,4 g du composé obtenu à l'étape précédente, 2 g de nickel de Raney® dans 30 ml de THF et 20 ml de MeOH. On filtre le catalyseur, le lave au DCM et concentre sous vide le filtrat. On obtient 2,2 g du produit attendu que l'on utilise tel quel à l'étape suivante.

C) 5-Ethoxy-1,3-dihydro-3-[4(a,e)-tétrahydropyran-2(R,S)-yloxy)cyclohexyl]-1-[2-méthoxy-4-(phénoxycarboxamido)benzènesulfonyl]-2*H*-benzimidazol-2-one.

On refroidit à 5°C un mélange de 2,1 g du composé obtenu à l'étape précédente, 3 ml de triéthylamine dans 10 ml de THF et ajoute une solution de 2 ml de chloroformiate de phényle dans 5 ml de THF. On laisse 3 heures sous agitation à TA et évapore sous vide le mélange réactionnel. On reprend le résidu à l'eau, extrait à l'AcOEt, lave par une solution saturée de $NaHCO_3$, à l'eau, sèche sur $Na_2SO_4$ et évapore sous vide le solvant. On obtient 2,5 g du produit attendu après cristallisation dans l'éther iso.

D) 5-Ethoxy-1-[4-(N',N'-diéthyluréido)-2-méthoxybenzènesulfonyl]1,3-dihydro-3-(4(a,e)-hydroxycyclohexyl)-2*H*-benzimidazol-2-one.

On laisse 20 heures sous agitation à TA un mélange de 2,4 g du composé obtenu à l'étape précédente, 8 g de diéthylamine dans 30 ml de THF. On évapore sous vide et obtient une huile jaune. On dissout cette huile dans 20 ml d'EtOH 96 et ajoute 2 ml d'HCl concentré puis laisse 2 heures sous agitation à TA. On évapore sous vide le mélange réactionnel à 40°C, reprend le résidu à l'eau, extrait à l'AcOEt, lave par une solution d'HCl

1N, à l'eau, par une solution de NaOH 1N, à l'eau, sèche sur $Na_2SO_4$ et évapore sous vide le solvant. On obtient 1,8 g du produit attendu après cristallisation dans l'éther iso, F = 221°C.

EXEMPLE 40

3-(Adamant-1-yl)-5-éthoxy-1-[4-(N',N'-diéthyluréido)-2-méthoxybenzène-sulfonyl]-1,3-dihydro-2*H*-benzimidazol-2-one.

A) 3-(Adamant-1-yl)-5-éthoxy-1,3-dihydro-1-(2-méthoxy-4-nitrobenzène-sulfonyl)-2*H*-benzimidazol-2-one.

A une suspension de 2,5 g de 3-(adamant-1-yl)-5-éthoxy-1,3-dihydro-2*H*-benzimidazol-2-one dans 10 ml de THF et 20 ml de DMF on ajoute par portions 0,35 g d'hydrure de sodium à 60 % dans l'huile et laisse 30 minutes sous agitation à TA. Puis on ajoute 2,2 g de chlorure de 2-méthoxy-4-nitrobenzènesulfonyle et on observe la formation d'un précipité jaune. On verse le mélange réactionnel sur de l'eau glacée, essore le précipité obtenu, le lave à l'eau, à l'EtOH 95 et à l'éther. On obtient 3,8 g du produit attendu, F = 231°C.

B) 3-(Adamant-1-yl)-1-(4-amino-2-méthoxybenzènesulfonyl)-5-éthoxy-1,3-dihydro-2*H*-benzimidazol-2-one.

On chauffe à reflux un mélange de 3,8 g du composé obtenu à l'étape précédente, 5 g de fer en poudre, 120 ml d'EtOH 95 et 2 ml d'eau et ajoute goutte à goutte 2 ml d'HCl concentré. On maintient 2 heures à reflux puis après refroidissement on ajoute 300 ml d'AcOEt, filtre sur Célite® et lave à l'AcOEt. Après décantation du filtrat, on sèche la phase organique sur $Na_2SO_4$ et concentre le solvant jusqu'à précipitation du produit. On essore le précipité et le lave à l'éther iso. On obtient 1,5 g du produit attendu, F = 248°C.

C) 3-(Adamant-1-yl)-5-éthoxy-1,3-dihydro-1-[2-méthoxy-4-(phénoxy-carboxamido)benzènesulfonyl]-2*H*-benzimidazol-2-one.

On refroidit à 10°C un mélange de 0,5 g du composé obtenu à l'étape précédente dans 20 ml de THF et ajoute une solution de 0,06 g de NaOH en pastilles dans 1 ml d'eau puis 0,6 ml de chloroformiate de phényle. On laisse 18 heures sous agitation à TA et évapore sous vide le solvant. On reprend le résidu à l'eau, essore le précipité formé et le lave à l'AcOEt. On obtient 0,56 g du produit attendu, F = 239°C.

D) 3-(Adamant-1-yl)-5-éthoxy-1-[4-(N',N'-diéthyluréido)-2-méthoxybenzènesulfonyl]-1,3-dihydro-2*H*-benzimidazol-2-one.

A une solution de 0,55 g du composé obtenu à l'étape précédente dans 20 ml de DCM on ajoute 2 ml de diéthylamine et laisse 3 heures sous agitation à TA. On lave le mélange réactionnel à l'eau, sèche sur $Na_2SO_4$ et évapore sous vide le solvant. On obtient 0,36 g du produit attendu après cristallisation dans l'AcOEt, F = 221°C.

EXEMPLE 41

Chlorhydrate de 3-(Adamant-1-yl)-5-éthoxy-1-[4-(3,3-diéthylguanidino)-2-méthoxybenzènesulfonyl]-1,3-dihydro-2*H*-benzimidazol-2-one, monohydrate.

On chauffe à reflux pendant 22 heures un mélange de 1,5 g du composé obtenu à l'EXEMPLE 40 étape B, 1,5 g de N,N-diéthylcyanamide, 20 ml d'AcOEt et 3 ml d'une solution à 20 % d'HCl dans l'EtOH. Puis on rajoute 2 g de N,N-diéthylcyanamide, 3 ml d'une solution à 20 % d'HCl dans l'EtOH et poursuit le reflux pendant 5 heures. Après refroidissement, on extrait le mélange réactionnel à l'AcOEt, lave la phase organique à l'eau, par une solution de $NaHCO_3$ 1N, à l'eau, sèche sur $Na_2SO_4$ et évapore sous vide le solvant. On chromatographie le résidu sur silice en éluant par le mélange DCM/AcOEt (94/6 ; v/v). On dissout le produit obtenu dans l'EtOH, ajoute 1 ml d'HCl concentré et évapore sous vide. On obtient 0,2 g du chlorhydrate du produit attendu après cristallisation dans le mélange EtOH/éther (50/50 ; v/v), F = 186°C.

EXEMPLE 42

5-Chloro-3-(2-chlorophényl)-1-[4-(N',N'-diéthyluréido)benzènesulfonyl]-1,3-dihydro-2*H*-benzimidazol-2-one.

A une solution de 0,5 g de 5-chloro-3-(2-chlorophényl)-1,3-dihydro-2*H*-benzimidazol-2-one dans 40 ml de DMF, on ajoute 0,075 g d'hydrure de sodium à 60 % dans l'huile et laisse 30 minutes sous agitation à TA. Puis on ajoute 0,6 g de chlorure de 4-(N',N'-diéthyluréido)benzènesulfonyle et laisse 4 heures sous agitation à TA. On verse le mélange réactionnel dans de l'eau glacée, extrait à l'AcOEt lave à l'eau, sèche sur $Na_2SO_4$ et évapore sous vide le solvant. On chromatographie le résidu sur silice en éluant par le mélange DCM/AcOEt (95/5 ; v/v). On obtient 0,45 g du produit attendu, F = 222°C.

EXEMPLE 43

5-Ethoxy-1,3-dihydro-1-[4-(N',N'-diéthyluréido)-2-méthoxybenzènesulfonyl]-3-(pyrid-2-yl)-2*H*-benzimidazol-2-one.

A)5-Ethoxy-1,3-dihydro-1-(2-méthoxy-4-nitrobenzènesulfonyl)-3-(pyrid-2-yl)-2*H*-benzimidazol-2-one.

On prépare ce composé selon le mode opératoire décrit à l'EXEMPLE 37 étape A à partir de 1 g de 5-éthoxy-1,3-dihydro-3-(pyrid-2-yl)-2*H*-benzimidazol-2-one et 1,2 g de chlorure de 2-méthoxy-4-nitrobenzènesulfonyle. On obtient 1,2 g du produit attendu après cristallisation dans l'éther iso, F = 175°C.

B) 1-(4-Amino-2-méthoxybenzènesulfonyl)-5-éthoxy-1,3-dihydro-3-(pyrid-2-yl)-2*H*-benzimidazol-2-one.

On hydrogène à TA et à pression atmosphérique un mélange de 1,2 g du composé obtenu à l'étape précédente, 0,2 g de palladium sur charbon à 5 % dans 200 ml d'AcOEt. On filtre le catalyseur et évapore sous vide le filtrat. On obtient 0,82 g du produit attendu après cristallisation dans l'éther iso, F = 208°C.

C) 5-Ethoxy-1,3-dihydro-1-[2-méthoxy-4-(phénoxycarboxamido)benzènesulfonyl]-3-(pyrid-2-yl)-2*H*-benzimidazol-2-one.

A une solution de 0,82 g du composé obtenu à l'étape précédente dans 10 ml de THF, on ajoute une solution de 0,1 g de NaOH en pastilles dans 20 ml d'eau. Puis on ajoute 1,5 ml de chloroformiate de phényle et laisse 2 heures sous agitation à TA. On extrait le mélange réactionnel à l'AcOEt, lave à l'eau, sèche sur $Na_2SO_4$ et évapore sous vide le solvant. On obtient 0,7 g du produit attendu que l'on utilise tel quel à l'étape suivante.

D) 5-Ethoxy-1,3-dihydro-1-[4-(N',N'-diéthyluréido)-2-méthoxybenzènesulfonyl]-3-(pyrid-2-yl)-2*H*-benzimidazol-2-one.

A une solution de 0,7 g du composé obtenu à l'étape précédente dans 20 ml de DCM, on ajoute 1 ml de diéthylamine et laisse 3 heures sous agitation à TA. On concentre sous vide le mélange réactionnel, extrait le résidu à l'AcOEt, lave par une solution d'HCl 1N, sèche sur $Na_2SO_4$ et évapore sous vide le solvant. On chromatographie le résidu sur silice en éluant par le mélange DCM/MeOH (99/1 ; v/v). On obtient 0,32 g du produit attendu après cristallisation dans le mélange AcOEt/éther iso, F = 171°C.

En procédant selon les modes opératoires décrits dans les EXEMPLES ci-dessus, à partir des 1,3-dihydro-2*H*-benzimidazol-2-ones décrites dans les Préparations ci-dessus, on prépare les composés selon l'invention rassemblés dans le TABLEAU 3 ci-après :

## TABLEAU 3

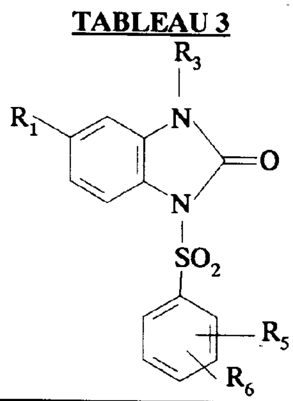

| EXEMPLES | R1 | R3 | R5 | R6 | F°C solvant de cristallisation |
|---|---|---|---|---|---|
| *44 (1) | -OEt | 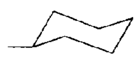 | 2-OMe | 4-NHCON Et iPr | 197 éther iso/AcOEt |
| *45 (2) | -OEt | 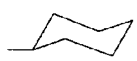 | 2-OMe | 4-NHCON 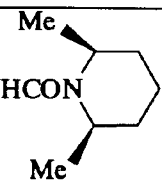 | 234 éther iso |
| *46 (3) | -OEt | Me Me<br>-C-CH$_2$-OMe | 2-OMe | 4-$NH_2$ | 213 |
| *47 (4) | -OEt | Me Me<br>-C-CH$_2$-OMe | 2-OMe | 4-NHCON Et Et | 211 |
| *48 (5) | -OEt | 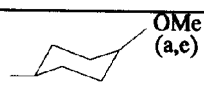 | 2-OMe | 4-$NO_2$ | 108-111 |
| *49 (6) | -OEt | 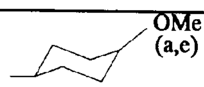 | 2-OMe | 4-NHCOO 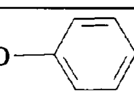 | 167-170 éther iso |

| | | | | | |
|---|---|---|---|---|---|
| *50 (7) | -OEt | 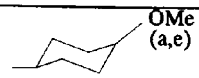 | 2-OMe | 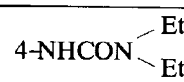 | 215-217 éther iso |
| *51 (5) | -OEt | 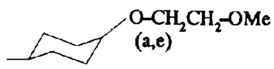 | 2-OMe | 4-$NO_2$ | 136-138 |
| *52 (6) | -OEt | 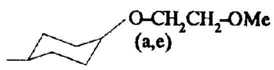 | 2-OMe | 4-NHCOO 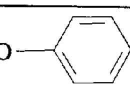 | 150-153 éther iso |
| *53 (7) | -OEt | 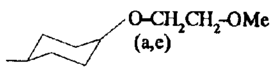 | 2-OMe | 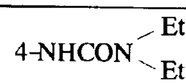 | 138-140 éther iso |
| *54 (5) | -OEt | 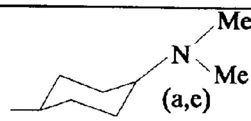 | 2-OMe | 4-$NO_2$ | 185 |
| *55 (8) | -OEt | 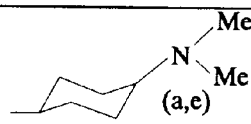 | 2-OMe | 4-$NH_2$ | 253 |
| *56 (9) | -OEt | 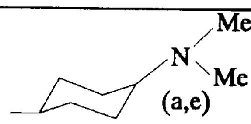 | 2-OMe | 4-NHCOO 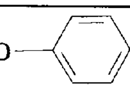 | 230-234 éther iso |
| *57 (10) | -OEt | 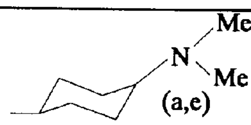 | 2-OMe | 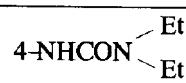 | 217-218 éther iso |
| *58 (5) | -OEt | 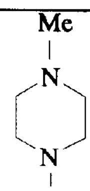 | 2-OMe | 4-$NO_2$ | 200 éther iso |
| *59 (11) | -OEt | 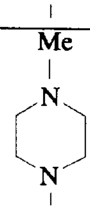 | 2-OMe | 4-NHCOO 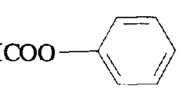 | 136 |

| | | | | | |
|---|---|---|---|---|---|
| *60 (12) | -OEt | 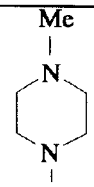 | 2-OMe | 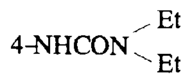 | 151 éther iso |
| *61 (5) | -OEt | 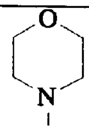 | 2-OMe | 4-$NO_2$ | 193 EtOH |
| *62 (13) | -OEt | 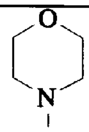 | 2-OMe | 4-$NH_2$ | 209 |
| *63 (14) | -OEt | 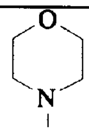 | 2-OMe | 4-NHCOO 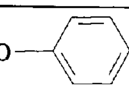 | 215 |
| *64 (7) | -OEt | 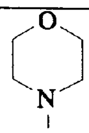 | 2-OMe | 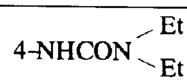 | 242 |
| *65 (3) | -OEt | 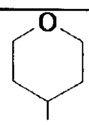 | 2-OMe | 4-$NH_2$ | 192 |
| *66 (14) | -OEt | 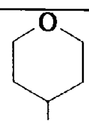 | 2-OMe | 4-NHCOO 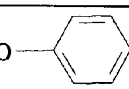 | 215 |
| *67 (7) | -OEt | 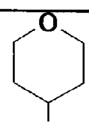 | 2-OMe | 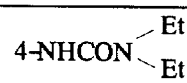 | 201 |
| *68 (5) | -OEt | 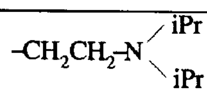 | 2-OMe | 4-$NO_2$ | 172 éther iso |
| *69 (15) | -OEt | 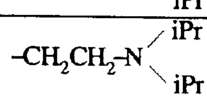 | 2-OMe | 4-$NH_2$ | 177 |
| *70 (14) | -OEt | 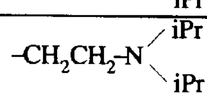 | 2-OMe | 4-NHCOO 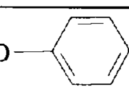 | 135-137 éther iso |

| | | | | | |
|---|---|---|---|---|---|
| *71 (7) | -OEt | 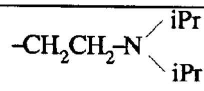 | 2-OMe | 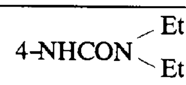 | 168 éther iso |
| *72 (5) | -OEt | 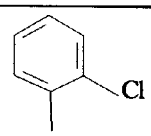 | 2-OMe | 4-$NO_2$ | 156 éther iso |
| *73 (16) | -OEt | 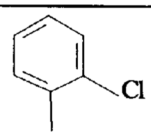 | 2-OMe | 4-$NH_2$ | 214 |
| *74 (14) | -OEt | 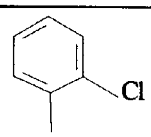 | 2-OMe | 4-NHCOO 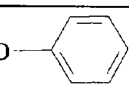 | 138 éther iso |
| *75 (12) | -OEt | 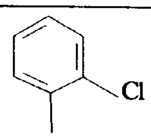 | 2-OMe | 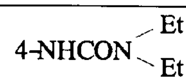 | 235 éther iso |
| *76 (5) | -OEt | 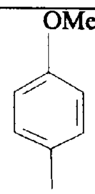 | 2-OMe | 4-$NO_2$ | 165 éther iso |
| *77 (17) | -OEt | 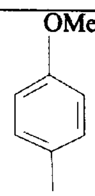 | 2-OMe | 4-NHCOO 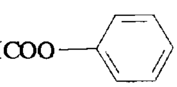 | 186 éther iso |
| *78 (7) | -OEt | 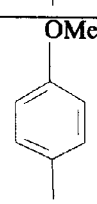 | 2-OMe | 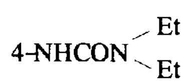 | 157 éther iso |
| *79 (5) | -OEt | 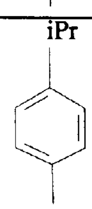 | 2-OMe | 4-$NO_2$ | 134-138 éther iso |

| | | | | | |
|---|---|---|---|---|---|
| *80 (18) | -OEt | 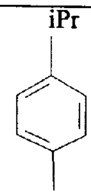 | 2-OMe | 4-$NH_2$ | 108-115 |
| *81 (14) | -OEt | 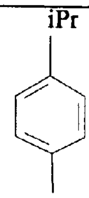 | 2-OMe | 4-NHCOO 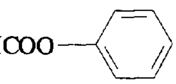 | 202-205 éther iso |
| *82 (19) | -OEt | 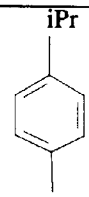 | 2-OMe | 4-NHCON Et Et | 203 éther iso |
| *83 (5) | -OEt | 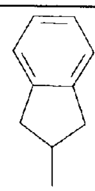 | 2-OMe | 4-$NO_2$ | 187 |
| *84 (20) | -OEt | 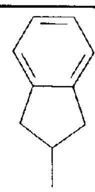 | 2-OMe | 4-NHCOO 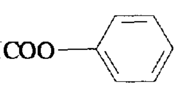 | 198 éther iso/EtOH |
| *85 (7) | -OEt | 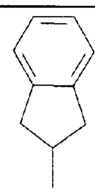 | 2-OMe | 4-NHCON Et Et | 207 |
| *86 (21) | $-OCH_2CH_2-$ OMe | 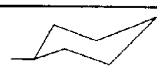 | 2-OMe | 4-$NO_2$ | 168 |
| *87 (18) | $-OCH_2CH_2-$ OMe | 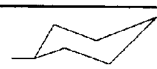 | 2-OMe | 4-$NH_2$ | 193 |
| *88 (4) | $-OCH_2CH_2-$ OMe | 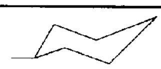 | 2-OMe | 4-NHCON Et Et | 205 éther iso |

| | | | | | |
|---|---|---|---|---|---|
| *89 (5) | 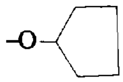 | 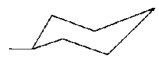 | 2-OMe | 4-$NO_2$ | 113 |
| *90 (16) | 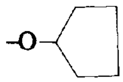 | 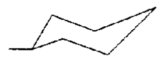 | 2-OMe | 4-$NH_2$ | 130 |
| *91 (4) | 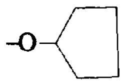 | 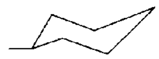 | 2-OMe | 4-NHCON Et Et | 222 éther iso |
| *92 (5) | -OEt | 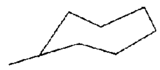 | 2-OMe | 4-$NO_2$ | 154 |
| *93 (22) | -OEt | 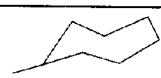 | 2-OMe | 4-$NH_2$ | 224 éther iso |
| *94 (4) | -OEt | 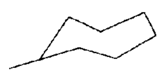 | 2-OMe | 4-NHCON Et Et | 208 éther iso |
| *95 (21) | -OEt | 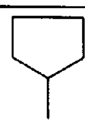 | 2-OMe | 4-$NO_2$ | 139 |
| *96 (23) | -OEt | 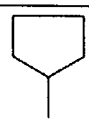 | 2-OMe | 4-$NH_2$ | 198 |
| *97 (14) | -OEt | 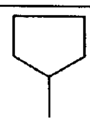 | 2-OMe | 4-NHCOO 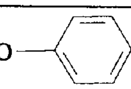 | 195 éther iso |
| *98 (12) | -OEt | 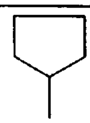 | 2-OMe | 4-NHCON Et Et | 204 AcOEt/ éther iso |

(1) On prépare ce composé selon le mode opératoire décrit à l'EXEMPLE 35 en utilisant la N-éthylisopropylamine.
(2) On prépare ce composé selon le mode opératoire décrit à l'EXEMPLE 35 en utilisant la cis-2,6-diméthylpipéridine.
(3) On prépare ce composé selon les modes opératoires décrits à l'EXEMPLE 37 étape A puis étape B.
(4) On prépare ce composé selon les modes opératoires décrits à l'EXEMPLE 37 étape C puis étape D.
(5) On prépare ce composé selon le mode opératoire décrit à l'EXEMPLE 37 étape A.
(6) On prépare ce composé selon les modes opératoires décrits à l'EXEMPLE 38 étape B (hydrogénation sous 70 bars de pression) puis à l'EXEMPLE 37 étape C.
(7) On prépare ce composé selon le mode opératoire décrit à l'EXEMPLE 37 étape D.
(8) On hydrogène à TA et pression atmosphérique un mélange de 2,9 g du composé de l'EXEMPLE 54 dans 30 ml de THF et 30 ml de MeOH et 1 g de nickel de Raney®. On filtre le catalyseur et évapore sous vide le filtrat.
(9) On dissout à 10°C 1,3 g du composé de l'EXEMPLE 55 dans 5 ml de chloroformiate de phényle et laisse 1 heure sous agitation à 10-15°C. On concentre le mélange réactionnel à 40°C sous 0,1 mm de Hg, reprend

le résidu dans l'eau glacée, neutralise par ajout de NaOH concentrée, extrait à l'AcOEt, sèche sur $Na_2SO_4$ et évapore sous vide. On obtient 0,7 g du produit attendu.

(10) On chauffe à 40°C jusqu'à dissolution, un mélange de 0,68 g du composé de l'EXEMPLE 56, 2 g de diéthylamine, 20 ml de THF et 20 ml d'EtOH. On laisse 20 heures sous agitation à TA. On essore le précipité formé, et le lave à éther iso. On obtient 0,38 g du produit attendu.

(11) On prépare ce composé selon les modes opératoires décrits à l'EXEMPLE 38 étape B puis à l'EXEMPLE 37 étape C.

(12) On prépare ce composé selon le mode opératoire décrit à l'EXEMPLE 38 étape D.

(13) On prépare ce composé selon le mode opératoire décrit à l'EXEMPLE 37 étape B.

(14) On prépare ce composé selon le mode opératoire décrit à l'EXEMPLE 37 étape C.

(15) On prépare ce composé selon le mode opératoire décrit à l'EXEMPLE 38 étape B (hydrogénation sous 50 bars de pression).

(16) On prépare ce composé selon le mode opératoire décrit à l'EXEMPLE 38 étape B (hydrogénation à pression atmosphérique).

(17) On prépare ce composé selon les modes opératoires décrits à la référence (8) puis à l'EXEMPLE 37 étape C.

(18) On prépare ce composé selon le mode opératoire décrit à la référence (8) à partir du dérivé nitré correspondant.

(19) On laisse 2 heures sous agitation à TA un mélange de 1,1 g du composé de l'EXEMPLE 81, 5 ml de diéthylamine dans 20 ml de THF. On évapore sous vide, extrait le résidu à l'AcOEt, lave par NaOH 1N, à l'eau, par HCl 1N, à l'eau, sèche sur $Na_2SO_4$ et évapore sous vide. On chromatographie le résidu sur silice en éluant par le mélange DCM/AcOEt (80/20 ; v/v).

(20) On prépare ce composé selon les modes opératoires décrits à l'EXEMPLE 37 étape B puis étape C.

(21) On prépare ce composé selon le mode opératoire décrit à l'EXEMPLE 40 étape A.

(22) On hydrogène à TA et sous 2 bars de pression un mélange de 0,8 g du composé de l'EXEMPLE 92 dans 100 ml d'EtOH 96, 20 ml d'AcOEt et 50 ml de MeOH, en présence de 0,5 g de nickel de Raney®. On filtre le catalyseur et évapore sous vide le filtrat.

(23) On hydrogène à TA et sous 2 bars de pression un mélange de 1,4 g du composé de l'EXEMPLE 95 dans 250 ml d'EtOH 95 et 100 ml d'AcOEt, en présence de nickel de Raney®. On filtre le catalyseur et évapore sous vide le filtrat. On obtient 1,1 g du produit attendu.

EXEMPLE 99

3-Cyclohexyl-5-éthoxy-1,3-dihydro-1-[2-méthoxy-4-[N-(1,1-diméthylpropyl) carbamoyl]benzènesulfonyl]-2*H*-benzimidazol-2-one.

On laisse 2 heures sous agitation à TA un mélange de 1,09 g du composé obtenu à l'EXEMPLE 13, 1 ml de 1,1-diméthylpropylamine, 1,2 g de BOP, 1 ml de DIPEA et 20 ml de DCM. On évapore sous vide le mélange réactionnel, extrait le résidu à l'AcOEt, lave par une solution d'HCl 1N, par une solution de NaOH 1N, à l'eau, sèche sur $Na_2SO_4$ et évapore sous vide le solvant. On chromatographie le résidu sur silice en éluant par le mélange DCM/AcOEt (99/1; v/v). On obtient 0,88 g du produit attendu sous forme de cristaux blancs, F = 219°C.

EXEMPLE 100

3-Cyclohexyl-5-éthoxy-1,3-dihydro-1-[2-méthoxy-4-[N-(1,1,3,3-tétraméthylbutyl)carbamoyl]benzènesulfonyl]-2*H*-benzimidazol-2-one.

On laisse 2 heures sous agitation à TA un mélange de 0,620 g du composé obtenu à l'EXEMPLE 13, 1 ml de 1,1,3,3-tétraméthylbutylamine, 0,7 g de BOP, 1 ml de DIPEA et 20 ml de DCM. Puis on lave le mélange réactionnel à l'eau, par une solution d'HCl 1N, par une solution de NaOH 1N, sèche sur $Na_2SO_4$ et évapore sous vide le solvant. On chromatographie le résidu sur silice en éluant au DCM. On obtient 0,28 g du produit attendu sous forme de cristaux blancs, F = 180-C.

EXEMPLE 101

3-Cyclohexyl-5-éthoxy-1,3-dihydro-1-[4-[N-(2-hydroxy-1,1-diméthyléthyl) carbamoyl]-2-méthoxybenzènesulfonyl]-2*H*-benzimidazol-2-one.

On prépare ce composé selon le mode opératoire décrit à l'EXEMPLE 100 à partir de 0,5 g du composé obtenu à l'EXEMPLE 13, 0,5 g de 2-amino-2-méthylpropan-1-ol, 0,6 g de BOP, 1 ml de DIPEA et 20 ml de DCM. On chromatographie sur silice en éluant par le mélange DCM/MeOH (99/1; v/v). On obtient 0,12 g du produit attendu sous forme de cristaux blancs, F = 218°C.

EXEMPLE 102

3-Cyclohexyl-5-éthoxy-1,3-dihydro-1-[2-méthoxy-4-[N-(2-méthylphényl) carbamoyl]benzènesulfonyl]-2*H*-benzimidazol-2-one.

On chauffe à reflux pendant 6 heures un mélange de 0,44 g du composé obtenu à l'EXEMPLE 13, 0,17 g de chlorure de thionyle dans 1 ml de DMF et 20 ml de DCM. On évapore sous vide le mélange réactionnel, ajoute au chlorure d'acide ainsi obtenu 0,6 g d'o-toluidine dans 20 ml de DCM et laisse 1 heure sous agitation à TA. On évapore sous vide le solvant, extrait le résidu à l'AcOEt, lave deux fois par une solution d'HCl 12N, par une solution de NaOH 3N, à l'eau, sèche sur $Na_2SO_4$ et évapore sous vide le solvant. On chromatographie le résidu sur silice en éluant par le mélange DCM/AcOEt (98/2 ; v/v). On obtient 0,14 g du produit attendu sous forme de cristaux blancs, F = 179°C.

EXEMPLE 103

3-Cyclohexyl-5-éthoxy-1,3-dihydro-1-[2-méthoxy-4-[N-(thiazol-2-yl)carbamoyl] benzènesulfonyl]-2*H*-benzimidazol-2-one.

On prépare ce composé selon le mode opératoire décrit à l'EXEMPLE 100 à partir de 1 g du composé obtenu à l'EXEMPLE 13, 0,3 g de 2-aminothiazole, 1,2 g de BOP, 1 ml de DIPEA et 20 ml de DCM. On chromatographie sur silice en éluant par le mélange DCM/MeOH (99,5/0,5 ; v/v). On obtient 0,09 g du produit attendu après cristallisation dans l'EtOH, F = 213°C.

EXEMPLE 104

3-Cyclohexyl-5-éthoxy-1,3-dihydro-1-[4-[N-(indan-2-yl)carbamoyl]-2-méthoxybenzènesulfonyl]-2*H*-benzimidazol-2-one.

On prépare ce composé selon le mode opératoire décrit à l'EXEMPLE 100 à partir de 0,8 g du composé obtenu à l'EXEMPLE 13, 0,35 g de chlorhydrate de 2-aminoindane, 0,8 g de BOP, 2 ml de DIPEA et 20 ml de DCM. On chromatographie sur silice en éluant par le mélange DCM/AcOEt (96/4 ; v/v). On obtient 0,7 g du produit attendu sous forme de cristaux blancs, F = 160°C.

EXEMPLE 105

1-[4-[N-(Adamant-1-yl)carbamoyl]-2-méthoxybenzènesulfonyl]-3-cyclohexyl-5-éthoxy-1,3-dihydro-2*H*-benzimidazol-2-one.

On prépare ce composé selon le mode opératoire décrit à l'EXEMPLE 100 à partir de 0,5 g du composé obtenu à l'EXEMPLE 13, 0,3 g de chlorhydrate de 1-adamantanamine, 0,5 g de BOP, 2 ml de DIPEA et 25 ml de DCM. On chromatographie sur silice en éluant par le mélange DCM/MeOH (99/1; v/v). On obtient 0,27 g du produit attendu après cristallisation dans le mélange EtOH/éther iso (80/20; v/v), F = 228°C.

EXEMPLE 106

3-Cyclohexyl-5-cyclopentyloxy-1,3-dihydro-1-[2-méthoxy-4-[N-(1,1-diméthylpropyl)carbamoyl]benzènesulfonyl]-2*H*-benzimidazol-2-one.

A) 4-[3-Cyclohexyl-5-cyclopentyloxy-2,3-dihydro-2-oxo-1*H*-benzimidazol-1-yl] sulfonyl-3-méthoxybenzoate de méthyle.

A un mélange de 0,7 g de 3-cyclohexyl-5-cyclopentyloxy-1,3-dihydro-2*H*-benzimidazol-2-one, 50 ml de DMF et 50 ml de THF, on ajoute par portions 0,095 g d'hydrure de sodium à 60 % dans l'huile et laisse 30 minutes sous agitation à TA. On ajoute ensuite 0,6 g de chlorure de 4-méthoxycarbonyl-2-méthoxybenzènesulfonyle et laisse 18 heures sous agitation à TA. On verse le mélange réactionnel sur un mélange d'eau et de glace, essore le précipité formé, le lave à l'eau puis à l'heptane. On obtient 1 g du produit attendu que l'on utilise tel quel à l'étape suivante.

B) Acide 4-[3-cyclohexyl-5-cyclopentyloxy-2,3-dihydro-2-oxo-1*H*-benzimidazol-1-yl] sulfonyl-3-méthoxybenzoïque.

A une solution de 1 g du composé obtenu à l'étape précédente dans 40 ml de THF, on ajoute une solution de 0,2 g de NaOH en pastilles dans 20 ml d'eau et chauffe 2 heures à reflux. On concentre sous vide, dissout le résidu dans l'eau, lave à l'AcOEt, acidifie la phase aqueuse à pH = 1 par ajout d'HCl concentré, extrait à l'AcOEt, sèche sur $Na_2SO_4$ et évapore sous vide le solvant. On obtient 0,56 g du produit attendu, F = 245°C.

C) 3-Cyclohexyl-5-cyclopentyloxy-1,3-dihydro-1-[2-méthoxy-4-[N-(1,1-diméthylpropyl)carbamoyl]benzènesulfonyl]-2*H*-benzimidazol-2-one.

On prépare ce composé selon le mode opératoire décrit à l'EXEMPLE 100 à partir de 0,55 g du composé obtenu à l'étape précédente, 1 ml de 1,1-diméthylpropylamine, 0,6 g de BOP, 1,5 ml de DIPEA et 20 ml de DCM. On chromatographie sur silice en éluant par le mélange DCM/AcOEt (90/10 ; v/v). On obtient 0,32 g du produit attendu après cristallisation dans l'éther iso, F = 199°C.

EXEMPLE 107

3-Cyclohexyl-1,3-dihydro-5-(2-méthoxyéthoxy)-1-[2-méthoxy-4-[N-(1,1-diméthylpropyl)carbamoyl]benzènesulfonyl]-2*H*-benzimidazol-2-one.

A) 4-[3-Cyclohexyl-2,3-dihydro-5-(2-méthoxyéthoxy)-2-oxo-1*H*-benzimidazol-1-yl]sulfonyl-3-méthoxybenzoate de méthyle.

A un mélange de 0,7 g de 3-cyclohexyl-1,3-dihydro-5-(2-méthoxyéthoxy)-2*H*-benzimidazol-2-one dans 50 ml de DMF, on ajoute par portions 0,095 g d'hydrure de sodium à 60 % dans l'huile et laisse 30 minutes sous agitation à TA. On ajoute ensuite 0,6 g de chlorure de 4-méthoxycarbonyl-2-méthoxybenzènesulfonyle et laisse deux heures sous agitation à TA. On verse le mélange réactionnel sur un mélange d'eau et de glace, extrait à l'AcOEt, lave à l'eau, sèche sur $Na_2SO_4$ et évapore sous vide le solvant. On obtient 0,8 g du produit attendu que l'on utilise tel quel à l'étape suivante.

B) Acide 4-[3-cyclohexyl-2,3-dihydro-5-(2-méthoxyéthoxy)-2-oxo-1*H*-benzimidazol-1-yl]sulfonyl-3-méthoxybenzoïque.

On prépare ce composé selon le mode opératoire décrit à l'EXEMPLE 106 étape B à partir de 0,8 g du composé obtenu à l'étape précédente. On obtient 0,6 g du produit attendu, F = 105°C.

C) 3-Cyclohexyl-1,3-dihydro-5-(2-méthoxyéthoxy)-1-[2-méthoxy-4-[N-(1,1-diméthylpropyl)carbamoyl]benzènesulfonyl]-2*H*-benzimidazol-2-one.

On prépare ce composé selon le mode opératoire décrit à l'EXEMPLE 99 à partir de 0,55 g du composé obtenu à l'étape précédente, 1 ml de 1,1-diméthylpropylamine, 0,6 g de BOP, 1,5 ml de DIPEA et 20 ml de DCM. On chromatographie sur silice en éluant par le mélange DCM/AcOEt (95/5 ; v/v). On obtient 0,4 g du produit attendu après cristallisation à chaud dans l'éther iso, F = 163°C.

EXEMPLE 108

1-[4-(N-*tert*-butylcarbamoyl)-2-méthoxybenzènesulfonyl]-5-éthoxy-1,3-dihydro-3-(2-méthoxy-1,1-diméthyléthyl)-2*H*-benzimidazol-2-one.

A) 4-[5-Ethoxy-2,3-dihydro-3-(2-méthoxy-1,1-diméthyléthyl)-2-oxo-1*H*-benzimidazol-1-yl]sulfonyl-3-méthoxybenzoate de méthyle.

A un mélange de 1 g de 5-éthoxy-1,3-dihydro-3-(2-méthoxy-1,1-diméthyléthyl)-2*H*-benzimidazol-2-one dans 30 ml de THF, on ajoute par portions 0,12 g d'hydrure de sodium à 60 % dans l'huile et laisse 30 minutes sous agitation à TA. On ajoute ensuite 1,2 g de chlorure de 4-méthoxycarbonyl-2-méthoxybenzènesulfonyle et laisse 2 heures sous agitation à TA. On concentre sous vide, extrait le résidu à l'AcOEt, lave à l'eau, sèche sur $Na_2SO_4$ et évapore sous vide le solvant. On chromatographie le résidu sur silice en éluant par le mélange DCM/AcOEt (98/2 ; v/v). On obtient 1,7 g du produit attendu que l'on utilise tel quel à l'étape suivante.

B) Acide 4-[5-éthoxy-2,3-dihydro-3-(2-méthoxy-1,1-diméthyléthyl)-2-oxo-1*H*-benzimidazol-1-yl]sulfonyl-3-méthoxybenzoïque.

A une solution de 1,7 g du composé obtenu à l'étape précédente dans 50 ml de THF, on ajoute une solution de 0,1 g de NaOH en pastilles dans 30 ml d'eau et laisse 5 heures sous agitation à TA. On concentre sous vide, dissout le résidu dans 100 ml d'eau, lave à l'AcOEt, acidifie la phase aqueuse à pH = 1 par ajout d'HCl concentré, essore le précipité formé, le lave à l'EtOH puis à l'éther iso. On obtient 1,6 g du produit attendu, F = 250°C.

C) 1-[4-(N-*tert*-butylcarbamoyl)-2-méthoxybenzènesulfonyl]-5-éthoxy-1,3-dihydro-3-(2-méthoxy-1,1-diméthyléthyl)-2*H*-benzimidazol-2-one.

On prépare ce composé selon le mode opératoire décrit à l'EXEMPLE 99 à partir de 0,8 g du composé obtenu à l'étape précédente, 2 ml de tert-butylamine, 0,9 g de BOP, 2 ml de DIPEA et 30 ml de DCM. On chromatographie sur silice en éluant par le mélange DCM/AcOEt (96/4 ; v/v). On obtient 0,48 g du produit attendu sous forme de cristaux blancs, F = 181°C.

EXEMPLE 109

5-Ethoxy-1,3-dihydro-3-(tétrahydropyran-4-yl)-1-[2-méthoxy-4-[N-(1,1-diméthylpropyl)carbamoyl]benzènesulfonyl]-2*H*-benzimidazol-2-one.

A) 4-[5-Ethoxy-2,3-dihydro-3-(tétrahydropyran-4-yl)-2-oxo-1*H*-benzimidazol-1-yl]sulfonyl-3-méthoxybenzoate de méthyle.

A un mélange de 1 g de 5-éthoxy-1,3-dihydro-3-(tétrahydropyran-4-yl)-2*H*-benzimidazol-2-one, 30 ml de THF et 20 ml de DMF, on ajoute par portions 0,18 g d'hydrure de sodium à 60 % dans l'huile et laisse 30 minutes sous agitation à TA. On ajoute ensuite 1,2 g de chlorure de 4-méthoxycarbonyl-2-méthoxybenzènesulfonyle et laisse 3 heures sous agitation à TA. On verse le mélange réactionnel dans 200 ml d'eau, essore le précipité formé et le lave à l'éther iso. On obtient 1,13 g du produit attendu après séchage, F = 165°C.

B) Acide 4-[5-éthoxy-2,3-dihydro-3-(tétrahydropyran-4-yl)-2-oxo-1*H*-benzimidazol-1-yl]sulfonyl-3-méthoxybenzoïque.

A une solution de 1,13 g du composé obtenu à l'étape précédente dans 20 ml de THF, on ajoute une solution de 0,5 g de NaOH en pastilles dans 30 ml d'eau et laisse 16 heures sous agitation à TA. On lave le mélange réactionnel à l'AcOEt, acidifie la phase aqueuse à pH = 1 par ajout d'HCl concentré, extrait à l'AcOEt, sèche sur $Na_2SO_4$ et évapore sous vide le solvant. On obtient 0,8 g du produit attendu après cristallisation dans l'éther iso, F = 255°C.

C) 5-Ethoxy-1,3-dihydro-3-(tétrahydropyran-4-yl)-1-[2-méthoxy-4-[N-(1,1-diméthylpropyl)carbamoyl]benzènesulfonyl]-2*H*-benzimidazol-2-one.

On prépare ce composé selon le mode opératoire décrit à l'EXEMPLE 99 à partir de 0,39 g du composé obtenu à l'étape précédente, 1 ml de 1,1-diméthylpropylamine, 0,45 g de BOP, 1 ml de DIPEA et 30 ml de DCM. On chromatographie sur silice en éluant par le mélange DCM/MeOH (99/1 ; v/v). On obtient 0,28 g du produit attendu sous forme de cristaux blancs, F = 183°C.

EXEMPLE 110

3-Cycloheptyl-5-éthoxy-1,3-dihydro-1-[2-méthoxy-4-[N-(1,1-diméthylpropyl) carbamoyl]benzènesulfonyl]-2*H*-benzimidazol-2-one.

A) 4-[3-Cycloheptyl-5-éthoxy-2,3-dihydro-2-oxo-1*H*-benzimidazol-1-yl]sulfonyl-3-méthoxybenzoate de méthyle.

A un mélange de 0,7 g de 3-cycloheptyl-5-éthoxy-1,3-dihydro-2*H*-benzimidazol-2-one, 50 ml de DMF, on ajoute 0,095 g d'hydrure de sodium à 60 % dans l'huile et laisse 30 minutes sous agitation à TA. Puis on ajoute 0,7 g de chlorure de 4-méthoxycarbonyl-2-méthoxybenzènesulfonyle et laisse une nuit sous agitation à TA. On verse le mélange réactionnel sur de l'eau glacée, essore le précipité formé, le lave à l'eau. On obtient 0,9 g du produit attendu que l'on utilise tel quel à l'étape suivante.

B) Acide 4-[3-cycloheptyl-5-éthoxy-2,3-dihydro-2-oxo-1*H*-benzimidazol-1-yl] sulfonyl-3-méthoxybenzoïque.

A une solution de 0,9 g du composé obtenu à l'étape précédente dans 40 ml de THF, on ajoute une solution de 0,3 g de NaOH en pastilles dans 20 ml d'eau et chauffe 2 heures à reflux. Après refroidissement, on acidifie à pH = 1 par ajout d'HCl concentré, essore le précipité formé et le lave à l'eau. On dissout le précipité dans l'AcOEt, sèche sur $Na_2SO_4$ et évapore sous vide le solvant. On obtient 0,75 g du produit attendu que l'on utilise tel quel à l'étape suivante.

C) 3-Cycloheptyl-5-éthoxy-1,3-dihydro-1-[2-méthoxy-4-[N-(1,1-diméthylpropyl) carbamoyl]benzènesulfonyl]-2*H*-benzimidazol-2-one.

On prépare ce composé selon le mode opératoire décrit à l'EXEMPLE 99 à partir de 0,75 g du composé obtenu à l'étape précédente, 1,5 ml de 1,1-diméthylpropylamine, 0,85 g de BOP, 2 ml de DIPEA et 25 ml de DCM. On chromatographie sur silice en éluant par le mélange DCM/AcOEt (95/5 ; v/v). On obtient 0,26 g du produit attendu après cristallisation dans l'éther iso, F = 192°C.

EXEMPLE 111

5-Ethoxy-1,3-dihydro-1-[2-méthoxy-4-(N,N-diméthylcarbamoyl)benzènesulfonyl]-3-(pyrid-2-yl)-2*H*-benzimidazol-2-one.

A) 4-[5-Ethoxy-2,3-dihydro-2-oxo-3-(pyrid-2-yl)-1*H*-benzimidazol-1-yl] sulfonyl-3-méthoxybenzoate de méthyle.

A un mélange de 1 g de 5-éthoxy-1,3-dihydro-3-(pyrid-2-yl)-2*H*-benzimidazol-2-one, 25 ml de DMF et 25 ml de THF, on ajoute 0,2 g d'hydrure de sodium à 60 % dans l'huile et laisse 30 minutes sous agitation à TA. Puis on ajoute 1 g de chlorure de 4-méthoxycarbonyl-2-méthoxybenzènesulfonyle et laisse 3 heures sous agitation à TA. On concentre sous vide le mélange réactionnel, reprend le résidu à l'eau, extrait à l'AcOEt, lave à l'eau, sèche sur $Na_2SO_4$ et évapore sous vide le solvant. On obtient 1 g du produit attendu que l'on utilise tel quel à l'étape suivante.

B) Acide 4-[5-éthoxy-2,3-dihydro-2-oxo-3-(pyrid-2-yl)-1*H*-benzimidazol-1-yl] sulfonyl-3-méthoxybenzoïque.

A une solution de 1 g du composé obtenu à l'étape précédente dans 20 ml de THF, on ajoute une solution de 0,3 g de NaOH en pastilles dans 20 ml d'eau et laisse 3 heures sous agitation à TA. On extrait le mélange réactionnel à l'AcOEt, acidifie la phase aqueuse à pH = 1 par ajout d'HCl concentré, extrait à l'AcOEt, sèche sur $Na_2SO_4$ et évapore sous vide le solvant. On obtient 0,72 g du produit attendu après cristallisation dans l'éther iso, F = 241°C.

C) 5-Ethoxy-1,3-dihydro-1-[2-méthoxy-4-(N,N-diméthylcarbamoyl)benzènesulfonyl]-3-(pyrid-2-yl)-2*H*-benzimidazol-2-one.

On laisse 3 heures sous agitation à TA un mélange de 0,72 g du composé obtenu à l'étape précédente, 0,17 g de chlorhydrate de diméthylamine, 2 ml de DIPEA, 0,9 g de BOP et 20 ml de DCM. On concentre sous vide la mélange réactionnel, extrait le résidu à l'AcOEt, lave à l'eau, par une solution d'HCl 1N, par une solution de NaOH 1N, sèche sur $Na_2SO_4$ et évapore sous vide le solvant. On chromatographie le résidu sur silice en éluant par le mélange DCM/AcOEt (85/15 ; v/v). On obtient 0,2 g du produit attendu, F = 201°C.

EXEMPLE 112

5-Ethoxy-1,3-dihydro-1-[2-méthoxy-4-[N-(1,1-diméthylpropyl)carbamoyl] benzènesulfonyl]-3-(pyrid-2-yl)-2*H*-benzimidazol-2-one.

On laisse 3 heures sous agitation à TA un mélange de 0,3 g du composé obtenu à l'EXEMPLE 111 étape B, 0,5 ml de 1,1-diméthylpropylamine, 0,5 ml de DIPEA, 0,35 g de BOP et 20 ml de DCM. On concentre sous vide le mélange réactionnel, extrait le résidu à l'AcOEt, lave par une solution d'HCl 1N, par une solution de NaOH 1N, sèche sur $Na_2SO_4$ et évapore sous vide le solvant. On chromatographie le résidu sur silice en éluant par le mélange DCM/AcOEt (90/10 ; v/v). On obtient 0,15 g du produit attendu après cristallisation dans l'éther iso.

Spectre de RMN à 200 MHz dans DMSO.

0,7 ppm : t: 3H

1,25 ppm : m : 9H

1,7 ppm : q : 2H
3,55 ppm : s : 3H
3,95 ppm : q : 2H
6,7 à 8,7 ppm : m : 11H

EXEMPLE 113

3-Cyclopentyl-5-éthoxy-1,3-dihydro-1-[2-méthoxy-4-[N-(1,1-diméthylpropyl) carbamoyl]benzènesulfonyl]-2*H*-benzimidazol-2-one.

A) 4-[3-Cyclopentyl-5-éthoxy-2,3-dihydro-2-oxo-1*H*-benzimidazol-1-yl]sulfonyl-3-méthoxybenzoate de méthyle.

On prépare ce composé selon le mode opératoire décrit à l'EXEMPLE 107 étape A à partir de 1 g de 3-cyclopentyl-5-éthoxy-1,3-dihydro-2*H*-benzimidazol-2-one et 1,2 g de chlorure de 4-méthoxycarbonyl-2-méthoxybenzènesulfonyle. On chromatographie sur silice en éluant par le mélange DCM/AcOEt (95/5 ; v/v). On obtient 1,4 g du produit attendu, F = 151°C.

B) Acide 4-[3-cyclopentyl-5-éthoxy-2,3-dihydro-2-oxo-1*H*-benzimidazol-1-yl]sulfonyl-3-méthoxybenzoïque.

On prépare ce composé selon le mode opératoire décrit à l'EXEMPLE 106 étape B à partir de 1,4 g du composé obtenu à l'étape précédente. On obtient 0,9 g du produit attendu que l'on utilise tel quel à l'étape suivante.

C) 3-Cyclopentyl-5-éthoxy-1,3-dihydro-1-[2-méthoxy-4-[N-(1,1-diméthylpropyl) carbamoyl]benzènesulfonyl]-2*H*-benzimidazol-2-one.

On laisse 3 heures sous agitation à TA un mélange de 0,9 g du composé obtenu à l'étape précédente, 2 ml de 1,1-diméthylpropylamine, 1 g de BOP, 3 ml de DIPEA et 30 ml de DCM. On concentre sous vide le mélange réactionnel et chromatographie le résidu sur silice en éluant par le mélange DCM/AcOEt (90/10 ; v/v). On obtient 0,32 g du produit attendu après cristallisation dans l'éther iso, F = 210°C.

EXEMPLE 114

5-Chloro-3-(3-chlorophényl)-1,3-dihydro-1-(2,4-diméthoxybenzène-sulfonyl)-2*H*-benzimidazol-2-one.

On refroidit à -40°C une solution de 2 g de 5-chloro-3-(3-chlorophényl)-1,3-dihydro-2*H*-benzimidazol-2-one dans 170 ml de THF et ajoute 0,97 g de *tert*-butylate de potassium. On laisse 30 minutes sous agitation à -10°C, puis refroidit à -50°C et ajoute une solution de 1,7 g de chlorure de 2,4-diméthoxybenzènesulfonyle dans 70 ml de THF. On laisse 2 heures sous agitation en laissant remonter la température à TA puis concentre sous vide le mélange réactionnel. On reprend le résidu à l'eau, extrait au DCM, sèche sur $Na_2SO_4$ et évapore sous vide le solvant. On obtient 2,3 g du produit attendu après cristallisation dans un mélange cyclohexane/DCM (90/10 ; v/v), F = 173°C.

EXEMPLE 115

3-cyclohexyl-5-éthoxy-1,3-dihydro-1-[2-méthoxy-4-[N-(2-diméthylamino-1,1-diméthyléthyl)carbamoyl]benzènesulfonyl]-2*H*-benzimidazol-2-one

On laisse 16 heures sous agitation à TA un mélange de 1 g du composé obtenu à l'Exemple 13, 1 g de 2-diméthylamino-1,1-diméthyléthylamine (synthétisée selon J. Am. Chem. Soc., 1946, 68, 10-12), 1 g de BOP, 1 ml de DIPEA et 20 ml de DCM. On évapore sous vide le mélange réactionnel, extrait le résidu à l'AcOEt, lave par une solution de NaOH 1N, extrait à l'AcOEt, lave par une solution d'HCl 6N, alcalinise la phase aqueuse acide par ajout de NaOH 2N, extrait à l'AcOEt, sèche sur $Na_2SO_4$ et évapore sous vide le solvant. On chromatographie le résidu sur silice en éluant par le mélange DCM/MeOH (99/1 ; v/v). On obtient 0,65 g du produit attendu après cristallisation dans l'éther iso, F = 161° C.

EXEMPLE 116

Fumarate de 3-cyclohexyl-5-éthoxy-1-dihydro-1-[2-méthoxy-4-[N-(2-diméthylamino-1,1-diméthyléthyl)carbamoyl]benzènesulfonyl]-2*H*-benzimidazol-2-one

On chauffe à reflux pendant 5 minutes un mélange de 0,48 g du composé obtenu à l'Exemple 115, 0,05 g d'acide fumarique et 15 ml d'acétone. On concentre sous vide le mélange réactionnel. On obtient 0,13 g du produit attendu après cristallisation dans l'éther, F = 135°C.

## Revendications

1. Composé de formule :

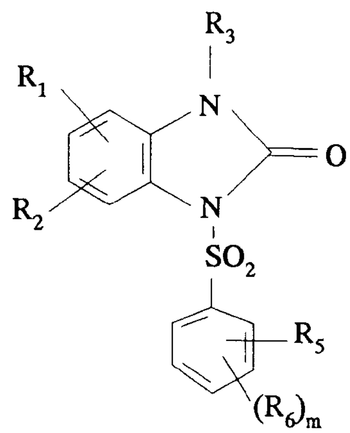

(I)

dans laquelle :

- $R_1$ et $R_2$ représentent chacun indépendamment un hydrogène ; un halogène ; un hydroxy ; un ω-halogéno($C_1$-$C_7$)alcoxy ; un ($C_1$-$C_7$)alkyle ; un trifluorométhyle ; un ($C_1$-$C_7$)alcoxy ; un polyhalogéno($C_1$-$C_7$)alcoxy ; un ω-hydroxy($C_2$-$C_7$)alcoxy ; un ω-méthoxy($C_2$-$C_7$)alcoxy ; un ω-amino($C_2$-$C_7$)alcoxy dans lequel l'amino est libre ou substitué par un ou deux ($C_1$-$C_7$)alkyles ; un ($C_3$-$C_7$)cycloalkyloxy ; un ($C_3$-$C_7$)cycloalkylméthoxy ; un phénoxy ; un benzyloxy ; un ($C_1$-$C_7$)alkylthio ; un phénylthio ; un nitro ; un amino libre ou substitué par un ou deux ($C_1$-$C_7$)alkyles ; un cyano ; un formyle ; un ($C_1$-$C_7$)alkylcarbonyle ; un benzoyle ; un formyloxy ; un ($C_1$-$C_7$)alkylcarbonyloxy ; un benzoyloxy ; un ($C_1$-$C_7$)alkylsulfonamido ; un phénylsulfonamido ; un benzylsulfonamido ; un ($C_1$-$C_7$) alkylcarbonylamino ; un ($C_1$-$C_7$) alcoxycarbonylamino; un uréido non substitué ou substitué par un phényle, un benzyle ou par un ou deux ($C_1$-$C_7$)alkyles ; un thiouréido non substitué ou substitué par un phényle, un benzyle ou par un ou deux ($C_1$-$C_7$)alkyles ;
- $R_3$ représente $R_4$ ; un ($C_1$-$C_8$)alkyle ; un ($C_1$-$C_8$)alkylène substitué par $R_4$ ; un ($C_1$-$C_8$)alkylène substitué par un ($C_1$-$C_4$)alcoxy ; un indanyle ; un hexahydroindanyle ; un adamantyle ; un noradamantyle ; un norbornyle ; un cyclohexyle substitué par un di($C_1$-$C_7$)alkylamino, un carboxy, un ($C_1$-$C_4$)alcoxycarbonyle, un hydroxy, un tétrahydropyran-2-yloxy, un ($C_1$-$C_4$)alcoxy($C_1$-$C_4$)alcoxy ou un phényl($C_1$-$C_2$)alcoxy($C_1$-$C_4$)alcoxy ;
- $R_4$ représente un groupe $-NR_{16}R_{17}$ ; un ($C_3$-$C_7$)cycloalkyle non substitué ou substitué une ou deux fois par un ($C_1$-$C_4$)alkyle ou un ($C_1$-$C_4$)alcoxy ; un groupe Ar ; un furyle ; un thiényle ; un pyrrolyle ; un triazolyle ; un tétrazolyle ; un pyridyle ; un pyridyle N-oxyde ; un pyrimidinyle ; un pyrazolyle ; un pyrazinyle ; un tétrahydropyran-4-yle ; un azétidin-3-yle substitué en position 1 par $R_{18}$ ; un pipérid-4-yle substitué en position 1 par $R_{18}$ ou disubstitué en position 1 par un ou deux ($C_1$-$C_7$)alkyles et/ou un ou deux benzyles ; un pyrrolidinyle ; un perhydroazépinyle ; un morpholinyle ;
- $R_5$ et $R_6$ représentent chacun indépendamment un hydrogène ; un halogène ; un ($C_1$-$C_7$)alkyle ; un trifluorométhyle ; un cyano ; un nitro ; un hydroxylamino ; un carboxy ; un guanidino non substitué ou substitué en position 1 par un ($C_1$-$C_7$)alkyle et/ou en position 3 par un ou deux ($C_1$-$C_7$)alkyles, un phényle ou un benzyle et/ou en position 2 par un cyano; un groupe $-OR_7$; un groupe $-SR_7$ ; un formyle ; un ($C_1$-$C_7$)alkylcarbonyle ; un benzoyle ; un ($C_1$-$C_7$)alcoxycarbonyle ; un phénoxycarbonyle ; un benzyloxycarbonyle ; un groupe $-CONR_{19}R_{20}$ ; un groupe $-CSNR_{11}R_{27}$ ; un groupe $-SO_2NR_{21}R_{22}$ ;

un ($C_1$-$C_7$)alkylsulfonamido ; un benzylsulfonamido; un groupe $-NHSO_2Ar$ ; un groupe $-NR_8R_9$ ; un groupe $-CO-NHCR_{10}R_{23}COR_{12}$; un groupe $-CH_2NR_8R_9$.

- $R_7$ représente un hydrogène ; un ($C_1$-$C_7$)alkyle ; un phényle ; un benzyle ; un ($C_3$-$C_7$)cycloalkyle ; un ($C_2$-$C_7$)alcényle ; un ω-halogéno($C_2$-$C_7$)alkyle ; un polyhalogéno($C_1$-$C_7$)alkyle ; un ω-hydroxy($C_2$-$C_7$)alkyle ; un formyle ; un ($C_1$-$C_7$)alkylcarbonyle ; un benzoyle ; un ω-carboxy($C_1$-$C_7$)alkyle ; un ω-($C_1$-$C_7$)alcoxycarbonyl($C_1$-$C_7$)alkyle ; un ω-benzyloxycarbonyl($C_1$-$C_7$)alkyle ; un ω-amino($C_2$-$C_7$)alkyle dans lequel le groupe amino est libre ou substitué par un ou deux ($C_1$-$C_7$)alkyles, ou sous forme d'ion ammonium ; un ω-carbamoyl($C_1$-$C_7$)alkyle dans lequel le carbamoyle est libre ou substitué par un ou deux ($C_1$-$C_7$)alkyles ;
- $R_8$ et $R_9$ représentent chacun indépendamment un hydrogène ; un ($C_1$-$C_7$)alkyle ; un groupe $-CH_2Ar$ ; $R_9$ peut de plus représenter un groupe Ar ; un ($C_3$-$C_8$)alcényle ; un formyle ; un ($C_1$-$C_7$)alkylcarbonyle ; un ($C_1$-$C_7$)alkylthiocarbonyle ; un ($C_3$-$C_7$)cycloalkylcarbonyle ; un ($C_3$-$C_7$)cycloalkylthiocarbonyle ; un ω-amino($C_2$-$C_7$)alkylcarbonyle dans lequel l'amino est libre ou substitué par un ou deux ($C_1$-$C_7$)alkyles ; un ω-hydroxy($C_1$-$C_7$)alkyicarbonyle ; un ω-benzyloxy ($C_1$-$C_7$)alkylcarbonyle ; un pyridylcarbonyle ; un méthylpyridylcarbonyle ; un thiénylcarbonyle ; un groupe -COAr ; un groupe $-COCH_2Ar$ ; un ($C_1$-$C_7$)alcoxycarbonyle ; un phénoxycarbonyle ; un phénoxythiocarbonyle ; un benzyloxycarbonyle ; un groupe $-COCR_{10}R_{23}NR_{11}R_{27}$ ; un groupe $-CR_{10}R_{23}COR_{12}$ ; un groupe $-(CH_2)_tCOR_{12}$ ; un groupe $CO(CH_2)_uCOR_{12}$ ; un groupe $-CONR_{14}R_{24}$ ; un groupe $-CSNR_{14}R_{24}$ ; un radical hétérocyclique choisi parmi pyrazolyle, imidazolyle, triazolyle, tétrazolyle, pyridyle, pyridazinyle, pyrimidinyle, thiazolyle ;
- ou bien $R_8$ et $R_9$ ensemble avec l'atome d'azote auquel ils sont liés constituent l'hydantoïne, la N-méthylhydantoïne, ou un radical hétérocyclique choisi parmi le morpholin-4-yle, le pyrrol-1-yle, le Δ3-pyrrolin-1-yle, le pyrrolidin-1-yle, l'isoindolin-2-yle dans lequel le noyau benzénique est non substitué ou substitué par un halogène, un ($C_1$-$C_7$)alkyle, un trifluorométhyle ou un méthoxy ;
- $R_{10}$ et $R_{23}$ représentent chacun indépendamment l'hydrogène ; un ($C_1$-$C_7$)alkyle ; un benzyle ;
- ou bien $R_{10}$ et $R_{23}$ ensemble avec l'atome de carbone auquel ils sont liés constituent un ($C_3$-$C_7$)cycloalkyle ;
- $R_{11}$ et $R_{27}$ représentent chacun indépendamment l'hydrogène ou un ($C_1$-$C_7$)alkyle ;
- $R_{12}$ représente un hydroxy ; un ($C_1$-$C_7$)alcoxy ; un amino libre ou substitué par un ou deux ($C_1$-$C_7$)alkyles ;
- $R_{14}$ et $R_{24}$ représentent chacun indépendamment l'hydrogène ; un ($C_1$-$C_7$)alkyle ; $R_{24}$ peut de plus représenter un ($C_1$-$C_7$)alkyle substitué par $R_{15}$ ; un groupe Ar ; un ($C_3$-$C_7$)cycloalkyle ; un adamantyle ;
- ou $R_{14}$ et $R_{24}$ ensemble avec l'atome d'azote auquel ils sont liés constituent un hétérocycle choisi parmi : la morpholine, la thiomorpholine, la pipérazine, l'azétidine, la pyrrolidine, la pipéridine ou la perhydroazépine, ledit hétérocycle étant non substitué ou substitué par un ou plusieurs groupes méthyles, par un phényle, par un groupe amino libre ou portant un groupe protecteur ;
- $R_{15}$ représente un groupe Ar ; un pyridyle ; un hydroxy ; un ($C_1$-$C_7$)alcoxy ; un groupe $-NR_{11}R_{27}$ ; un carboxy ; un ($C_1$-$C_7$)alcoxycarbonyle ;
- $R_{16}$ et $R_{17}$ représentent chacun indépendamment l'hydrogène ou un ($C_1$-$C_7$)alkyle ;
- ou bien $R_{16}$ et $R_{17}$ ensemble avec l'atome d'azote auquel ils sont liés constituent un hétérocycle choisi parmi la morpholine, la thiomorpholine, l'azétidine, la pyrrolidine, la pipéridine, la pipérazine substitué en position 4 par $R_{18}$, ou la perhydroazépine ;
- $R_{18}$ représente un hydrogène ; un ($C_1$-$C_7$)alkyle ; un phényle ; un benzyle ; un formyle ; un ($C_1$-$C_7$)alkylcarbonyle ; un benzoyle ; un ($C_1$-$C_7$)alcoxycarbonyle ; un phénoxycarbonyle ; un carbamoyle libre ou substitué par un ou deux ($C_1$-$C_7$)alkyles ;
- $R_{19}$ et $R_{20}$ représentent chacun indépendamment l'hydrogène ou un ($C_1$-$C_8$)alkyle ; $R_{20}$ peut de plus représenter un ($C_3$-$C_7$)cycloalkyle non substitué ou substitué par un ($C_1$-$C_4$)alkyle ; un groupe Ar ; un pyridyle ; un méthylpyridyle ; un pipérid-4-yle substitué en position 1 par $R_{18}$ ; un pipérid-1-yle ; un pyrrolidin-1-yle ; un morpholin-4-yle ; un thiazol-2-yle ; un indanyle ; un adamantyle ; un ($C_1$-$C_7$)alkyle substitué par un ou plusieurs halogènes ou par $R_{26}$.
- ou bien $R_{19}$ et $R_{20}$ ensemble avec l'atome d'azote auquel ils sont liés constituent un radical hétérocyclique $R_{25}$ ;
- $R_{21}$ et $R_{22}$ représentent chacun indépendamment l'hydrogène ou un ($C_1$-$C_7$)alkyle ;
- ou bien $R_{21}$ et $R_{22}$ ensemble avec l'atome d'azote auquel ils sont liés constituent un radical hétérocyclique $R_{25}$ ;
- $R_{25}$ représente un morpholin-4-yle ; un thiomorpholine-4-yle ; un azétidin-1-yle non substitué ou substitué en position 3 par un groupe $-NR_{11}R_{27}$, un ($C_1$-$C_7$)alkyle, un phényle, un benzyle ou un ($C_1$-

$C_7$)alkylcarbonyle ; un perhydroazépin-1-yle ; un pipérazin-1-yle non substitué ou substitué en position 4 par un ($C_1$-$C_7$)alkyle, un phényle, un benzyle, un ($C_1$-$C_7$)alkylcarbonyle, un ($C_1$-$C_7$)alcoxycarbonyle ou un benzyloxycarbonyle ; un pipérid-1-yle non substitué ou substitué en position 4 par un ($C_1$-$C_7$)alkyle, un phényle, un benzyle, un ($C_1$-$C_7$)alkylcarbonyle, un groupe -$NR_{11}R_{27}$ ; un pyrrolidin-1-yle non substitué ou substitué par un ($C_1$-$C_7$)alkyle, un phényle, un benzyle, un ($C_1$-$C_7$)alkylcarbonyle, un hydroxyméthyle, un carboxy, un ($C_1$-$C_7$)alcoxycarbonyle, un carbamoyle non substitué ou substitué par un ou deux ($C_1$-$C_7$)alkyles ;

- $R_{26}$ représente un hydroxy ; un ($C_1$-$C_7$)alcoxy ; un cyano ; un carboxy ; un ($C_1$-$C_7$)alcoxycarbonyle ; un benzyloxycarbonyle ; un groupe -$NR_{11}R_{27}$ ; un carbamoyle libre ou substitué par un ou deux ($C_1$-$C_7$)alkyles ; un pyrrolidin-1-ylcarbonyle ; un pipérid-1-ylcarbonyle ; un perhydroazépin-1-ylcarbonyle ; un groupe Ar ; un ($C_3$-$C_7$)cycloalkyle ; un adamantyle ; un radical hétérocyclique choisi parmi un pyridyle, un méthylpyridyle, un furanyle, un tétrahydrofuranyle, un thiényle, un méthyltiényle, un pyrrolidin-1-yle, un pipérid-1-yle, un perhydroazépin-1-yle ;
- Ar représente un phényle non substitué ou substitué une ou plusieurs fois par un substituant choisi parmi : un atome d'halogène, un ($C_1$-$C_7$)alkyle, un trifluorométhyle, un hydroxy, un ($C_1$-$C_7$)alcoxy, un carboxy, un ($C_1$-$C_7$)alcoxycarbonyle, un ($C_1$-$C_7$)alkylcarbonyloxy, un nitro, un cyano, un amino, un ($C_1$-$C_7$)alkylamino, un di($C_1$-$C_7$)alkylamino, lesdits substituants étant identiques ou différents;
- t représente un nombre entier qui peut varier de 2 à 5 ;
- u représente un nombre entier qui peut varier de 0 à 5 ;
- m est 1 ou, lorsque $R_6$ est un halogène, un ($C_1$-$C_7$)alkyle ou un ($C_1$-$C_7$)alcoxy, m peut également être 2, 3 ou 4 ou bien $(R_6)_m$ peut représenter m substituants ayant différentes valeurs choisies parmi halogène, ($C_1$-$C_7$)alkyle ou ($C_1$-$C_7$)alcoxy ; ainsi que ses sels.

**2.** Un composé selon la revendication 1 de formule :

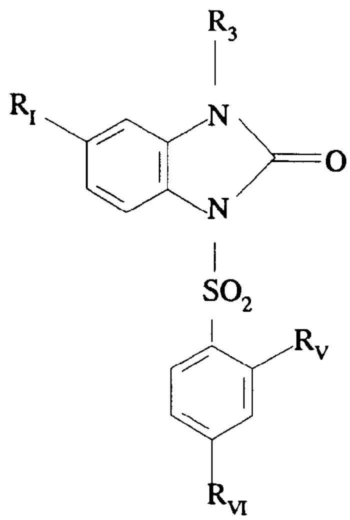

($I_a$)

dans laquelle :

- $R_I$ représente un ($C_1$-$C_4$)alcoxy, un atome de chlore ou de fluor,
- $R_V$ représente l'hydrogène ou un méthoxy,
- $R_{VI}$ représente un ($C_1$-$C_7$)alkylcarboxamido, un groupe -NHCOAr, un groupe -$CONR_{19}R_{20}$, un groupe -$NR_6CONR_{14}R_{24}$, un ($C_1$-$C_7$)alcoxy, un amino libre ou substitué par un ou deux ($C_1$-$C_7$)alkyles ;
- les substituants $R_3$, Ar, $R_6$, $R_{19}$, $R_{20}$, $R_{14}$, $R_{24}$ sont tels que définis pour le composé de formule (I) dans la revendication 1.

ainsi que ses sels.

**3.** Un composé de formule :

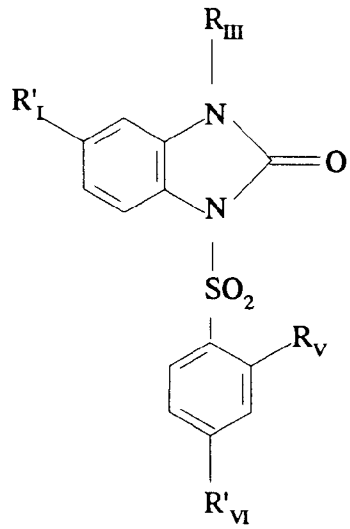

($I_b$)

dans laquelle

- $R'_I$ représente un éthoxy ou un chlore ;
- $R_{III}$ représente un cyclohexyle ou un groupe Ar ;
- $R_V$ représente l'hydrogène ou un méthoxy ;
- $R'_{VI}$ représente un groupe $-CONR_{19}R_{20}$ ou un groupe $-NR_8CONR_{14}R_{24}$ ;
- les substituants Ar, $R_{19}$, $R_{20}$, $R_8$, $R_{14}$, $R_{24}$ sont tels que définis pour le composé de formule (I) dans la revendication 1 ;

ainsi que ses sels.

**4.** Un procédé pour la préparation d'un composé selon l'une quelconque des revendications 1, 2 ou 3 ou d'un de ses sels, caractérisé en ce que :

1) On fait agir sur un composé de formule :

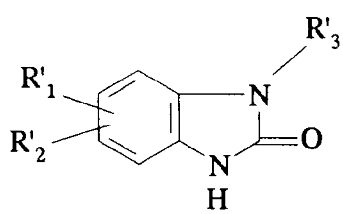

(II)

dans laquelle $R'_1$, $R'_2$ et $R'_3$ représentent, respectivement, soit $R_1$, $R_2$ et $R_3$ tels que définis pour (I) dans la revendication 1, soit des groupes précurseurs de $R_1$, $R_2$ et $R_3$, un halogénure de benzènesulfonyle de formule :

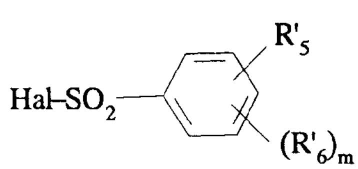

(III)

dans laquelle $R'_5$ et $R'_6$ représentent, respectivement, soit $R_5$ et $R_6$ tels que définis pour (I) dans la revendication 1, soit des groupes précurseurs de $R_8$ et $R_6$ et m est tel que défini pour (I) dans la revendication 1; et,

2/ soit, lorsque $R'_1 = R_1$, $R'_2$, $R_2$, $R'_3 = R_3$, $R'_5 = R_5$ et $R'_6 = R_8$, on isole le composé de formule (I) ainsi obtenu;

3/ soit, lorsque l'un quelconque des groupes $R'_1$, $R'_2$, $R'_3$,$R'_5$ et/ou $R'_6$ représente respectivement un groupe précurseur de $R_1$, $R_2$, $R_3$, $R_5$ et/ou $R_6$, on soumet le composé obtenu à l'étape 1, à un traitement ultérieur pour préparer le composé de formule (I) par transformation de l'un quelconque des groupes $R'_1$, $R'_2$, $R'_3$, $R'_5$ et/ou $R'_6$ en, respectivement, $R_1$, $R_2$, $R_3$, $R_8$ et/ou $R_8$; et

4/ éventuellement, on transforme le composé obtenu à l'étape 2/ ou à l'étape 3/ en l'un de ses sels.

**5.** Composition pharmaceutique contenant à titre de principe actif, un composé selon l'une quelconque des revendications 1 à 3, ou un de ses sels pharmaceutiquement acceptables.

**6.** Composition pharmaceutique contenant un composé selon l'une quelconque des revendications 1 à 3 ou un de ses sels pharmaceutiquement acceptables en association avec un autre principe actif.

**7.** Composition pharmaceutique contenant deux composés selon l'une quelconque des revendications 1 à 3, l'un étant un antagoniste spécifique du récepteur $V_1$ et l'autre étant un antagoniste spécifique du récepteur $V_2$.

**8.** Composition pharmaceutique contenant deux composés selon l'une quelconque des revendications 1 à 3, l'un étant un antagoniste spécifique du récepteur $V_1$ et l'autre étant un antagoniste spécifique de l'ocytocine.

Office européen des brevets

# RAPPORT DE RECHERCHE EUROPEENNE

Numero de la demande
EP 94 40 1736

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X,P, D | WO-A-93 15051 (ELF SANOFI)<br>* le document en entier *<br>--- | 1-8 | C07D235/26<br>C07D401/04<br>C07D403/12<br>A61K31/415<br>C07D417/12<br>C07D405/04 |
| A,D | EP-A-0 526 348 (ELF SANOFI)<br>* le document en entier *<br>--- | 1-8 | |
| A,D | EP-A-0 470 514 (OTSUKA PHARMACEUTICAL CO., LTD.)<br>* revendications 1,30,31; exemple 22 *<br>--- | 1-8 | |
| A | EP-A-0 324 988 (CHINOIN GYOGYSZER ÉS VEGYÉSZETI TERMÉKEK GYÁRA RT.)<br>* le document en entier *<br>----- | 1-8 | |

DOMAINES TECHNIQUES RECHERCHES (Int.Cl.6)

C07D
A61K

Le présent rapport a été établi pour toutes les revendications

| Lieu de la recherche | Date d'achèvement de la recherche | Examinateur |
|---|---|---|
| LA HAYE | 7 Septembre 1994 | Bosma, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)